# EUROPÄISCHE PATENTSCHRIFT

(11) **EP 1 745 009 B1**
(45) Veröffentlichungstag und Bekanntmachung des Hinweises auf die Patenterteilung: **14.11.2012**
(21) Anmeldenummer: 05759144.8
(22) Anmeldetag: 06.05.2005
(51) Int. Cl.: C07D 213/82, C07D 231/14, C07D 249/06, C07D 261/18, C07D 277/30, C07D 307/68, C07D 409/04, C07D 409/12, C07D 417/12, C07C 231/02

(54) **HETEROARYLSUBSTITUIERTE CYCLOHEXYL-1,4-DIAMIN-DERIVATE**
HETEROARYL SUBSTITUTED CYCLOHEXYL-1,4-DIAMINE DERIVATIVES
DERIVES DE CYCLOHEXYLE-1,4-DIAMINE SUBSTITUES PAR HETEROARYLE

(30) Priorität: 10.05.2004 DE 102004023635
(43) Veröffentlichungstag der Anmeldung: 24.01.2007
(73) Patentinhaber: Grünenthal GmbH, 52078 Aachen (DE)
(72) Erfinder: SUNDERMANN, Corinna, 61381 Friedrichsdorf (DE); SUNDERMANN, Bernd, 61381 Friedrichsdorf (DE)
(74) Vertreter: Bülle, Jan
(86) Internationale Anmeldenummer: PCT/EP2005/004911
(87) Internationale Veröffentlichungsnummer: WO 2005/110971

(56) Entgegenhaltungen:
- EP-A- 1 323 710
- WO-A-2004/043949

## Beschreibung

Die vorliegende Erfindung betrifft substituierte Cyclohexyl-1,4-diamin-Derivate, Verfahren zu deren Herstellung, Arzneimittel enthaltend diese Verbindungen und die Verwendung von substituierten Cyclohexyl-1,4-diamin-Derivate-Derivaten zur Herstellung von Arzneimitteln.

Die Behandlung chronischer und nichtchronischer Schmerzzustände hat in der Medizin eine große Bedeutung. Es besteht ein weltweiter Bedarf an gut wirksamen Schmerztherapien. Der dringende Handlungsbedarf für eine patientengerechte und zielorientierte Behandlung chronischer und nicht chronischer Schmerzzustände, wobei hierunter die erfolgreiche und zufriedenstellende Schmerzbehandlung für den Patienten zu verstehen ist, dokumentiert sich in der großen Anzahl von wissenschaftlichen Arbeiten, die auf dem Gebiet der angewandten Analgetik bzw. der Grundlagenforschung zur Nociception in letzter Zeit erschienen sind.

Klassische µ-Opioide wie Morphin sind bei der Therapie starker bis stärkster Schmerzen gut wirksam und von größter Bedeutung für die Schmerztherapie. Es kann jedoch von Vorteil sein, wenn neben dem µ-Opioid-Rezeptor auch andere Opioid-Rezeptoren, insbesondere der ORL-1-Rezeptor, beeinflusst werden, da die reinen µ-Opioide auch unerwünschte Nebenwirkungen wie Obstipation und Atemdepression aufweisen, aber auch zu Abhängigkeit führen können. Auch die Opioid-Rezeptoren δ, κ und ORL-1 sind am Schmerzgeschehen beteiligt (Opioids: Introduction, S. 127-150, Further Opioid Receptors, 455-476 in: Analgesics - From Chemistry and Pharmacology to Clinical Application, Wiley VCH, 2002).

Darüber hinaus ist bekannt, dass eine sich eine Beeinflussung der Serotonin - und/oder Noradrenalin-Wiederaufnahme günstig auf das Wirk- und Nebenwirkungsspektrum von Opioiden auswirken kann (Beispiel: Tramadol, s. Opioids with Clinical Relevance: Tramadol, 228-230 in: Analgesics - From Chemistry and Pharmacology to Clinical Application, Wiley VCH 2002).

Der ORL1-Rezeptor ist außerdem noch an der Regulation weiterer physiologischer und pathophysiologischer Prozesse beteiligt. Hierzu gehören unter anderem Lernen und Gedächtnisbildung (Manabe et al., Nature, 394, 1997, S. 577-581), Hörvermögen (Nishi et al., EMBO J., 16, 1997, S. 1858-1864) sowie zahlreiche weitere Prozesse. In einem Übersichtsartikel von Calo et al. (Br.J. Pharmacol., 129, 2000, 1261 - 1283) wird ein Überblick über die Indikationen oder biologischen Vorgänge gegeben, in denen der ORL1-Rezeptor eine Rolle spielt oder mit hoher Wahrscheinlichkeit spielen könnte. Genannt werden u.a.: Analgesie, Stimulation und Regulation der Nahrungsaufnahme, Einfluß auf µ-Agonisten wie Morphin, Behandlung von Entzugserscheinungen, Reduzierung des Suchtpotentials von Opioiden, Anxiolyse, Modulation der Bewegungsaktivität, Gedächtnis-Störungen, Epilepsie; Modulation der Neurotransmitter-Ausschüttung, insbesondere von Glutamat, Serotonin und Dopamin, und damit neurodegenerative Erkrankungen; Beeinflußung des cardiovaskulären Systems, Auslösung einer Erektion, Diurese, Antinatriurese, Elektrolyt-Haushalt, arterieller Blutdruck, Wasserspeicher-Krankheiten, intestinale Motilität (Diarrhoe), relaxierende Effekte auf die Atemwege, Mikturations Reflex (Harninkontinenz). Weiter wird die Verwendung von Agonisten und Antagonisten als Anoretika, Analgetika (auch in Coadministration mit Opioiden) oder Nootropika diskutiert.

Aus dem Stand der Technik (WO 02090317) sind strukturell verwandte Verbindungen bekannt, die eine Affinität zum ORL-1-Rezeptor besitzen. Für diese Strukturklasse wurde bislang kein Einfluss auf die Noradrenalin- und Serotonin-Wiederaufnahme beschrieben.

EP-A 1 323 710 beschreibt ebenfalls strukturell vergleichbare Verbindungen, wobei jedoch keine dem Rest R⁴ in Formel (I) entsprechende Gruppe mittelbar oder unmittelbar an A gebunden ist. In wie weit solche Verbindungen noch pharmakologisch aktiv sind oder sein könnten, ist EP-A 1 323 710 nicht zu entnehmen.

Aufgabe der vorliegenden Erfindung war es, Arzneimittel zur Verfügung zu stellen, die auf das Opioid-Rezeptor-System wirken und damit für Arzneimittel insbesondere zur Behandlung der verschiedenen mit diesem System nach dem Stand der Technik in Verbindung stehenden Krankeiten bzw. zum Einsatz in den dort genannten Indikationen geeignet sind. Daüber hinaus sollten die Verbindungen die Noradrenalin- und Serotonin-Wiederaufnahme beeinflussen.

Gegenstand der Erfindung sind daher substituierte Cyclohexyl-1,4-diamin-Derivate-Derivate der allgemeinen Formel I, 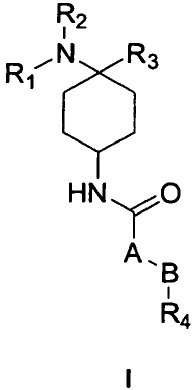 worin
R¹ und R², unabhängig voneinander für H; C₁₋₅-Alkyl jeweils gesättigt oder ungesättigt, verzweigt oder unverzweigt, einfach oder mehrfach substituiert oder unsubstituiert; C₃₋₈-Cycloalkyl, jeweils einfach oder mehrfach substituiert oder unsubstituiert; oder über C₁₋₃-Alkyl gebundenes Aryl, C₃₋₈-Cycloalkyl oder Heteroaryl, jeweils einfach oder mehrfach substituiert oder unsubstituiert, stehen;
oder die Reste R¹ und R² zusammen für CH₂CH₂OCH₂CH₂, CH₂CH₂NR¹⁰CH₂CH₂ oder (CH₂)₃₋₆ stehen,
   wobei R¹⁰ H; C₁₋₅-Alkyl, jeweils gesättigt oder ungesättigt, verzweigt oder unverzweigt, einfach oder mehrfach substituiert oder unsubstituiert; C₃₋₈-Cycloalkyl, jeweils einfach oder mehrfach substituiert oder unsubstituiert; Aryl-, oder Heteroaryl, jeweils einfach oder mehrfach substituiert oder unsubstituiert; oder über C₁₋₃-Alkyl gebundenes Aryl, C₃₋₈-Cycloalkyl oder Heteroaryl, jeweils einfach oder mehrfach substituiert oder unsubstituiert; C(O)Phenyl, C(O)Heteroaryl, C(O)C₁₋₅-Alkyl, jeweils substituiert oder unsubstituiert, bedeutet;
R³ für C₁₋₅-Alkyl, jeweils gesättigt oder ungesättigt, verzweigt oder unverzweigt, einfach oder mehrfach substituiert oder unsubstituiert; C₃₋₈-Cycloalkyl, jeweils einfach oder mehrfach substituiert oder unsubstituiert; Aryl oder Heteroaryl, jeweils unsubstituiert oder einfach oder mehrfach substituiert; über C₁₋₃-Alkyl-Gruppe gebundenes Aryl, Heteroaryl oder C₃₋₈-Cycloalkyl, jeweils unsubstituiert oder einfach oder mehrfach substituiert, steht;
A für Heteroaryl, unsubstituiert oder einfach oder mehrfach substituiert, steht
B für (CH₂)ₘ mit m = 0, 1; SO₂, O, S, C(O), C(S) steht,
R⁴ für Heteroaryl oder Aryl, unsubstituiert oder einfach oder mehrfach substituiert, oder einen über eine C₁₋₃-Alkylkette verknüpften Heteroaryl- oder Aryl-Rest, unsubstituiert oder einfach oder mehrfach substituiert, steht,
   in Form des Razemats; der Enantiomere, Diastereomere, Mischungen der Enantiomere oder Diastereomere oder eines einzelnen Enantiomers oder Diastereomers; der Basen und/oder Salze physiologisch verträglicher Säuren oder Kationen.

Die erfindungsgemäßen Verbindungen zeigen gute Bindung an den µ-Rezeptor und den ORL-1-Rezeptor, aber auch an andere Opioidrezeptoren. Überraschenderweise zeigte es sich, dass die Verbindungen auch gute Inhibitoren der Noradrenalin- und der Serotonin-Wiederaufnahme sind. Damit eignen sie sich auch zur Behandlung von Depressionen, und/oder Bulimie und/oder Anorexie und/oder Katalepsie und/oder zur Anxiolyse und/oder zur Vigilanz- und/oder Libidosteigerung.

Die Ausdrücke "C₁₋₅-Alkyl" und "C₁₋₃-Alkyl" umfassen im Sinne dieser Erfindung acyclische gesättigte oder ungesättigte Kohlenwasserstoffreste, die verzweigt- oder geradkettig sowie unsubstituiert oder ein- oder mehrfach substituiert sein können, mit 1, 2, 3, 4 oder 5 C-Atomen bzw. 1, 2 oder 3 C-Atomen, d.h. C₁₋₅-Alkanyle, C₂₋₅-Alkenyle und C₂₋₅-Alkinyle bzw. C₁₋₃-Alkanyle, C₂₋₃-Alkenyle und C₂₋₃-Alkinyle. Dabei weisen Alkenyle mindestens eine C-C-Doppelbindung und Alkinyle mindestens eine C-C-Dreifachbindung auf. Vorteilhaft ist Alkyl aus der Gruppe ausgewählt, die Methyl, Ethyl, n-Propyl, 2-Propyl, n-Butyl, iso-Butyl, sec.-Butyl, tert.-Butyl, n-Pentyl, iso-Pentyl, neo-Pentyl, Ethylenyl (Vinyl), Ethinyl, Propenyl (-CH₂CH=CH₂, -CH=CH-CH₃, -C(=CH₂)-CH₃), Propinyl (-CH-C≡CH, -C≡C-CH₃), 1,1-Dimethylethyl, 1,1-Dimethylpropyl, Butenyl, Butinyl, Pentenyl und Pentinyl umfaßt.

Der Ausdruck "Cycloalkyl" oder "C₃₋₈-Cycloalkyl" bedeutet für die Zwecke dieser Erfindung cyclische Kohlenwasserstoffe mit 3, 4, 5, 6, 7 oder 8 Kohlenstoffatomen, wobei die Kohlenwasserstoffe gesättigt oder ungesättigt (aber nicht aromatisch), unsubstituiert oder ein- oder mehrfach substituiert sein können. In Bezug auf Cycloalkyl umfasst der Begriff auch gesättigte oder ungesättigte (aber nicht aromatische) Cycloalkyle, in denen ein oder zwei Kohlenstoffatome durch ein Heteroatom S, N oder O ersetzt sind. Vorteilhaft ist C₃₋₈-Cycloalkyl aus der Gruppe ausgewählt, die Cyclopropyl, Cyclobutyl, Cyclopentyl, Cyclohexyl, Cycloheptyl, Cyclooctyl, Cyclopentenyl, Cyclohexenyl, Cycloheptenyl und Cyclooctenyl, aber auch Tetrahydropyranyl, Dioxanyl, Dioxolanyl, Morpholinyl, Piperidinyl, Piperazinyl, Pyrazolinonyl und Pyrrolidinyl enthält.

Unter dem Begriff (CH₂)₃₋₆ ist -CH₂-CH₂-CH₂-, -CH₂-CH₂-CH₂-CH₂-, -CH₂-CH₂-CH₂-CH₂-CH₂- und CH₂-CH₂-CH₂-CH₂-CH₂-CH₂- zu verstehen.

Der Ausdruck "Aryl" bedeutet im Sinne dieser Erfindung carbocyclische Ringsysteme mit mindestens einem aromatischen Ring, aber ohne Heteroatome in nur einem der Ringe, u.a. Phenyle, Naphthyle und Phenanthrenyle, Fluoranthenyle, Fluorenyle, Indanyle und Tetralinyle. Die Aryl-Reste können auch mit weiteren gesättigten, (partiell) ungesättigten oder aromatischen Ringsystemen kondensiert sein. Jeder Aryl-Rest kann unsubstituiert oder einfach oder mehrfach substituiert vorliegen, wobei die Aryl-Substituenten gleich oder verschieden und in jeder beliebigen und möglichen Position des Aryls sein können. Besonders vorteilhaft sind Phenyl- oder Naphthyl-Reste.

Der Ausdruck "Heteroaryl" steht für einen 5-, 6- oder 7-gliedrigen cyclischen aromatischen Rest, der mindestens 1, ggf. auch 2, 3, 4 oder 5 Heteroatome, enthält, wobei die Heteroatome gleich oder verschieden sind und der Heterocyclus unsubstituiert oder ein- oder mehrfach substituiert sein kann; im Falle der Substitution am Heterocyclus können die Substituenten gleich oder verschieden sein und in jeder beliebigen und möglichen Position des Heteroaryls sein. Der Heterocyclus kann auch Teil eines bi- oder polycyclischen Systems sein. Bevorzugte Heteroatome sind Stickstoff, Sauerstoff und Schwefel. Es ist bevorzugt, daß der Heteroaryl-Rest ausgewählt ist aus der Gruppe, die Pyrrolyl, Indolyl, Furyl (Furanyl), Benzofuranyl, Thienyl (Thiophenyl), Benzothienyl, Benzothiadiazolyl, Benzothiazolyl, Benzotriazolyl, Benzodioxolanyl, Benzodioxanyl, Phtalazinyl, Pyrazolyl, Imidazolyl, Thiazolyl, Oxazolyl, Isoxazolyl, Pyridinyl, Pyridazinyl, Pyrimidinyl, Pyrazinyl, Pyranyl, Indazolyl, Purinyl, Indolizinyl, Chinolinyl, Isochinolinyl, Isothiazolyl, Imidazolyl, Triazolyl, Triazinyl, Chinazolinyl, Carbazolyl, Phenazinyl, Phenothiazinyl oder Oxadiazolyl enthält, wobei die Bindung an die Verbindungen der allgemeinen Struktur I über jedes beliebige und mögliche Ringglied des Heteroaryl-Restes erfolgen kann.

Im Zusammenhang mit "Alkyl" versteht man unter dem Begriff "substituiert" im Sinne dieser Erfindung die Substitution eines oder mehrerer Wasserstoffreste durch F, Cl, Br, I, -CN, =O, =S, NH₂, NH-Alkyl, NH-Aryl, NH-Heteroaryl, NH-Cycloalkyl, NH-Alkyl-Aryl, NH-Alkyl-Heteroaryl, NH-Alkyl-OH, N(Alkyl)₂, N(Alkyl-Aryl)₂, N(Alkyl-Heteroaryl)₂, N(Cycloalkyl)₂, N(Alkyl-OH)₂, NH(C=O)Alkyl, NH(C=O)Aryl), NO₂, SH, S-Alkyl, S-Aryl, S-Heteroaryl, S-Alkyl-Aryl, S-Alkyl-Heteroaryl, S-Cycloalkyl, S-Alkyl-OH, S-Alkyl-SH, OH, O-Alkyl, O-Aryl, O-Heteroaryl, O-Alkyl-Aryl, O-Alkyl-Heteroaryl, O-Cycloalkyl, O-Alkyl-OH, CHO, C(=O)C₁₋₆-Alkyl, C(=S)C₁₋₆-Alkyl, C(=O)Aryl, C(=S)Aryl, C(=O)C₁₋₆-Alkyl-Aryl, C(=S)C₁₋₆-Alkyl-Aryl, C(=O)-Heteroaryl, C(=S)-Heteroaryl, C(=O)-Cycloalkyl, C(=S)-Cycloalkyl, CO₂H, CO₂-Alkyl, CO₂-Alkyl-Aryl, C(=O)NH₂, C(=O)NH-Alkyl, C(=O)NHAryl, C(=O)NH-Cycloalkyl, C(=O)N(Alkyl)₂, C(=O)N(Alkyl-Aryl)₂, C(=O)N(Alkyl-Heteroaryl)₂, C(=O)N(Cycloalkyl)₂, SO-Alkyl, SO₂-Alkyl, SO₂NH₂, SO₃H, PO(O-C₁₋₆-Alkyl)₂, Cycloalkyl, Aryl oder Heteroaryl, wobei unter mehrfach substituierten Resten solche Reste zu verstehen sind, die entweder an verschiedenen oder an gleichen Atomen mehrfach, z. B. zwei- oder dreifach, substituiert sind, beispielsweise dreifach am gleichen C-Atom wie im Falle von CF₃ oder -CH₂CF₃ oder an verschiedenen Stellen wie im Falle von -CH(OH)-CH=CH-CHCl₂. Die Mehrfachsubstitution kann mit dem gleichen oder mit verschiedenen Substituenten erfolgen. Ggf. kann ein Substituent auch seinerseits substituiert sein; so umfaßt -OAlkyl u.a. auch ―O-CH₂-CH₂-O-CH₂-CH₂-OH.

In Bezug auf "Aryl", "Heteroaryl" sowie "Cycloalkyl" versteht man im Sinne dieser Erfindung unter "ein- oder mehrfach substituiert" die ein- oder mehrfache, z.B. zwei-, drei- vier- oder fünffache, Substitution eines oder mehrerer Wasserstoffatome des Ringsystems durch F, Cl, Br, I, CN, NH₂, NH-Alkyl, NH-Aryl, NH-Heteroaryl, NH-Alkyl-Aryl, NH-Alkyl-Heteroaryl, NH-Cycloalkyl, NH-Alkyl-OH, N(Alkyl)₂, N(Alkyl-Aryl)₂, N(Alkyl-Heteroaryl)₂, N(Cycloalkyl)₂, N(Alkyl-OH)₂, NO₂, SH, S-Alkyl, S-Cycloalkyl, S-Aryl, S-Heteroaryl, S-Alkyl-Aryl, S-Alkyl-Heteroaryl, S-Cycloalkyl, S-Alkyl-OH, S-Alkyl-SH, OH, O-Alkyl, O-Cycloalkyl, O-Aryl, O-Heteroaryl, O-Alkyl-Aryl, O-Alkyl-Heteroaryl, O-Cycloalkyl, O-Alkyl-OH, CHO, C(=O)C₁₋₆-Alkyl, C(=S)C₁₋₆-Alkyl, C(=O)Aryl, C(=S)Aryl, C(=O)-C₁₋₆-Alkyl-Aryl, C(=S)C₁₋₆-Alkyl-Aryl, C(=O)-Heteroaryl, C(=S)-Heteroaryl, C(=O)-Cycloalkyl, C(=S)-Cycloalkyl, CO₂H, CO₂-Alkyl, CO₂-Alkyl-Aryl, C(=O)NH₂, C(=O)NH-Alkyl, C(=O)NHAryl, C(=O)NH-Cycloalkyl, C(=O)N(Alkyl)₂, C(=O)N(Alkyl-Aryl)₂, C(=O)N(Alkyl-Heteroaryl)₂, C(=O)N(Cycloalkyl)₂, S(O)-Alkyl, S(O)-Aryl, SO₂-Alkyl, SO₂-Aryl, SO₂NH₂, SO₃H, CF₃, =O, =S; 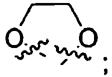 Alkyl, Cycloalkyl, Aryl und/oder Heteroaryl; an einem oder ggf. verschiedenen Atomen (wobei ein Substituent ggf. seinerseits substituiert sein kann). Die Mehrfachsubstitution erfolgt dabei mit dem gleichen oder mit unterschiedlichen Substituenten.

Unter dem Begriff Salz ist jegliche Form des erfindungsgemäßen Wirkstoffes zu verstehen, in dem dieser eine ionische Form annimmt bzw. geladen ist und mit einem Gegenion (einem Kation oder Anion) gekoppelt ist bzw. sich in Lösung befindet. Darunter sind auch Komplexe des Wirkstoffes mit anderen Molekülen und Ionen zu verstehen, insbesondere Komplexe, die über ionische Wechselwirkungen komplexiert sind. Insbesondere versteht man darunter (und dies ist auch eine bevorzugte Ausführungsform dieser Erfindung) physiologisch verträgliche Salze, insbesondere physiologisch verträgliche Salze mit Kationen oder Basen und physiologisch verträgliche Salze mit Anionen oder Säuren oder auch ein mit einer physiologisch verträglichen Säure oder einem physiologisch verträglichen Kation gebildetes Salz.

Unter dem Begriff des physiologisch verträglichen Salzes mit Anionen oder Säuren versteht man im Sinne dieser Erfindung Salze mindestens einer der erfindungsgemäßen Verbindungen ― meist, beispielsweise am Stickstoff, protoniert - als Kation mit mindestens einem Anion, die physiologisch ― insbesondere bei Anwendung im Menschen und/oder Säugetier ― verträglich sind. Insbesondere versteht man darunter im Sinne dieser Erfindung das mit einer physiologisch verträglichen Säure gebildete Salz, nämlich Salze des jeweiligen Wirkstoffes mit anorganischen bzw. organischen Säuren, die physiologisch - insbesondere bei Anwendung im Menschen und/oder Säugetier - verträglich sind. Beispiele für physiologisch verträgliche Salze bestimmter Säuren sind Salze der: Salzsäure, Bromwasserstoffsäure, Schwefelsäure, Methansulfonsäure, Ameisensäure, Essigsäure, Oxalsäure, Bernsteinsäure, Apfelsäure, Weinsäure, Mandelsäure, Fumarsäure, Milchsäure, Zitronensäure, Glutaminsäure, Saccharinsäure, Monomethylsebacinsäure, 5-Oxo-prolin, Hexan-1-sulfonsäure, Nicotinsäure, 2-, 3-oder 4-Aminobenzoesäure, 2,4,6-Trimethyl-benzoesäure, α-Liponsäure, Acetylglycin, Phosphorsäure, Maleinsäure, Malonsäure, Hippursäure und/oder Asparaginsäure. Besonders bevorzugt ist das Hydrochlorid-Salz, das Citrat und das Hemicitrat.

Unter dem Begriff des mit einer physiologisch verträglichen Säure gebildeten Salzes versteht man im Sinne dieser Erfindung Salze des jeweiligen Wirkstoffes mit anorganischen bzw. organischen Säuren, die physiologisch - insbesondere bei Anwendung im Menschen und/oder Säugetier - verträglich sind. Besonders bevorzugt ist das Hydrochlorid und das Citrat. Beispiele für physiologisch verträgliche Säuren sind: Salzsäure, Bromwasserstoffsäure, Schwefelsäure, Methansulfonsäure, Ameisensäure, Essigsäure, Oxalsäure, Bernsteinsäure, Weinsäure, Mandelsäure, Fumarsäure, Milchsäure, Zitronensäure, Glutaminsäure, Saccharinsäure, Monomethylsebacinsäure, 5-Oxo-prolin, Hexan-1-sulfonsäure, Nicotinsäure, 2-, 3-oder 4-Aminobenzoesäure, 2,4,6-Trimethyl-benzoesäure, α-Liponsäure, Acetylglycin, Hippursäure und/oder Asparaginsäure.

Unter dem Begriff des physiologisch verträglichen Salzes mit Kationen oder Basen versteht man im Sinne dieser Erfindung Salze mindestens einer der erfindungsgemäßen Verbindungen - meist einer (deprotonierten) Säure - als Anion mit mindestens einem, vorzugsweise anorganischen, Kation, die physiologisch ― insbesondere bei Anwendung im Menschen und/oder Säugetier ― verträglich sind. Besonders bevorzugt sind die Salze der Alkali- und Erdalkalimetalle aber auch Ammoniumsalze, insbesondere aber (Mono-) oder (Di-) Natrium-, (Mono-) oder (Di-) Kalium-, Magnesium- oder Calzium-Salze.

Unter dem Begriff des mit einem physiologisch verträglichen Kation gebildeten Salzes versteht man im Sinne dieser Erfindung Salze mindestens einer der jeweiligen Verbindungen als Anion mit mindestens einem anorganischen Kation, das physiologisch ― insbesondere bei Anwendung im Menschen und/oder Säugetier ― veträglich ist. Besonders bevorzugt sind die Salze der Alkali- und Erdalkalimetalle aber auch Ammoniumsalze, insbesondere aber (Mono-) oder (Di-) Natrium-, (Mono-) oder (Di-) Kalium-, Magnesium- oder Calzium-Salze.

Für eine bevorzugte Ausführungsform der erfindungsgemäßen substituierten Cyclohexyl-1,4-diamin-Derivate gilt, dass
R¹ und R² unabhängig voneinander für H; C₁₋₅-Alkyl, gesättigt oder ungesättigt, verzweigt oder unverzweigt, einfach oder mehrfach substituiert oder unsubstituiert, stehen;
oder die Reste R¹ und R² zusammen einen Ring bilden und CH₂CH₂OCH₂CH₂, CH₂CH₂NR¹⁰CH₂CH₂ oder (CH₂)₃₋₆ bedeuten,
wobei R¹⁰ H; C₁₋₅-Alkyl, gesättigt oder ungesättigt, verzweigt oder unverzweigt, einfach oder mehrfach substituiert oder unsubstituiert, bedeutet.

Besonders bevorzugt sind substituierte Cyclohexyl-1,4-diamin -Derivate, worin R¹ und R² unabhängig voneinander für CH₃ oder H stehen, wobei R¹ und R² nicht gleichzeitig H bedeuten, oder R¹ und R² für CH₂CH₂OCH₂CH₂, (CH₂)₄, (CH₂)₅ oder (CH₂)₆ stehen.

Weiterhin bevorzugt sind substituierte Cyclohexyl-1,4-diamin -Derivate, worin
R³ für Cyclopentyl, Cyclohexyl, Phenyl, Benzyl, Naphthyl, Anthracenyl, Thiophenyl, Benzothiophenyl, Furyl, Benzofuranyl, Benzodioxolanyl, Indolyl, Indanyl, Benzodioxanyl, Pyrrolyl, Pyridyl, Pyrimidyl oder Pyrazinyl, jeweils unsubstituiert oder einfach oder mehrfach substituiert; über eine gesättigte, unverzweigte C₁₋₂-Alkyl-Gruppe gebundenen C₅₋₆-Cycloalkyl, Phenyl, Naphthyl, Anthracenyl, Thiophenyl, Benzothiophenyl, Pyridyl, Furyl, Benzofuranyl, Benzodioxolanyl, Indolyl, Indanyl, Benzodioxanyl, Pyrrolyl, Pyrimidyl oder Pyrazinyl, jeweils unsubstituiert oder einfach oder mehrfach substituiert, steht;
insbesondere
R³ Phenyl, Furyl, Thiophenyl, Naphthyl, Benzyl, Benzofuranyl, Indolyl, Indanyl, Benzodioxanyl, Benzodioxolanyl, Pyridyl, Pyrimidyl, Pyrazinyl oder Benzothiophenyl, jeweils unsubstituiert oder einfach oder mehrfach substituiert; über eine gesättigte, unverzweigte C₁₋₂-Alkyl-Gruppe gebundenen Phenyl, Furyl oder Thiophenyl, jeweils unsubstituiert oder einfach oder mehrfach substituiert, bedeutet.

Besonders bevorzugt sind substituierte Cyclohexyl-1,4-diamin -Derivate, worin R³ für Phenyl, Phenethyl, Thiophenyl, Pyridyl oder Benzyl, jeweils substituiert oder unsubstituiert, steht, besonders bevorzugt für 4-Methylbenzyl, 2-Methylbenzyl, 3-Chlorbenzyl, 4-Chlorbenzyl, 2-Chlorbenzyl, 4-Fluorbenzyl, 3-Fluorbenzyl, 3-Methylbenzyl, 2-Fluorbenzyl, Benzyl, Phenethyl, Phenyl, Pyridyl, Thiophenyl und 3-Fluorphenyl.

Bevorzugt sind auch substituierte Cyclohexyl-1,4-diamin ―Derivate, bei denen A für Pyrrolyl, Thiophenyl, Furanyl, Pyrazolyl, Isoxazolyl, Oxazolyl, Isothiazolyl, Imidazolyl, Triazolyl, Tetrahydroisoxazolyl, Pyridinyl, Pyrimidinyl, Pyrazinyl, Triazinyl, Indolyl, Benzofuranyl, Benzothiophenyl, Indazolyl, Benzimidazolyl, Purinyl, Chinolinyl, Isochinolinyl, Phthalazin, Benzothiadiazolyl, Benzothiazolyl, Benzotriazolyl, Benzodioxolanyl, Benzodioxanyl und Carbazolyl, unsubstituiert oder einfach oder mehrfach substituiert, steht.

Besonders bevorzugt sind substituierte Cyclohexyl-1,4-diamin-Derivate, bei denen A für Pyridyl, Thiophenyl, Pyrazolyl, Triazolyl, Tetrahydroisoxazolyl, Isoxazolyl, Thiazolyl und Furanyl steht.

Bevorzugt sind weiterhin substituierte Cyclohexyl-1,4-diamin ―Derivate, bei denen B für (CH₂)ₘ mit m = 0, 1; SO₂, O, S steht.

Darüber hinaus sind substituierte Cyclohexyl-1,4-diamin ―Derivate bevorzugt, bei denen R⁴ für Phenyl, Benzyl, Naphthyl, Anthracenyl, Thiophenyl, Benzothiophenyl, Furanyl, Isothiazolyl, Imidazolyl, Triazolyl, Triazinyl, Pyrazolyl, Benzofuranyl, Benzodioxolanyl, Isochinolinyl, Phthalazin, Benzo[1,2,5]thiadiazol, Benzothiazol, Benzotriazol, Chinolinyl, Carbazol, Isoxazolyl, Oxazolyl, Indolyl, Indanyl, Benzodioxanyl, Indazolyl, Benzimidazolyl, Pyrrolyl, Pyridyl, Pyrimidyl oder Pyrazinyl, jeweils unsubstituiert oder einfach oder mehrfach substituiert; über eine gesättigte, unverzweigte C₁₋₂-Alkyl-Gruppe gebundenen Phenyl, Naphthyl, Anthracenyl, Thiophenyl, Benzothiophenyl, Pyridyl, Furyl, Benzofuranyl, Indolyl, Indanyl, Benzodioxanyl, Pyrrolyl, Pyrimidyl oder Pyrazinyl, jeweils unsubstituiert oder einfach oder mehrfach substituiert, steht,
insbesondere
R⁴ für Phenyl, Benzyl, Naphthyl, Thiophenyl, Benzothiophenyl, Furanyl, Isothiazolyl, Imidazolyl, Triazolyl, Pyrazolyl, Benzofuranyl, Isochinolinyl, Benzothiazol, Benzotriazol, Chinolinyl, Isoxazolyl, Oxazolyl, Indolyl, Pyrrolyl, Pyridyl, Pyrimidyl oder Pyrazinyl, jeweils unsubstituiert oder einfach oder mehrfach substituiert; Benzyl oder Phenethyl, jeweils unsubstituiert oder einfach oder mehrfach substituiert, steht.

Besonders bevorzugt sind substituierte Cyclohexyl-1,4-diamin -Derivate, bei denen R⁴ für Phenyl, Pyrazolyl oder Thiophenyl, unsubstituiert oder einfach oder mehrfach substituiert, steht.

Ganz besonders bevorzugt sind substituierte Cyclohexyl-1,4-diamin-Derivate aus der Gruppe
5-Methyl-2-phenyl-2H-[1,2,3]triazol-4-carbonsäure (4-dimethylamino-4-phenyl-cyclohexyl)-amid
1-Phenyl-5-propyl-1H-pyrazol-4-carbonsäure [4-dimethylamino-4-(3-fluor-phenyl)-cyclohexyl]-amid
3-(2,6-Dichlor-phenyl)-5-methyl-isoxazol-4-carbonsäure (4-benzyl-4-pyrrolidin-1-yl-cyclohexyl)-amid
5-Methyl-2-phenyl-2H-[1,2,3]triazol-4-carbonsäure (4-benzyl-4-piperidin-1-yl-cyclohexyl)-amid
5-Methyl-2-phenyl-2H-[1,2,3]triazol-4-carbonsäure (4-phenyl-4-piperidin-1-yl-cyclohexyl)-amid
N-(4-Dimethylamino-4-phenyl-cyclohexyl)-2-phenoxy-nicotinamid
2-(4-Chlor-phenylsulfanyl)-N-(4-dimethylamino-4-pyridin-2-yl-cyclohexyl)-nicotinamid
N-(4-Dimethylamino-4-thiophen-2-yl-cyclohexyl)-2-(5-methyl-2-phenyl-thiazol-4-yl)-acetamid
2-(4-Chlor-phenylsulfanyl)-N-[4-dimethylamino-4-(3-fluor-phenyl)-cyclohexyl]-nicotinamid
N-(4-Dimethylamino-4-thiophen-2-yl-cyclohexyl)-2-phenoxy-nicotinamid
3-(2-Chlor-phenyl)-5-methyl-isoxazol-4-carbonsäure (4-dimethylamino-4-phenyl-cyclohexyl)-amid
5-(4-Chlor-phenyl)-2-methyl-furan-3-carbonsäure [4-dimethylamino-4-(3-fluor-phenyl)-cyclohexyl]-amid
3-(2-Chlor-6-fluor-phenyl)-5-methyl-isoxazol-4-carbonsäure (4-dimethylamino-4-phenethyl-cyclohexyl)-amid
N-(4-Benzyl-4-pyrrolidin-1-yl-cyclohexyl)-2-(4-chlor-phenylsulfanyl)-nicotinamid
3-(2-Chlor-6-fluor-phenyl)-5-methyl-isoxazol-4-carbonsäure (4-dimethylamino-4-phenyl-cyclohexyl)-amid
3-(2,6-Dichlor-phenyl)-5-methyl-isoxazol-4-carbonsäure (4-benzyl-4-piperidin-1-yl-cyclohexyl)-amid
N-(4-Dimethylamino-4-phenethyl-cyclohexyl)-2-(5-methyl-2-phenyl-thiazol-4-yl)-acetamid
3-(2-Chlor-phenyl)-5-methyl-isoxazol-4-carbonsäure (4-dimethylamino-4-phenethyl-cyclohexyl)-amid
5-Benzyl-furan-2-carbonsäure (4-benzyl-4-dimethylamino-cyclohexyl)-amid 1-(4
-Chlor-phenyl)-5-trifluormethyl-1H-pyrazol-4-carbonsäure [4-dimethylamino-4-(3-fluorphenyl)-cyclohexyl]-amid
N-(4-Dimethylamino-4-pyridin-2-yl-cyclohexyl)-2-phenoxy-nicotinamid
5-Benzyl-furan-2-carbonsäure (4-phenyl-4-piperidin-1-yl-cyclohexyl)-amid
5-Methyl-2-phenyl-2H-[1,2,3]triazol-4-carbonsäure [4-dimethylamino-4-(2-methyl-benzyl)-cyclohexyl]-amid
2-(4-Chlor-phenylsulfanyl)-N-[4-dimethylamino-4-(2-fluor-benzyl)-cyclohexyl]-nicotinamid
3-(2,6-Dichlor-phenyl)-5-methyl-isoxazol-4-carbonsäure [4-dimethylamino-4-(2-fluor-benzyl)-cyclohexyl]-amid
5-Methyl-2-phenyl-2H-[1,2,3]triazol-4-carbonsäure [4-dimethylamino-4-(4-fluor-benzyl)-cyclohexyl]-amid
5-Benzyl-furan-2-carbonsäure [4-dimethylamino-4-(2-fluor-benzyl)-cyclohexyl]-amid
3-(2-Chlor-phenyl)-5-methyl-isoxazol-4-carbonsäure [4-dimethylamino-4-(3-fluor-phenyl)-cyclohexyl]-amid
3-(2-Chlor-6-fluor-phenyl)-5-methyl-isoxazol-4-carbonsäure [4-dimethylamino-4-(3-fluorphenyl)-cyclohexyl]-amid
N-(4-Dimethylamino-4-phenethyl-cyclohexyl)-2-phenoxy-nicotinamid
3-(2-Chlor-phenyl)-5-methyl-isoxazol-4-carbonsäure [4-(3-chlor-benzyl)-4-dimethylamino-cyclohexyl]-amid
3-(2,6-Dichlor-phenyl)-5-methyl-isoxazol-4-carbonsäure [4-dimethylamino-4-(3-fluor-phenyl)-cyclohexyl]-amid
5-Benzyl-furan-2-carbonsäure (4-dimethylamino-4-phenethyl-cyclohexyl)-amid
5-Methyl-2-phenyl-2H-[1,2,3]triazol-4-carbonsäure [4-dimethylamino-4-(4-methyl-benzyl)-cyclohexyl]-amid
5-Benzyl-furan-2-carbonsäure [4-dimethylamino-4-(2-methyl-benzyl)-cyclohexyl]-amid
3-(2,6-Dichlor-phenyl)-5-methyl-isoxazol-4-carbonsäure (4-dimethylamino-4-phenethyl-cyclohexyl)-amid
N-(4-Benzyl-4-pyrrolidin-1-yl-cyclohexyl)-2-phenoxy-nicotinamid
5-Benzyl-furan-2-carbonsäure [4-dimethylamino-4-(3-methyl-benzyl)-cyclohexyl]-amid
5-Benzyl-furan-2-carbonsäure [4-dimethylamino-4-(3-fluor-benzyl)-cyclohexyl]-amid
N-[4-(3-Chlor-benzyl)-4-dimethylamino-cyclohexyl]-2-(4-chlor-phenylsulfanyl)-nicotinamid
2-(5-Methyl-2-phenyl-thiazol-4-yl)-N-(4-phenyl-4-piperidin-1-yl-cyclohexyl)-acetamid
5-Methyl-2-phenyl-2H-[1,2,3]triazol-4-carbonsäure [4-dimethylamino-4-(3-methyl-benzyl)-cyclohexyl]-amid
N-[4-Dimethylamino-4-(2-methyl-benzyl)-cyclohexyl]-2-(5-methyl-2-phenyl-thiazol-4-yl)-acetamid
1-(4-Chlor-phenyl)-5-trifluormethyl-1H-pyrazol-4-carbonsäure [4-(3-chlor-benzyl)-4-dimethylamino-cyclohexyl]-amid
5-Methyl-2-phenyl-2H-[1,2,3]triazol-4-carbonsäure [4-(3-chlor-benzyl)-4-dimethylamino-cyclohexyl]-amid
5-Methyl-2-phenyl-2H-[1,2,3]triazol-4-carbonsäure (4-dimethylamino-4-phenethyl-cyclohexyl)-amid
3-(2-Chlor-6-fluor-phenyl)-5-methyl-isoxazol-4-carbonsäure (4-benzyl-4-pyrrolidin-1-yl-cyclohexyl)-amid
N-[4-Dimethylamino-4-(2-fluor-benzyl)-cyclohexyl]-2-phenoxy-nicotinamid
N-[4-Dimethylamino-4-(3-fluor-benzyl)-cyclohexyl]-2-(5-methyl-2-phenyl-thiazol-4-yl)-acetamid
N-(4-Benzyl-4-dimethylamino-cyclohexyl)-2-phenoxy-nicotinamid
5-(Thiophen-2-sulfonyl)-4,5,6,7-tetrahydro-isoxazolo[4,5-c]pyridine-3-carbonsäure (4-benzyl-4-dimethylamino-cyclohexyl)-amid
1-(4-Chlor-phenyl)-5-trifluormethyl-1H-pyrazol-4-carbonsäure [4-dimethylamino-4-(2-fluorbenzyl)-cyclohexyl]-amid
5-Benzyl-furan-2-carbonsäure [4-(4-chlor-benzyl)-4-dimethylamino-cyclohexyl]-amid
5-Methyl-2-phenyl-2H-[1,2,3]triazol-4-carbonsäure (4-benzyl-4-dimethylamino-cyclohexyl)-amid
N-(4-Benzyl-4-dimethylamino-cyclohexyl)-2-(5-methyl-2-phenyl-thiazol-4-yl)-acetamid
N-[4-Dimethylamino-4-(2-fluor-benzyl)-cyclohexyl]-2-(5-methyl-2-phenyl-thiazol-4-yl)-acetamid
3-(2-Chlor-phenyl)-5-methyl-isoxazol-4-carbonsäure [4-dimethylamino-4-(2-fluor-benzyl)-cyclohexyl]-amid
5-Methyl-2-phenyl-2H-[1,2,3]triazol-4-carbonsäure [4-(2-chlor-benzyl)-4-dimethylamino-cyclohexyl]-amid
2-(4-Chlor-phenylsulfanyl)-N-[4-dimethylamino-4-(3-fluor-benzyl)-cyclohexyl]-nicotinamid
3-(2,6-Dichlor-phenyl)-5-methyl-isoxazol-4-carbonsäure (4-benzyl-4-dimethylamino-cyclohexyl)-amid
N-[4-(3-Chlor-benzyl)-4-dimethylamino-cyclohexyl]-2-phenoxy-nicotinamid
2-Phenoxy-N-(4-phenyl-4-piperidin-1-yl-cyclohexyl)-nicotinamid
5-(4-Chlor-phenyl)-2-methyl-furan-3-carbonsäure (4-benzyl-4-pyrrolidin-1-yl-cyclohexyl)-amid
5-(3-Trifluormethyl-benzylsulfonyl)-4,5,6,7-tetrahydro-isoxazolo[4,5-c]pyridine-3-carbonsäure (4-benzyl-4-dimethylamino-cyclohexyl)-amid
N-[4-Dimethylamino-4-(4-methyl-benzyl)-cyclohexyl]-2-phenoxy-nicotinamid
N-[4-Dimethylamino-4-(3-fluor-benzyl)-cyclohexyl]-2-phenoxy-nicotinamid
3-(2-Chlor-6-fluor-phenyl)-5-methyl-isoxazol-4-carbonsäure (4-phenyl-4-piperidin-1-yl-cyclohexyl)-amid
5-(4-Chlor-phenyl)-2-methyl-furan-3-carbonsäure [4-dimethylamino-4-(4-fluor-benzyl)-cyclohexyl]-amid
1-(4-Chlor-phenyl)-5-trifluormethyl-1H-pyrazol-4-carbonsäure [4-dimethylamino-4-(4-methylbenzyl)-cyclohexyl]-amid
5-Methyl-2-phenyl-2H-[1,2,3]triazol-4-carbonsäure [4-(4-chlor-benzyl)-4-dimethylamino-cyclohexyl]-amid
5-Methyl-2-phenyl-2H-[1,2,3]triazol-4-carbonsäure [4-dimethylamino-4-(3-fluor-benzyl)-cyclohexyl]-amid
3-(2,6-Dichlor-phenyl)-5-methyl-isoxazol-4-carbonsäure (4-phenyl-4-piperidin-1-yl-cyclohexyl)-amid
1-(4-Chlor-phenyl)-5-trifluormethyl-1H-pyrazol-4-carbonsäure (4-morpholin-4-yl-4-phenyl-cyclohexyl)-amid
N-[4-(4-Chlor-benzyl)-4-dimethylamino-cyclohexyl]-2-phenoxy-nicotinamid
N-[4-(4-Chlor-benzyl)-4-dimethylamino-cyclohexyl]-2-(5-methyl-2-phenyl-thiazol-4-yl)-acetamid
3-(2-Chlor-phenyl)-5-methyl-isoxazol-4-carbonsäure (4-azepan-1-yl-4-benzyl-cyclohexyl)-amid
3-(2-Chlor-6-fluor-phenyl)-5-methyl-isoxazol-4-carbonsäure [4-dimethylamino-4-(4-fluorbenzyl)-cyclohexyl]-amid
3-(2-Chlor-6-fluor-phenyl)-5-methyl-isoxazol-4-carbonsäure [4-(3-chlor-benzyl)-4-dimethylamino-cyclohexyl]-amid
3-(2-Chlor-phenyl)-5-methyl-isoxazol-4-carbonsäure [4-dimethylamino-4-(2-methyl-benzyl)-cyclohexyl]-amid
3-(2-Chlor-6-fluor-phenyl)-5-methyl-isoxazol-4-carbonsäure [4-dimethylamino-4-(2-methylbenzyl)-cyclohexyl]-amid
5-(1-Methyl-5-trifluormethyl-1H-pyrazol-3-yl)-thiophen-2-carbonsäure (4-benzyl-4-piperidin-1-yl-cyclohexyl)-amid
3-(2-Chlor-phenyl)-5-methyl-isoxazol-4-carbonsäure [4-dimethylamino-4-(4-fluor-benzyl)-cyclohexyl]-amid
3-(2,6-Dichlor-phenyl)-5-methyl-isoxazol-4-carbonsäure [4-dimethylamino-4-(4-methylbenzyl)-cyclohexyl]-amid
3-(2-Chlor-phenyl)-5-methyl-isoxazol-4-carbonsäure (4-benzyl-4-pyrrolidin-1-yl-cyclohexyl)-amid
3-(2-Chlor-6-fluor-phenyl)-5-methyl-isoxazol-4-carbonsäure [4-dimethylamino-4-(3-fluorbenzyl)-cyclohexyl]-amid
3-(2-Chlor-6-fluor-phenyl)-5-methyl-isoxazol-4-carbonsäure [4-dimethylamino-4-(4-methylbenzyl)-cyclohexyl]-amid
1-(4-Chlor-phenyl)-5-trifluormethyl-1H-pyrazol-4-carbonsäure [4-(4-chlor-benzyl)-4-dimethylamino-cyclohexyl]-amid
1-Phenyl-5-propyl-1H-pyrazol-4-carbonsäure (4-azepan-1-yl-4-benzyl-cyclohexyl)-amid
3-(2-Chlor-phenyl)-5-methyl-isoxazol-4-carbonsäure [4-dimethylamino-4-(3-fluor-benzyl)-cyclohexyl]-amid
1-Phenyl-5-propyl-1H-pyrazol-4-carbonsäure [4-(2-chlor-benzyl)-4-dimethylamino-cyclohexyl]-amid
3-(2-Chlor-6-fluor-phenyl)-5-methyl-isoxazol-4-carbonsäure (4-benzyl-4-dimethylamino-cyclohexyl)-amid
3-(2,6-Dichlor-phenyl)-5-methyl-isoxazol-4-carbonsäure [4-dimethylamino-4-(3-fluor-benzyl)-cyclohexyl]-amid
1-Phenyl-5-propyl-1H-pyrazol-4-carbonsäure [4-dimethylamino-4-(4-fluor-benzyl)-cyclohexyl]-amid
1-(4-Chlor-phenyl)-5-trifluormethyl-1H-pyrazol-4-carbonsäure (4-benzyl-4-dimethylamino-cyclohexyl)-amid
5-(1-Methyl-5-trifluormethyl-1H-pyrazol-3-yl)-thiophen-2-carbonsäure [4-dimethylamino-4-(3-fluor-benzyl)-cyclohexyl]-amid
3-(2-Chlor-6-fluor-phenyl)-5-methyl-isoxazol-4-carbonsäure [4-dimethylamino-4-(2-fluorbenzyl)-cyclohexyl]-amid
1-(4-Chlor-phenyl)-5-trifluormethyl-1H-pyrazol-4-carbonsäure (4-dimethylamino-4-phenethyl-cyclohexyl)-amid
5-(1-Methyl-5-trifluormethyl-1H-pyrazol-3-yl)-thiophen-2-carbonsäure [4-(3-chlor-benzyl)-4-dimethylamino-cyclohexyl]-amid
5-(1-Methyl-5-trifluormethyl-1H-pyrazol-3-yl)-thiophen-2-carbonsäure [4-dimethylamino-4-(2-fluor-benzyl)-cyclohexyl]-amid
5-(1-Methyl-5-trifluormethyl-1H-pyrazol-3-yl)-thiophen-2-carbonsäure [4-(4-chlor-benzyl)-4-dimethylamino-cyclohexyl]-amid
5-(1-Methyl-5-trifluormethyl-1H-pyrazol-3-yl)-thiophen-2-carbonsäure [4-dimethylamino-4-(4-methyl-benzyl)-cyclohexyl]-amid
5-Benzyl-furan-2-carbonsäure [4-dimethylamino-4-(4-methyl-benzyl)-cyclohexyl]-amid
5-Methyl-2-phenyl-2H-[1,2,3]triazol-4-carbonsäure (4-azepan-1-yl-4-benzyl-cyclohexyl)-amid
2-(4-Chlor-phenylsulfanyl)-N-[4-dimethylamino-4-(4-fluor-benzyl)-cyclohexyl]-nicotinamid
N-[4-Dimethylamino-4-(4-fluor-benzyl)-cyclohexyl]-2-phenoxy-nicotinamid
5-Benzyl-furan-2-carbonsäure (4-benzyl-4-piperidin-1-yl-cyclohexyl)-amid
3-(2,6-Dichlor-phenyl)-5-methyl-isoxazol-4-carbonsäure [4-(4-chlor-benzyl)-4-dimethylamino-cyclohexyl]-amid
N-(4-Benzyl-4-morpholin-4-yl-cyclohexyl)-2-(4-chlor-phenylsulfanyl)-nicotinamid
5-(1-Methyl-5-trifluormethyl-1H-pyrazol-3-yl)-thiophen-2-carbonsäure (4-benzyl-4-dimethylamino-cyclohexyl)-amid
1-(4-Chlor-phenyl)-5-trifluormethyl-1H-pyrazol-4-carbonsäure [4-dimethylamino-4-(4-fluorbenzyl)-cyclohexyl]-amid
1-(4-Chlor-phenyl)-5-trifluormethyl-1H-pyrazol-4-carbonsäure [4-dimethylamino-4-(2-methylbenzyl)-cyclohexyl]-amid
N-(4-Azepan-1-yl-4-benzyl-cyclohexyl)-2-phenoxy-nicotinamid
3-(2,6-Dichlor-phenyl)-5-methyl-isoxazol-4-carbonsäure [4-(2-chlor-benzyl)-4-dimethylamino-cyclohexyl]-amid
N-[4-Dimethylamino-4-(2-methyl-benzyl)-cyclohexyl]-2-phenoxy-nicotinamid
3-(2,6-Dichlor-phenyl)-5-methyl-isoxazol-4-carbonsäure [4-dimethylamino-4-(4-fluor-benzyl)-cyclohexyl]-amid
2-(4-Chlor-phenylsulfanyl)-N-[4-dimethylamino-4-(2-methyl-benzyl)-cyclohexyl]-nicotinamid
3-(2,6-Dichlor-phenyl)-5-methyl-isoxazol-4-carbonsäure [4-dimethylamino-4-(2-methylbenzyl)-cyclohexyl]-amid
3-(2-Chlorphenyl)-5-methylisoxazol-4-carbonsäure [4-dimethylamino-4-(3-fluorphenyl)cyclohexyl]amid Hydrochlorid, unpolareres Diastereoisomer
3-(2-Chlorphenyl)-5-methylisoxazol-4-carbonsäure [4-dimethylamino-4-(3-fluorphenyl)cyclohexyl]amid Hydrochlorid, polareres Diastereoisomer
5-Methyl-2-phenyl-2H-[1,2,3]triazol-4-carbonsäure (4-dimethylamino-4-phenylcyclohexyl)-amid Hydrochlorid, unpolareres Diastereoisomer
5-Methyl-2-phenyl-2H-[1,2,3]triazol-4-carbonsäure (4-dimethylamino-4-phenylcyclohexyl)-amid Hydrochlorid, polareres Diastereoisomer
3-(2,6-Dichlorphenyl)-5-methylisoxazol-4-carbonsäure [4-dimethylamino-4-(3-fluorphenyl)-cyclohexyl]amid Hydrochlorid, unpolareres Diastereoisomer
3-(2,6-Dichlorphenyl)-5-methylisoxazol-4-carbonsäure [4-dimethylamino-4-(3-fluorphenyl)-cyclohexyl]amid Hydrochlorid, polareres Diastereoisomer
3-(2,6-Dichlorphenyl)-5-methylisoxazol-4-carbonsäure (4-dimethylamino-4-phenylcyclohexyl)amid Hydrochlorid, unpolareres Diastereoisomer
3-(2,6-Dichlorphenyl)-5-methylisoxazol-4-carbonsäure (4-dimethylamino-4-phenylcyclohexyl)amid Hydrochlorid, polareres Diastereoisomer
5-Methyl-3-phenylisoxazol-4-carbonsäure [4-dimethylamino-4-(3-fluorphenyl)cyclohexyl]amid Hydrochlorid, unpolareres Diastereoisomer
5-Methyl-3-phenylisoxazol-4-carbonsäure (4-dimethylamino-4-phenylcyclohexyl)amid Hydrochlorid, unpolareres Diastereoisomer
5-Methyl-3-phenylisoxazol-4-carbonsäure (4-dimethylamino-4-phenylcyclohexyl)amid Hydrochlorid, polareres Diastereoisomer
in Form des Razemats; der Enantiomere, Diastereomere, Mischungen der Enantiomere oder Diastereomere oder eines einzelnen Enantiomers oder Diastereomers; der Basen und/oder Salze physiologisch verträglicher Säuren oder Kationen.

Die erfindungsgemäßen Substanzen wirken beispielsweise auf den im Zusammenhang mit verschiedenen Erkrankungen relevanten ORL-1-Rezeptor, sodass sie sich als pharmazeutischer Wirkstoff in einem Arzneimittel eignen. Ein weiterer Gegenstand der Erfindung sind daher Arzneimittel enthaltend wenigstens ein erfindungsgemäßes substituiertes Cyclohexycarbonsäure-Derivat, sowie gegebenenfalls geeignete Zusatz- und/oder Hilfsstoffe und/oder gegebenenfalls weitere Wirkstoffe.

Die erfindungsgemäßen Arzneimittel enthalten neben mindestens einem erfindungsgemäßen substituierten Cyclohexyl-1,4-diamin-Derivat gegebenenfalls geeignete Zusatz- und/oder Hilfsstoffe, so auch Trägermaterialien, Füllstoffe, Lösungsmittel, Verdünnungsmittel, Farbstoffe und/oder Bindemittel und können als flüssige Arzneiformen in Form von Injektionslösungen, Tropfen oder Säfte, als halbfeste Arzneiformen in Form von Granulaten, Tabletten, Pellets, Patches, Kapseln, Pflaster/Sprühpflaster oder Aerosolen verabreicht werden. Die Auswahl der Hilfsstoffe etc. sowie die einzusetzenden Mengen derselben hängen davon ab, ob das Arzneimittel oral, peroral, parenteral, intravenös, intraperitoneal, intradermal, intramuskulär, intranasal, buccal, rektal oder örtlich, zum Beispiel auf die Haut, die Schleimhäute oder in die Augen, appliziert werden soll. Für die orale Applikation eignen sich Zubereitungen in Form von Tabletten, Dragees, Kapseln, Granulaten, Tropfen, Säften und Sirupen, für die parenterale, topische und inhalative Applikation Lösungen, Suspensionen, leicht rekonstituierbare Trockenzubereitungen sowie Sprays. Erfindungsgemäße substituierte Cyclohexyl-1,4-diamin -Derivate in einem Depot, in gelöster Form oder in einem Pflaster, gegebenenfalls unter Zusatz von die Hautpenetration fördernden Mitteln, sind geeignete perkutane Applikationszubereitungen. Oral oder perkutan anwendbare Zubereitungsformen können die erfindungsgemäßen substituierten Cyclohexyl-1,4-diamin -Derivate verzögert freisetzen. Die erfindungsgemäßen substituierten Cyclohexyl-1,4-diamin -Derivate können auch in parenteralen Langzeitdepotformen wie z. B. Implantaten oder implantierten Pumpen angewendet werden. Prinzipiell können den erfindungsgemäßen Arzneimitteln andere dem Fachmann bekannte weitere Wirkstoffe zugesetzt werden.

Die an den Patienten zu verabreichende Wirkstoffmenge variiert in Abhängigkeit vom Gewicht des Patienten, von der Applikationsart, der Indikation und dem Schweregrad der Erkrankung. Üblicherweise werden 0,00005 bis 50 mg/kg, bevorzugt 0,01 bis 5 mg/kg wenigstens eines erfindungsgemäßen substituierten Cyclohexyl-1,4-diamin-Derivats appliziert.

Für alle vorstehenden Formen der erfindungsgemäßen Arzneimittel ist es besonders bevorzugt, wenn das Arzneimittel neben wenigstens einem substituierten Cyclohexyl-1,4-diamin-Derivat noch einen weiteren Wirkstoff, insbesondere ein Opioid, vorzugsweise ein starkes Opioid, insbesondere Morphin, oder ein Anesthetikum, vorzugsweise Hexobarbital oder Halothan, enthält.

In einer bevorzugten Form des Arzneimittel liegt ein enthaltenes erfindungsgemäßes substituiertes Cyclohexyl-1,4-diamin-Derivat als reines Diastereomer und/oder Enantiomer, als Razemat oder als nicht-äquimolare oder äquimolare Mischung der Diastereomere und/oder Enantiomere vor.

Der ORL-1-Rezeptor, aber auch die anderen Opioid-Rezeptoren, wurden insbesondere im Schmerzgeschehen identifiziert. Entsprechend können erfindungsgemäße substituierte Cyclohexyl-1,4-diamin-Derivate zur Herstellung eines Arzneimittels zur Behandlung von Schmerz, insbesondere von akutem, neuropathischem oder chronischem Schmerz, verwendet werden.

Ein weiterer Gegenstand der Erfindung ist daher die Verwendung eines erfindungsgemäßen substituierten Cyclohexyl-1,4-diamin-Derivats zur Herstellung eines Arzneimittels zur Behandlung von Schmerz, insbesondere von akutem, viszeralem, neuropathischem oder chronischem Schmerz.

Ein weiterer Gegenstand der Erfindung ist die Verwendung eines erfindungsgemäßen substituierten Cyclohexyl-1,4-diamin-Derivats zur Herstellung eines Arzneimittels zur Behandlung von Angstzuständen, von Stress und mit Stress verbundenen Syndromen, Depressionen, Epilepsie, Alzheimer Erkrankung, seniler Demenz, allgemeinen kognitiven Dysfunktionen, Lern- und Gedächtnis-Störungen (als Nootropikum), Entzugserscheinungen, Alkohol- und/oder Drogen- und/oder Medikamentenmißbrauch und/oder -abhängigkeit, sexuellen Dysfunktionen, cardiovaskulären Erkrankungen, Hypotension, Hypertension, Tinitus, Pruritus, Migräne, Schwerhörigkeit, mangelnder Darmmotilität, gestörter Nahrungsaufnahme, Anorexie, Fettsucht, lokomotorischen Störungen, Diarrhoe, Kachexie, Harninkontinenz bzw. als Muskelrelaxanz, Antikonvulsivum oder Anesthetikum bzw. zur Coadministration bei Behandlung mit einem opioiden Analgetikum oder mit einem Anesthetikum, zur Diurese oder Antinatriurese, Anxiolyse, zur Modulation der Bewegungsaktivität, zur Modulation der Neurotransmitter-Ausschüttung und Behandlung damit verbundener neurodegenerativer Erkrankungen, zur Behandlung von Entzugserscheinungen und/oder zur Reduzierung des Suchtpotentials von Opioiden.

Dabei kann es in einer der vorstehenden Verwendungen bevorzugt sein, wenn ein verwendetes substituiertes Cyclohexyl-1,4-diamin-Derivat als reines Diastereomer und/oder Enantiomer, als Razemat oder als nicht-äquimolare oder äquimolare Mischung der Diastereomere und/oder Enantiomere vorliegt.

Ein weiterer Gegenstand der Erfindung ist ein Verfahren zur Behandlung, insbesondere in einer der vorgenannten Indikationen, eines nichthumanen Säugetieres oder Menschen, das oder der eine Behandlung von Schmerzen, insbesondere chronischer Schmerzen, benötigt, durch Verabreichung einer therapeutisch wiksamen Dosis eines erfindungsgemäßen substituierten Cyclohexyl-1,4-diamin-Derivats, oder eines erfindungsgemäßen Arzneimittels.
Ein weiterer Gegenstand der Erfindung ist ein Verfahren zur Herstellung der erfindungsgemäßen substituierten Cyclohexyl-1,4-diamin-Derivate wie in der folgenden Beschreibung und Beispielen ausgeführt.
Die Reste R⁰¹ und R⁰² haben die für erfindungsgemäße Verbindungen gemäß Formel I für R¹ und R² angegebene Bedeutung und können zusätzlich unabhängig voneinander für eine Schutzgruppe stehen. Die übrigen Reste haben die in Formel I angegebene Bedeutung:

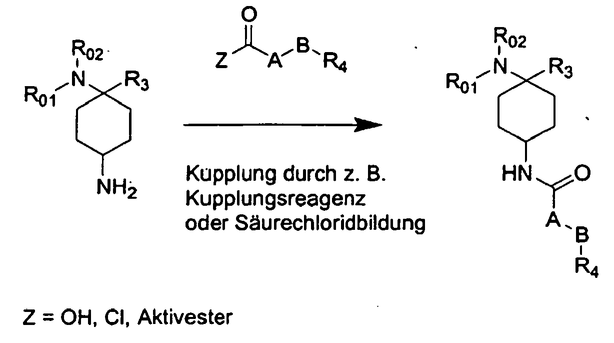

Zur Darstellung der erfindungsgemäßen Cyclohexan-1,4-diamine sind grundsätzlich die vielfältigen, dem Fachmann bekannten Methoden zur Herstellung von Amiden geeignet.

Das erfindungsgemäße Verfahren beruht darauf, substituierte Cyclohexan-1,4-diamine, deren Herstellung aus der Literatur bekannt ist (WO 02090317), mit Anhydriden, Carbonsäuren oder vorzugsweise deren aktivierte Analoga wie insbesondere deren Säurehalogenide oder Aktivester zu verknüpfen und so in erfindungsgemäße Verbindungen zu überführen.

Die Umsetzungen mit Anhydriden finden vorzugsweise in polaren oder unpolaren aprotischen Lösungsmitteln wie DMF, DMSO, Diethylether, Diisopropylether, THF, Toluol, Dichlormethan oder Acetonitril bei Temperaturen zwischen -20 und +110 °C, bevorzugt -10 und +40 °C statt.

Bei Umsetzungen mit Säurechloriden werden ebenfalls polaren oder unpolaren aprotischen Lösungsmitteln eingesetzt, denen eine organische oder anorganische Hilfsbase, vorzugsweise tertiäre Amine wie Triethylamin, Diisopropylethylamin oder DMAP, zugesetzt wurde. Neben solchen Aminen ist auch beispielsweise Pyridin als Base und als Lösungsmittel geeignet. Vorzugsweise werden Säurechloride mit Aminen zwischen -10 und +40 °C in Dichlormethan oder Chloroform in Gegenwart von Triethylamin oder Pyridin und ggf. katalytischer Mengen DMAP umgesetzt.

Für die Umsetzung der Carbonsäurefunktion mit einem Amin steht die gesamte Bandbreite der dem Fachmann bekannten Methoden zur Herstellung von Amiden zur Verfügung. Vorteilhaft ist dabei der Einsatz organischer oder anorganischer wasserentziehender Mittel wie z.B. Molsieb, Magnesiumsulfat, Schwefelsäure oder Carbodiimiden wie DCC oder DIC, letztere ggf. in Gegenwart von HOBt. Auch diese Umsetzungen werden vorzugsweise in polaren oder unpolaren aprotischen Lösungsmitteln bei Temperaturen zwischen -20 und +110 °C, bevorzugt ―10 und +40 °C durchgeführt.

Gegebenenfalls werden anschließend die Schutzgruppen abgespalten.

### Beispiele

Die folgenden Beispiele dienen zur näheren Erläuterung der Erfindung, schränken aber den allgemeinen Erfindungsgedanken nicht ein.

Die Ausbeuten der hergestellten Verbindungen sind nicht optimiert.

Alle Temperaturen sind unkorrigiert.

Die Angabe "Ether" bedeutet Diethylether, "EE" Ethylacetat und "DCM" Dichlormethan. Die Angabe "Äquivalente" bedeutet Stoffmengenäquivalente, "Smp." Schmelzpunkt bzw. Schmelzbereich, "Zers." Zersetzung, "RT" Raumtemperatur, "abs." absolut (wasserfrei), ,"rac." racemisch , "konz." konzentriert, "min" Minuten, "h" Stunden, "d" Tage, "Vol.%" Volumenprozent, "m%" Massenprozent und "M" ist eine Konzentrationsangabe in mol/l.

Als stationäre Phase für die Säulenchromatographie wurde Kieselgel 60 (0.040 - 0.063 mm) der Firma E. Merck, Darmstadt, eingesetzt.

Die dünnschicht-chromatographischen Untersuchungen wurden mit HPTLC-Fertigplatten, Kieselgel 60 F 254, der Firma E. Merck, Darmstadt, durchgeführt.

Die Mischungsverhältnisse von Laufmitteln für chromatographische Untersuchungen sind stets in Volumen/Volumen angegeben.

Die im folgenden eingesetzten Verbindungen waren entweder kommerziell erhältlich, oder ihre Herstellung ist aus dem Stand der Technik bekannt oder in für den Fachmann offensichtlicher Weise aus dem Stand der Technik abgeleitet worden.

### Allgemeine Vorschrift:

Zu 0,1 mmol des Cyclohexan-1,4-diamins wurden 0,1 mmol eines Säurechlorids, das aus den entsprechenden Carbonsäuren nach dem Fachmann bekannten Methoden hergestellt wurde (s. Tabelle 1), in Gegenwart von 1,05 Equivalenten Triethylamin zugegeben. Es wurde 12 h gerührt und anschließend mit einer 1M Natriumcarbonatlösung versetzt. Durch Extraktion mit je 3 x 2 ml Dichlormethan und Entfernen des Lösungsmittels wurde das Produkt erhalten.

In Tabelle 1 sind die für den letzten Schritt eingesetzten Carbonsäuren für die Beispiele genannt.

**Tabelle 1: Auflistung der Beispiele und Abbildung der im letzten Syntheseschritt eingesetzten Carbonsäure.**

| **Beispiel** | **eingesetzte Carbonsäure** | **Name der Beispielverbindung** |
|---|---|---|
| **1** | 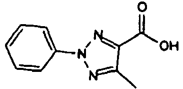 | 5-Methyl-2-phenyl-2H-[1,2,3]triazol-4-carbonsäure (4-dimethylamino-4-phenyl-cyclohexyl)-amid |
| **2** | 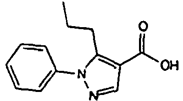 | 1-Phenyl-5-propyl-1H-pyrazol-4-carbonsäure [4-dimethylamino-4-(3-fluor-phenyl)-cyclohexyl]-amid |
| **3** | 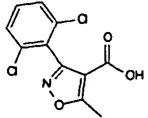 | 3-(2,6-Dichlor-phenyl)-5-methyl-isoxazol-4-carbonsäure (4-benzyl-4-pyrrolidin-1-yl-cyclohexyl)-amid |
| **4** | 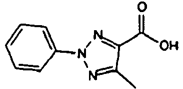 | 5-Methyl-2-phenyl-2H-[1,2,3]triazol-4-carbonsäure (4-benzyl-4-piperidin-1-yl-cyclohexyl)-amid |
| **5** | 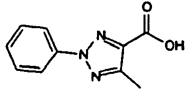 | 5-Methyl-2-phenyl-2H-[1,2,3]triazol-4-carbonsäure (4-phenyl-4-piperidin-1-yl-cyclohexyl)-amid |
| **6** | 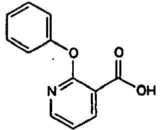 | N-(4-Dimethylamino-4-phenyl-cyclohexyl)-2-phenoxy-nicotinamid |
| **7** | 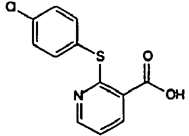 | 2-(4-Chlor-phenylsulfanyl)-N-(4-dimethylamino-4-pyridin-2-yl-cyclohexyl)-nicotinamid |
| **8** | 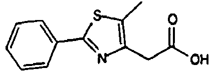 | N-(4-Dimethylamino-4-thiophen-2-yl-cyclohexyl)-2-(5-methyl-2-phenyl-thiazol-4-yl)-acetamid |
| **9** | 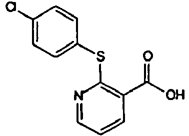 | 2-(4-Chlor-phenylsulfanyl)-N-[4-dimethylamino-4-(3-fluor-phenyl)-cyclohexyl]-nicotinamid |
| **10** | 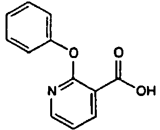 | N-(4-Dimethylamino-4-thiophen-2-yl-cyclohexyl)-2-phenoxy-nicotinamid |
| **11** | 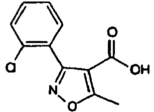 | 3-(2-Chlor-phenyl)-5-methyl-isoxazol-4-carbonsäure (4-dimethylamino-4-phenyl-cyclohexyl)-amid |
| **12** | 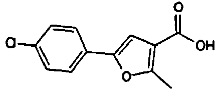 | 5-(4-Chlor-phenyl)-2-methyl-furan-3-carbonsäure [4-dimethylamino-4-(3-fluor-phenyl)-cyclohexyl]-amid |
| **13** | 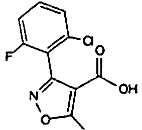 | 3-(2-Chlor-6-fluor-phenyl)-5-methyl-isoxazol-4-carbonsäure (4-dimethylamino-4-phenethyl-cyclohexyl)-amid |
| **14** | 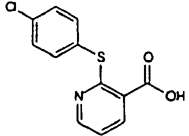 | N-(4-Benzyl-4-pyrrolidin-1-yl-cyclohexyl)-2-(4-chlor-phenylsulfanyl)-nicotinamid |
| **15** | 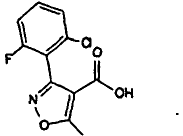 | 3-(2-Chlor-6-fluor-phenyl)-5-methyl-isoxazol-4-carbonsäure (4-dimethylamino-4-phenyl-cyclohexyl)-amid |
| **16** | 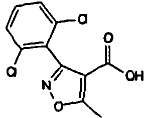 | 3-(2,6-Dichlor-phenyl)-5-methyl-isoxazol-4-carbonsäure (4-benzyl-4-piperidin-1-yl-cyclohexyl)-amid |
| **17** | 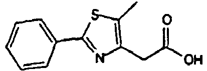 | N-(4-Dimethylamino-4-phenethyl-cyclohexyl)-2-(5-methyl-2-phenyl-thiazol-4-yl)-acetamid |
| **18** | 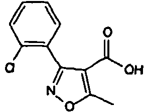 | 3-(2-Chlor-phenyl)-5-methyl-isoxazol-4-carbonsäure (4-dimethylamino-4-phenethyl-cyclohexyl)-amid |
| **19** | 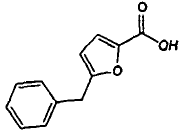 | 5-Benzyl-furan-2-carbonsäure (4-benzyl-4-dimethylamino-cyclohexyl)-amid |
| **20** | 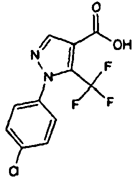 | 1-(4-Chlor-phenyl)-5-trifluormethyl-1H-pyrazol-4-carbonsäure [4-dimethylamino-4-(3-fluor-phenyl)-cyclohexyl]-amid |
| **21** | 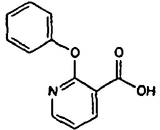 | N-(4-Dimethylamino-4-pyridin-2-yl-cyclohexyl)-2-phenoxy-nicotinamid |
| **22** | 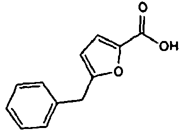 | 5-Benzyl-furan-2-carbonsäure (4-phenyl-4-piperidin-1-yl-cyclohexyl)-amid |
| **23** | 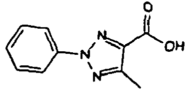 | 5-Methyl-2-phenyl-2H-[1,2,3]triazol-4-carbonsäure [4-dimethylamino-4-(2-methyl-benzyl)-cyclohexyl]-amid |
| **24** | 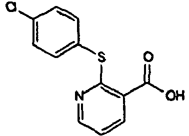 | 2-(4-Chlor-phenylsulfanyl)-N-[4-dimethylamino-4-(2-fluor-benzyl)-cyclohexyl]-nicotinamid |
| **25** | 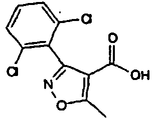 | 3-(2,6-Dichlor-phenyl)-5-methyl-isoxazol-4-carbonsäure [4-dimethylamino-4-(2-fluor-benzyl)-cyclohexyl]-amid |
| **26** | 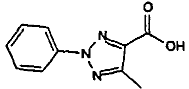 | 5-Methyl-2-phenyl-2H-[1,2,3]triazol-4-carbonsäure [4-dimethylamino-4-(4-fluor-benzyl)-cyclohexyl]-amid |
| **27** | 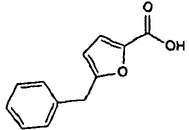 | 5-Benzyl-furan-2-carbonsäure [4-dimethylamino-4-(2-fluor-benzyl)-cyclohexyl]-amid |
| **28** | 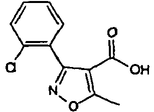 | 3-(2-Chlor-phenyl)-5-methyl-isoxazol-4-carbonsäure [4-dimethylamino-4-(3-fluor-phenyl)-cyclohexyl]-amid |
| **29** | 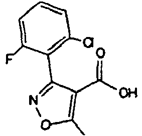 | 3-(2-Chlor-6-fluor-phenyl)-5-methyl-isoxazol-4-carbonsäure [4-dimethylamino-4-(3-fluor-phenyl)-cyclohexyl]-amid |
| **30** | 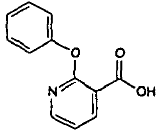 | N-(4-Dimethylamino-4-phenethyl-cyclohexyl)-2-phenoxy-nicotinamid |
| **31** | 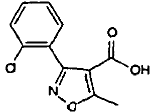 | 3-(2-Chlor-phenyl)-5-methyl-isoxazol-4-carbonsäure [4-(3-chlor-benzyl)-4-dimethylamino-cyclohexyl]-amid |
| **32** | 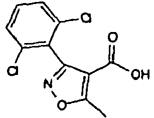 | 3-(2,6-Dichlor-phenyl)-5-methyl-isoxazol-4-carbonsäure [4-dimethylamino-4-(3-fluor-phenyl)-cyclohexyl]-amid |
| **33** | 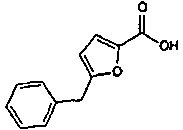 | 5-Benzyl-furan-2-carbonsäure (4-dimethylamino-4-phenethyl-cyclohexyl)-amid |
| **34** | 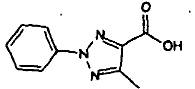 | 5-Methyl-2-phenyl-2H-[1,2,3]triazol-4-carbonsäure [4-dimethylamino-4-(4-methyl-benzyl)-cyclohexyl]-amid |
| **35** | 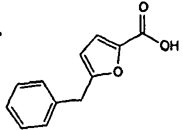 | 5-Benzyl-furan-2-carbonsäure [4-dimethylamino-4-(2-methyl-benzyl)-cyclohexyl]-amid |
| **36** | 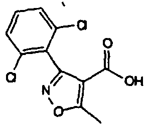 | 3-(2,6-Dichlor-phenyl)-5-methyl-isoxazol-4-carbonsäure (4-dimethylamino-4-phenethyl-cyclohexyl)-amid |
| **37** | 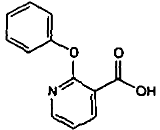 | N-(4-Benzyl-4-pyrrolidin-1-yl-cyclohexyl)-2-phenoxy-nicotinamid |
| **38** | 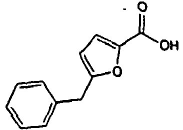 | 5-Benzyl-furan-2-carbonsäure [4-dimethylamino-4-(3-methyl-benzyl)-cyclohexyl]-amid |
| **39** | 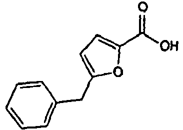 | 5-Benzyl-furan-2-carbonsäure [4-dimethylamino-4-(3-fluor-benzyl)-cyclohexyl]-amid |
| **40** | 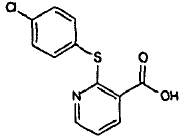 | N-[4-(3-Chlor-benzyl)-4-dimethylamino-cyclohexyl]-2-(4-chlor-phenylsulfanyl)-nicotinamid |
| **41** | 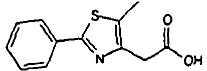 | 2-(5-Methyl-2-phenyl-thiazol-4-yl)-N-(4-phenyl-4-piperidin-1-yl-cyclohexyl)-acetamid |
| **42** | 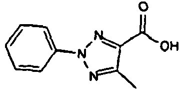 | 5-Methyl-2-phenyl-2H-[1,2,3]triazol-4-carbonsäure [4-dimethylamino-4-(3-methyl-benzyl)-cyclohexyl]-amid |
| **43** | 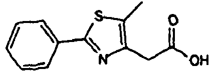 | N-[4-Dimethylamino-4-(2-methyl-benzyl)-cyclohexyl]-2-(5-methyl-2-phenyl-thiazol-4-yl)-acetamid |
| **44** | 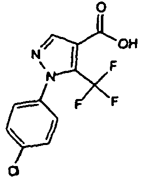 | 1-(4-Chlor-phenyl)-5-trifluormethyl-1H-pyrazol-4-carbonsäure [4-(3-chlor-benzyl)-4-dimethylamino-cyclohexyl]-amid |
| **45** | 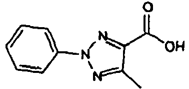 | 5-Methyl-2-phenyl-2H-[1,2,3]triazol-4-carbonsäure [4-(3-chlor-benzyl)-4-dimethylamino-cyclohexyl]-amid |
| **46** | 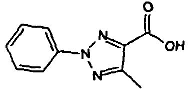 | 5-Methyl-2-phenyl-2H-[1,2,3]triazol-4-carbonsäure (4-dimethylamino-4-phenethyl-cyclohexyl)-amid |
| **47** | 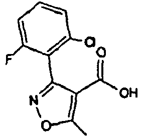 | 3-(2-Chlor-6-fluor-phenyl)-5-methyl-isoxazol-4-carbonsäure (4-benzyl-4-pyrrolidin-1-yl-cyclohexyl)-amid |
| **48** | 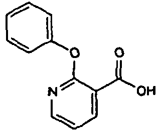 | N-[4-Dimethylamino-4-(2-fluor-benzyl)-cyclohexyl]-2-phenoxy-nicotinamid |
| **49** | 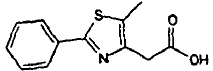 | N-[4-Dimethylamino-4-(3-fluor-benzyl)-cyclohexyl]-2-(5-methyl-2-phenyl-thiazol-4-yl)-acetamid |
| **50** | 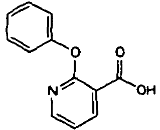 | N-(4-Benzyl-4-dimethylamino-cyclohexyl)-2-phenoxy-nicotinamid |
| **51** | 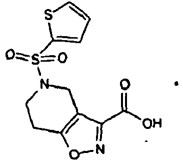 | 5-(Thiophen-2-sulfonyl)-4,5,6,7-tetrahydro-isoxazolo[4,5-c]pyridine-3-carbonsäure (4-benzyl-4-dimethylamino-cyclohexyl)-amid |
| **52** | 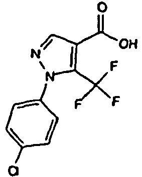 | 1-(4-Chlor-phenyl)-5-trifluormethyl-1H-pyrazol-4-carbonsäure [4-dimethylamino-4-(2-fluor-benzyl)-cyclohexyl]-amid |
| **53** | 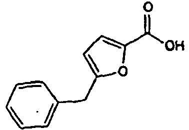 | 5-Benzyl-furan-2-carbonsäure [4-(4-chlor-benzyl)-4-dimethylamino-cyclohexyl]-amid |
| **54** | 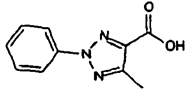 | 5-Methyl-2-phenyl-2H-[1,2,3]triazol-4-carbonsäure (4-benzyl-4-dimethylamino-cyclohexyl)-amid |
| **55** | 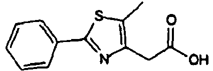 | N-(4-Benzyl-4-dimethylamino-cyclohexyl)-2-(5-methyl-2-phenyl-thiazol-4-yl)-acetamid |
| **56** | 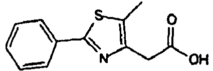 | N-[4-Dimethylamino-4-(2-fluor-benzyl)-cyclohexyl]-2-(5-methyl-2-phenyl-thiazol-4-yl)-acetamid |
| **57** | 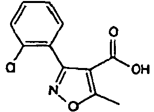 | 3-(2-Chlor-phenyl)-5-methyl-isoxazol-4-carbonsäure [4-dimethylamino-4-(2-fluor-benzyl)-cyclohexyl]-amid |
| **58** | 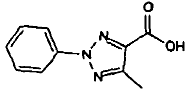 | 5-Methyl-2-phenyl-2H-[1,2,3]triazol-4-carbonsäure [4-(2-chlor-benzyl)-4-dimethylamino-cyclohexyl]-amid |
| **59** | 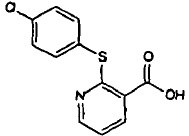 | 2-(4-Chlor-phenylsulfanyl)-N-[4-dimethylamino-4-(3-fluor-benzyl)-cyclohexyl]-nicotinamid |
| **60** | 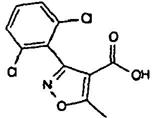 | 3-(2,6-Dichlor-phenyl)-5-methyl-isoxazol-4-carbonsäure (4-benzyl-4-dimethylamino-cyclohexyl)-amid |
| **61** | 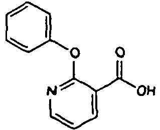 | N-[4-(3-Chlor-benzyl)-4-dimethylamino-cyclohexyl]-2-phenoxy-nicotinamid |
| **62** | 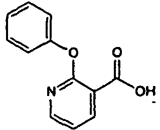 | 2-Phenoxy-N-(4-phenyl-4-piperidin-1-yl-cyclohexyl)-nicotinamid |
| **63** | 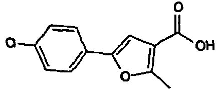 | 5-(4-Chlor-phenyl)-2-methyl-furan-3-carbonsäure (4-benzyl-4-pyrrolidin-1-yl-cyctohexyl)-amid |
| **64** | 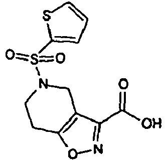 | 5-(3-Trifluormethyl-benzylsulfonyl)-4,5,6,7-tetrahydro-isoxazolo[4,5-c]pyridine-3-carbonsäure (4-benzyl-4-dimethylamino-cyclohexyl)-amid |
| **65** | 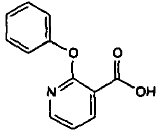 | N-[4-Dimethylamino-4-(4-methyl-benzyl)-cyclohexyl]-2-phenoxy-nicotinamid |
| **66** | 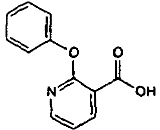 | N-[4-Dimethylamino-4-(3-fluor-benzyl)-cyclohexyl]-2-phenoxy-nicotinamid |
| **67** | 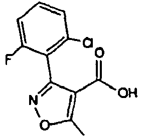 | 3-(2-Chlor-6-fluor-phenyl)-5-methyl-isoxazol-4-carbonsäure (4-phenyl-4-piperidin-1-yl-cyclohexyl)-amid |
| **68** | 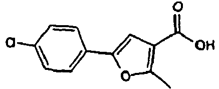 | 5-(4-Chlor-phenyl)-2-methyl-furan-3-carbonsäure [4-dimethylamino-4-(4-fluor-benzyl)-cyclohexyl]-amid |
| **69** | 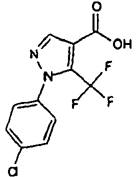 | 1-(4-Chlor-phenyl)-5-trifluormethyl-1H-pyrazol-4-carbonsäure [4-dimethylamino-4-(4-methyl-benzyl)-cyclohexyl]-amid |
| **70** | 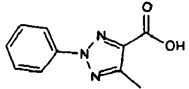 | 5-Methyl-2-phenyl-2H-[1,2,3]triazol-4-carbonsäure [4-(4-chlor-benzyl)-4-dimethylamino-cyclohexyl]-amid |
| **71** | 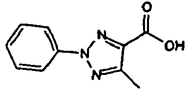 | 5-Methyl-2-phenyl-2H-[1,2,3]triazol-4-carbonsäure [4-dimethylamino-4-(3-fluor-benzyl)-cyclohexyl]-amid |
| **72** | 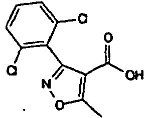 | 3-(2,6-Dichlor-phenyl)-5-methyl-isoxazol-4-carbonsäure (4-phenyl-4-piperidin-1-yl-cyclohexyl)-amid |
| **73** | 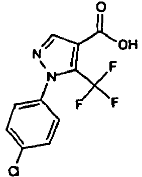 | 1-(4-Chlor-phenyl)-5-trifluormethyl-1H-pyrazol-4-carbonsäure (4-morpholin-4-yl-4-phenyl-cyclohexyl)-amid |
| **74** | 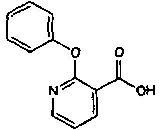 | N-[4-(4-Chlor-benzyl)-4-dimethylamino-cyclohexyl]-2-phenoxy-nicotinamid |
| **75** | 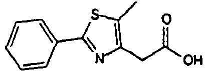 | N-[4-(4-Chlor-benzyl)-4-dimethylamino-cyclohexyl]-2-(5-methyl-2-phenyl-thiazol-4-yl)-acetamid |
| **76** | 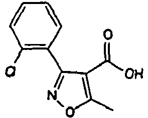 | 3-(2-Chlor-phenyl)-5-methyl-isoxazol-4-carbonsäure (4-azepan-1-yl-4-benzyl-cyclohexyl)-amid |
| **77** | 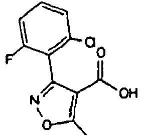 | 3-(2-Chlor-6-fluor-phenyl)-5-methyl-isoxazol-4-carbonsäure [4-dimethylamino-4-(4-fluor-benzyl)-cyclohexyl]-amid |
| **78** | 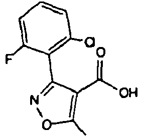 | 3-(2-Chlor-6-fluor-phenyl)-5-methyl-isoxazol-4-carbonsäure [4-(3-chlor-benzyl)-4-dimethylamino-cyclohexyl]-amid |
| **79** | 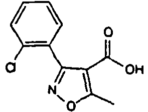 | 3-(2-Chlor-phenyl)-5-methyl-isoxazol-4-carbonsäure [4-dimethylamino-4-(2-methyl-benzyl)-cyclohexyl]-amid |
| **80** | 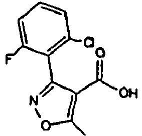 | 3-(2-Chlor-6-fluor-phenyl)-5-methyl-isoxazol-4-carbonsäure [4-dimethylamino-4-(2-methyl-benzyl)-cyclohexyl]-amid |
| **81** | 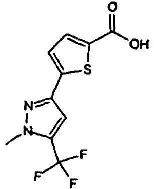 | 5-(1-Methyl-5-trifluormethyl-1H-pyrazol-3-yl)-thiophen-2-carbonsäure (4-benzyl-4-piperidin-1-yl-cyclohexyl)-amid |
| **82** | 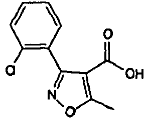 | 3-(2-Chlor-phenyl)-5-methyl-isoxazol-4-carbonsäure [4-dimethylamino-4-(4-fluor-benzyl)-cyclohexyl]-amid |
| **83** | 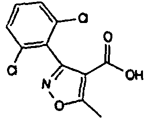 | 3-(2,6-Dichlor-phenyl)-5-methyl-isoxazol-4-carbonsäure [4-dimethylamino-4-(4-methyl-benzyl)-cyclohexyl]-amid |
| **84** | 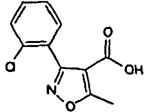 | 3-(2-Chlor-phenyl)-5-methyl-isoxazol-4-carbonsäure (4-benzyl-4-pyrrolidin-1-yl-cyclohexyl)-amid |
| **85** | 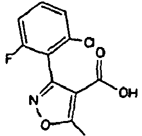 | 3-(2-Chlor-6-fluor-phenyl)-5-methyl-isoxazol-4-carbonsäure [4-dimethylamino-4-(3-fluor-benzyl)-cyclohexyl]-amid |
| **86** | 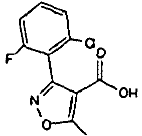 | 3-(2-Chlor-6-fluor-phenyl)-5-methyl-isoxazol-4-carbonsäure [4-dimethylamino-4-(4-methyl-benzyl)-cyclohexyl]-amid |
| **87** | 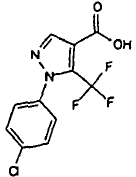 | 1-(4-Chlor-phenyl)-5-trifluormethyl-1H-pyrazol-4-carbonsäure [4-(4-chlor-benzyl)-4-dimethylamino-cyclohexyl]-amid |
| **88** | 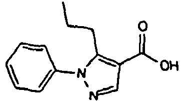 | 1-Phenyl-5-propyl-1H-pyrazol-4-carbonsäure (4-azepan-1-yl-4-benzyl-cyclohexyl)-amid |
| **89** | 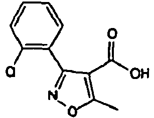 | 3-(2-Chlor-phenyl)-5-methyl-isoxazol-4-carbonsäure [4-dimethylamino-4-(3-fluor-benzyl)-cyclohexyl]-amid |
| **90** | 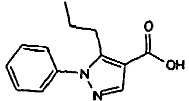 | 1-Phenyl-5-propyl-1H-pyrazol-4-carbonsäure [4-(2-chlor-benzyl)-4-dimethylamino-cyclohexyl]-amid |
| **91** | 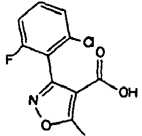 | 3-(2-Chlor-6-fluor-phenyl)-5-methyl-isoxazol-4-carbonsäure (4-benzyl-4-dimethylamino-cyclohexyl)-amid |
| **92** | 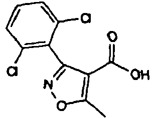 | 3-(2,6-Dichlor-phenyl)-5-methyl-isoxazol-4-carbonsäure [4-dimethylamino-4-(3-fluor-benzyl)-cyclohexyl]-amid |
| **93** | 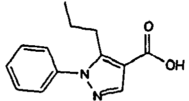 | 1-Phenyl-5-propyl-1H-pyrazol-4-carbonsäure [4-dimethylamino-4-(4-fluor-benzyl)-cyclohexyl]-amid |
| **94** | 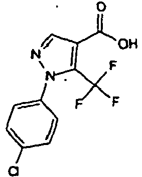 | 1-(4-Chlor-phenyl)-5-trifluormethyl-1H-pyrazol-4-carbonsäure (4-benzyl-4-dimethylamino-cyclohexyl)-amid |
| **95** | 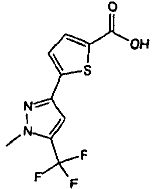 | 5-(1-Methyl-5-trifluormethyl-1H-pyrazol-3-yl)-thiophen-2-carbonsäure [4-dimethylamino-4-(3-fluor-benzyl)-cyclohexyl]-amid |
| **96** | 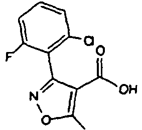 | 3-(2-Chlor-6-fluor-phenyl)-5-methyl-isoxazol-4-carbonsäure [4-dimethylamino-4-(2-fluor-benzyl)-cyclohexyl]-amid |
| **97** | 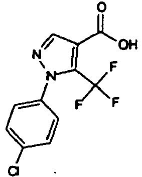 | 1-(4-Chlor-phenyl)-5-trifluormethyl-1H-pyrazol-4-carbonsäure (4-dimethylamino-4-phenethyl-cyclohexyl)-amid |
| **98** | 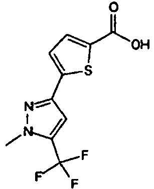 | 5-(1-Methyl-5-trifluormethyl-1H-pyrazol-3-yl)-thiophen-2-carbonsäure [4-(3-chlor-benzyl)-4-dimethylamino-cyclohexyl]-amid |
| **99** | 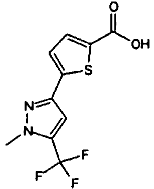 | 5-(1-Methyl-5-trifluormethyl-1H-pyrazol-3-yl)-thiophen-2-carbonsäure [4-dimethylamino-4-(2-fluor-benzyl)-cyclohexyl]-amid |
| **100** | 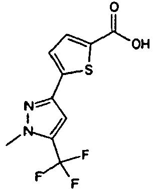 | 5-(1-Methyl-5-trifluormethyl-1H-pyrazol-3-yl)-thiophen-2-carbonsäure [4-(4-chlor-benzyl)-4-dimethylamino-cyclohexyl]-amid |
| **101** | 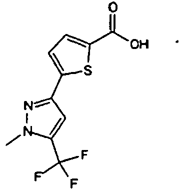 | 5-(1-Methyl-5-trifluormethyl-1H-pyrazol-3-yl)-thiophen-2-carbonsäure [4-dimethylamino-4-(4-methyl-benzyl)-cyclohexyl]-amid |
| **102** | 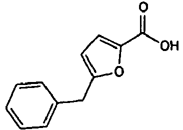 | 5-Benzyl-furan-2-carbonsäure [4-dimethylamino-4-(4-methyl-benzyl)-cyclohexyl]-amid |
| **103** | 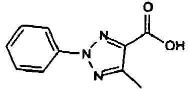 | 5-Methyl-2-phenyl-2H-[1,2,3]triazol-4-carbonsäure (4-azepan-1-yl-4-benzyl-cyclohexyl)-amid |
| **104** | 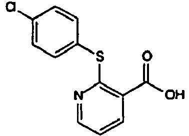 | 2-(4-Chlor-phenylsulfanyl)-N-[4-dimethylamino-4-(4-fluor-benzyl)-cyclohexyl]-nicotinamid |
| **105** | 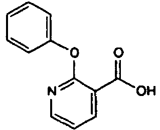 | N-[4-Dimethylamino-4-(4-fluor-benzyl)-cyclohexyl]-2-phenoxy-nicotinamid |
| **106** | 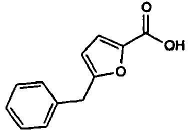 | 5-Benzyl-furan-2-carbonsäure (4-benzyl-4-piperidin-1-yl-cyclohexyl)-amid |
| **107** | 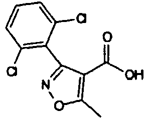 | 3-(2,6-Dichlor-phenyl)-5-methyl-isoxazol-4-carbonsäure [4-(4-chlor-benzyl)-4-dimethylamino-cyclohexyl]-amid |
| **108** | 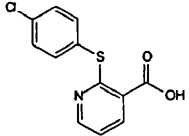 | N-(4-Benzyl-4-morpholin-4-yl-cyclohexyl)-2-(4-chlor-phenylsulfanyl)-nicotinamid |
| **109** | 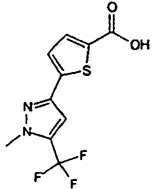 | 5-(1-Methyl-5-trifluormethyl-1H-pyrazol-3-yl)-thiophen-2-carbonsäure (4-benzyl-4-dimethylamino-cyclohexyl)-amid |
| **110** | 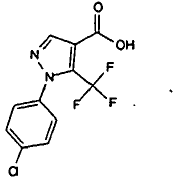 | 1-(4-Chlor-phenyl)-5-trifluormethyl-1H-pyrazol-4-carbonsäure [4-dimethylamino-4-(4-fluor-benzyl)-cyclohexyl]-amid |
| **111** | 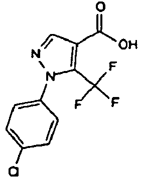 | 1-(4-Chlor-phenyl)-5-trifluormethyl-1H-pyrazol-4-carbonsäure [4-dimethylamino-4-(2-methyl-benzyl)-cyclohexyl]-amid |
| **112** | 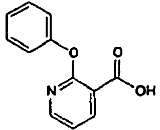 | N-(4-Azepan-1-yl-4-benzyl-cyclohexyl)-2-phenoxy-nicotinamid |
| **113** | 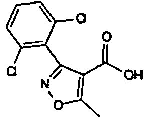 | 3-(2,6-Dichlor-phenyl)-5-methyl-isoxazol-4-carbonsäure [4-(2-chlor-benzyl)-4-dimethylamino-cyclohexyl]-amid |
| **114** | 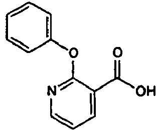 | N-[4-Dimethylamino-4-(2-methyl-benzyl)-cyclohexyl]-2-phenoxy-nicotinamid |
| **115** | 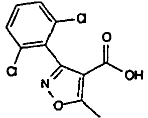 | 3-(2,6-Dichlor-phenyl)-5-methyl-isoxazol-4-carbonsäure [4-dimethylamino-4-(4-fluor-benzyl)-cyclohexyl]-amid |
| **116** | 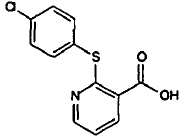 | 2-(4-Chlor-phenylsulfanyl)-N-[4-dimethylamino-4-(2-methyl-benzyl)-cyclohexyl]-nicotinamid |
| **117** | 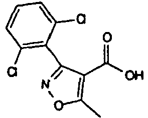 | 3-(2,6-Dichlor-phenyl)-5-methyl-isoxazol-4-carbonsäure [4-dimethylamino-4-(2-methyl-benzyl)-cyclohexyl]-amid |

Ausgewählte Beispiele wurden auch in größerem Maßstab synthetisiert.

### Beispiel 118: 3-(2-Chlorphenyl)-5-methylisoxazol-4-carbonsäure [4-dimethyl-amino-4-(3-fluorphenyl)cyclohexyl]amid Hydrochlorid, unpolareres Diastereoisomer

Das unpolarere Diastereoisomer von 1-(3-Fluorphenyl)-N,N-dimethylcyclohexan-1,4-diamin (350 mg) wurde mit 220 µl Triethylamin (1,05 Moläquivalente) und katalytischen Mengen DMAP (ca. 5 mg) in 3,5 ml Dichlormethan vorgelegt, bei -10 °C 400 mg 3-(2-Chlorphenyl)-5-methylisoxazol-4-carbonylchlorid (1,05 Moläquivalente), gelöst in 2 ml Dichlormethan, zugetropft und das Gemisch über Nacht unter Erwärmung auf Raumtemperatur gerührt. Zur Aufarbeitung wurde das Gemisch unter Eiskühlung mit zweimolarer Natronlauge alkalisch gestellt (pH > 10), die organische Phase abgetrennt, die wässrige Phase mit Dichlormethan (20 ml) extrahiert, der vereinigten organischen Phasen über Natriumsulfat getrocknet, filtriert und zur Trockne eingeengt. Das erhaltene Rohprodukt (690 mg) wurde an Kieselgel (4,0 x 15 cm) mit Methanol/Ethylacetat (V:V = 1:1) chromatographiert. Es wurden 530 mg des unpolareren Diastereoisomeren von 3-(2-Chlorphenyl)-5-methylisoxazol-4-carbonsäure [4-dimethylamino-4-(3-fluorphenyl)cyclohexyl]amid erhalten, die in 50 ml 2-Butanon gelöst durch Zugabe von 21 µl Wasser und 150 µl Chlortrimethylsilan, anschließendes Einengen zur Trockne und Rühren mit 10 ml Ethylacetat über Nacht in das korrespondierende Hydrochlorid überführt wurden (380 mg, Smp. 231 - 232 °C).

### Beispiel 119: 3-(2-Chlorphenyl)-5-methylisoxazol-4-carbonsäure [4-dimethyl-amino-4-(3-fluorphenyl)cyclohexyl]amid Hydrochlorid, polareres Diastereoisomer

Wie für Beispiel 118 beschrieben wurde das polarere Diastereoisomer von 1-(3-Fluorphenyl)-N,N-dimethylcyclohexan-1,4-diamin (350 mg) mit 400 mg 3-(2-Chlorphenyl)-5-methylisoxazol-4-carbonylchlorid umgesetzt und analog aufgearbeitet. Das erhaltene Rohprodukt (590 mg) wurde an Kieselgel (4,0 x 15 cm) mit Ethylacetat/n-Hexan/Methanol (V:V = 1:1:1) chromatographiert. Es wurden 440 mg des polareren Diastereoisomeren von 3-(2-Chlorphenyl)-5-methylisoxazol-4-carbonsäure [4-dimethylamino-4-(3-fluorphenyl)cyclohexyl]amid erhalten, die in 90 ml 2-Butanon gelöst durch Zugabe von 17 µl Wasser und 120 µl Chlortrimethylsilan in das korrespondierende Hydrochlorid überführt wurden (460 mg weißer Feststoff, Smp. 209 - 211 °C).

### Beispiel 120: 5-Methyl-2-phenyl-2H-[1,2,3]triazol-4-carbonsäure (4-dimethyl-amino-4-phenylcyclohexyl)amid Hydrochlorid, unpolareres Diastereoisomer

Zu 650 mg eines cis/trans-Gemisches von N,N-Dimethyl-1-phenylcyclohexan-1,4-diamin wurden unter Rühren bei 0 °C 600 mg 5-Methyl-2-phenyl-2H-[1,2,3]triazol-4-carbonsäure (1 Moläquivalent) gelöst in 5 ml DMF, 460 µl N,N-Diisopropylcarbodiimid (DIC, 1 Moläquivalent) und 400 µg 1-Hydroxybenzotriazol (HOBt, 1 Moläquivalent) gegeben. Nach drei Stunden bei dieser Temperatur wurde über Nacht unter Erwärmung auf Raumtemperatur nachgerührt. Zur Aufarbeitung wurde einmolare Natriumcarbonatlösung zugegeben (pH > 10) und das Rohprodukt durch Extraktion mit Ethylacetat/THF (V:V = 1:1), anschließende Trocknung über Natriumsulfat und Einengen isoliert. Die nach Säulenchromatographie an Kieselgel (3,0 x 18 cm) mit 100 ml Diethylether gefolgt von 350 ml Diethylether/Methanol (V:V = 2:1) erhaltene Hauptfraktion von 547 mg wurde in 20 ml 2-Butanon und 10 ml Ethylacetat gelöst und durch Zugabe von 24,4 µl Wasser, 172 µl Chlortrimethylsilan und 10 ml Diisopropylether in das korrespondierende Hydrochlorid des unpolareren Diastereoisomeren von 5-Methyl-2-phenyl-2H-[1,2,3]triazol-4-carbonsäure (4-dimethylamino-4-phenylcyclohexyl)amid überführt (210 mg weißer Feststoff, Smp. 245 - 247 °C).

### Beispiel 121: 5-Methyl-2-phenyl-2H-[1,2,3]triazol-4-carbonsäure (4-dimethyl-amino-4-phenylcyclohexyl)amid Hydrochlorid, polareres Diastereoisomer

Wie für Beispiel 120 beschrieben wurden auch 238 mg des polareren Diastereoisomeren von 5-Methyl-2-phenyl-2H-[1,2,3]triazol-4-carbonsäure (4-dimethylamino-4-phenylcyclohexyl)amid erhalten, die in 5 ml 2-Butanon und 5 ml Ethylacetat gelöst durch Zugabe von 10,6 µl Wasser, 75 µl Chlortrimethylsilan und 10 ml Diisopropylether in das korrespondierende Hydrochlorid überführt wurden (165 mg weißer Feststoff).

### Beispiel 122: 3-(2,6-Dichlorphenyl)-5-methylisoxazol-4-carbonsäure [4-dimethylamino-4-(3-fluorphenyl)cyclohexyl]amid Hydrochlorid, unpolareres Diastereoisomer

Wie für Beispiel 120 beschrieben wurden 700 mg eines cis/trans-Gemisches von 1-(3-Fluorphenyl)-N,N-dimethylcyclohexan-1,4-diamin mit 810 mg 3-(2,6-Dichlorphenyl)-5-methylisoxazol-4-carbonsäure (1 Moläquivalent) umgesetzt und das Rohprodukt (1,86 g) isoliert. Die nach Säulenchromatographie an Kieselgel (3,0 x 19 cm) mit 175 ml Diethylether gefolgt von 250 ml Diethylether/Methanol (V:V = 2:1), 250 ml Diethylether/Methanol (V:V = 1:1) und 100 ml Methanol erhaltene Hauptfraktion von 950 mg wurde in 25 ml 2-Butanon und 25 ml Ethylacetat gelöst und durch Zugabe von 35 µl Wasser, 246 µl Chlortrimethylsilan und 25 ml Diisopropylether in das korrespondierende Hydrochlorid des unpolareren Diastereoisomeren von 3-(2,6-Dichlorphenyl)-5-methylisoxazol-4-carbonsäure [4-dimethylamino-4-(3-fluorphenyl)cyclohexyl]amid überführt (263 mg weißer Feststoff, Smp. 248 - 250 °C).

### Beispiel 123: 3-(2,6-Dichlorphenyl)5-methylisoxazol-4-carbonsäure [4-dimethylamino-4-(3-fluorphenyl)cyclohexyl]amid Hydrochlorid, polareres Diastereoisomer

Wie für Beispiel 122 beschrieben wurden auch 448 mg des polareren Diastereoisomeren von 3-(2,6-Dichlorphenyl)-5-methylisoxazol-4-carbonsäure [4-dimethyl-amino-4-(3-fluorphenyl)cyclohexyl]amid erhalten, die in 15 ml 2-Butanon, 15 ml Ethylacetat und 2 ml Methanol gelöst durch Zugabe von 16,5 µl Wasser, 116 µl Chlortrimethylsilan und 25 ml Diisopropylether in das korrespondierende Hydrochlorid überführt wurden (364 mg weißer Feststoff, Smp. 246 - 248 °C).

### Beispiel 124: 3-(2,6-Dichlorphenyl)-5-methylisoxazol-4-carbonsäure (4-dimethylamino-4-phenylcyclohexyl)amid Hydrochlorid, unpolareres Diastereoisomer

Wie für Beispiel 120 beschrieben wurden 650 mg eines cis/trans-Gemisches von N,N-Dimethyl-1-phenylcyclohexan-1,4-diamin mit 810 mg 3-(2,6-Dichlorphenyl)-5-methylisoxazol-4-carbonsäure (1 Moläquivalent) umgesetzt und das Rohprodukt (1,92 g) isoliert. Die nach Säulenchromatographie an Kieselgel (3,0 x 19 cm) mit 175 ml Diethylether gefolgt von 250 ml Diethylether/Methanol (V:V = 2:1), 250 ml Diethylether/Methanol (V:V = 1:1) und 100 ml Methanol erhaltene Hauptfraktion von 1,10 g wurde in 15 ml 2-Butanon und 15 ml Ethylacetat gelöst und durch Zugabe von 42 µl Wasser, 296 µl Chlortrimethylsilan und 25 ml Diisopropylether in das korrespondierende Hydrochlorid des unpolareren Diastereoisomeren von 3-(2,6-Dichlorphenyl)-5-methylisoxazol-4-carbonsäure (4-dimethylamino-4-phenylcyclohexyl)amid überführt (311 mg weißer Feststoff).

### Beispiel 125: 3-(2,6-Dichlorphenyl)-5-methylisoxazol-4-carbonsäure (4-dimethylamino-4-phenylcyclohexyl)amid Hydrochlorid, polareres Diastereoisomer

Wie für Beispiel 124 beschrieben wurden auch 341 mg des polareren Diastereoisomeren von 3-(2,6-Dichlorphenyl)-5-methylisoxazol-4-carbonsäure (4-dimethyl-amino-4-phenylcyclohexyl)amid erhalten, die in 10 ml 2-Butanon, 10 ml Ethylacetat und 2 ml Methanol gelöst durch Zugabe von 13 µl Wasser, 92 µl Chlortrimethylsilan und 25 ml Diisopropylether bei 0 °C in das korrespondierende Hydrochlorid überführt wurden (292 mg weißer Feststoff).

### Beispiel 126: 5-Methyl-3-phenylisoxazol-4-carbonsäure [4-dimethylamino-4-(3-fluorphenyl)cyclohexyl]amid Hydrochlorid, unpolareres Diastereoisomer

Wie für Beispiel 120 beschrieben wurden 700 mg eines cis/trans-Gemisches von 1-(3-Fluorphenyl)-N,N-dimethylcyclohexan-1,4-diamin mit 600 mg 5-Methyl-3-phenyl-isoxazol-4-carbonsäure (1 Moläquivalent) umgesetzt und das Rohprodukt isoliert. Die nach Säulenchromatographie an Kieselgel (3,0 x 19 cm) mit 175 ml Diethylether gefolgt von 250 ml Diethylether/Methanol (V:V = 2:1), 250 ml Diethylether/Methanol (V:V = 1:1) und 200 ml Methanol erhaltene Hauptfraktion von 775 mg wurde in 10 ml 2-Butanon und 10 ml Ethylacetat gelöst und durch Zugabe von 33 µl Wasser und 233 µl Chlortrimethylsilan in das korrespondierende Hydrochlorid des unpolareren Diastereoisomeren von 5-Methyl-3-phenylisoxazol-4-carbonsäure [4-dimethylamino-4-(3-fluorphenyl)cyclohexyl]amid überführt (423 mg weißer Feststoff, Smp. 256 - 259 °C).

### Beispiel 127: 5-Methyl-3-phenylisoxazol-4-carbonsäure (4-dimethylamino-4-phenylcyclohexyl)amid Hydrochlorid, unpolareres Diastereoisomer

Wie für Beispiel 120 beschrieben wurden 650 mg eines cis/trans-Gemisches von N,N-Dimethyl-1-phenylcyclohexan-1,4-diamin mit 600 mg 5-Methyl-3-phenylisoxazol-4-carbonsäure (1 Moläquivalent) umgesetzt und das Rohprodukt isoliert. Die nach Säulenchromatographie an Kieselgel (3,0 x 18 cm) mit 100 ml Diethylether gefolgt von 350 ml Diethylether/Methanol (V:V = 2:1) erhaltene Hauptfraktion von 560 mg wurde in 20 ml 2-Butanon und 10 ml Ethylacetat gelöst und durch Zugabe von 25 µl Wasser und 176 µl Chlortrimethylsilan in das korrespondierende Hydrochlorid des unpolareren Diastereoisomeren von 5-Methyl-3-phenylisoxazol-4-carbonsäure (4-dimethylamino-4-phenylcyclohexyl)amid überführt (372 mg weißer Feststoff, Smp. 220 - 224 °C).

### Beispiel 128: 5-Methyl-3-phenylisoxazol-4-carbonsäure (4-dimethylamino-4-phenylcyclohexyl)amid Hydrochlorid, polareres Diastereoisomer

Wie für Beispiel 127 beschrieben wurden auch 190 mg des polareren Diastereoisomeren von 5-Methyl-3-phenylisoxazol-4-carbonsäure (4-dimethylamino-4-phenyl-cyclohexyl)amid erhalten, die in 10 ml 2-Butanon und 5 ml Ethylacetat gelöst durch Zugabe von 8,5 µl Wasser, 60 µl Chlortrimethylsilan und 10 ml Diisopropylether in das korrespondierende Hydrochlorid überführt wurden (135 mg weißer Feststoff, Smp. 243 - 245 °C).

### Untersuchungen zur Wirksamkeit der erfindungsgemäßen Verbindungen:

### Messung der ORL1-Bindung

Die Cyclohexan-Derivate der allgemeinen Formel I wurden in einem Rezeptorbindungsassay mit ³H-Nociceptin/Orphanin FQ mit Membranen von rekombinanten CHO-ORL1 Zellen untersucht. Dieses Testsystem wurde gemäß der von Ardati et al. (Mol. Pharmacol., 51, 1997, S. 816-824) vorgestellten Methode durchgeführt. Die Konzentration von ³H-Nociceptin/Orphanin FQ betrug bei diesen Versuchen 0.5 nM. Die Bindungsassays wurden mit je 20 µg Membranprotein je 200 µl Ansatz in 50 mM Hepes, pH 7,4, 10 mM MgCl₂ und 1 mM EDTA durchgeführt. Die Bindung an den ORL1-Rezeptor wurde unter Verwendung von je 1 mg WGA-SPA Beads (Amersham-Pharmacia, Freiburg), durch einstündige Inkubation des Ansatzes bei RT und anschliessende Messung im Szintillationscounter Trilux (Wallac, Finnland), bestimmt. Die Affinität wird in Tabelle 1 als nanomolarer Kᵢ-Wert in oder % Inhibition bei c=1 µM angegeben.

### Messung der µ-Bindung

Die Rezeptoraffinität zum humanen µ-Opiatrezeptor wurde in einem homogenen Ansatz in Mikrotiterplatten bestimmt. Hierzu wurden Verdünnungsreihen des jeweils zu prüfenden substituierten substituierten Cyclohexyl-1,4-diamin -Derivates mit einer Rezeptormembranpräparation (15-40 µg Protein pro 250 µl Inkubationsansatz) von CHO-K1-Zellen, welche den humanen µ-Opiatrezeptor exprimieren (RB-HOM-Rezeptormembran-Präparation der Firma NEN, Zaventem, Belgien) in Gegenwart von 1 nmol/l des radioaktiven Liganden [³H]-Naloxon (NET719, Firma NEN, Zaventem, Belgien) sowie von 1 mg WGA-SPA-Beads (Wheat germ agglutinin SPA Beads der Firma Amersham/Pharmacia, Freiburg, Deutschland) in einem Gesamtvolumen von 250 µl für 90 Minuten bei Raumtemperatur inkubiert. Als Inkubationspuffer wurde 50 mmol/l Tris-HCl supplementiert mit 0,05 Gew.-% Natriumazid und mit 0,06 Gew.-% bovinem Serumalbumin verwendet. Zur Bestimmung der unspezifischen Bindung wurde zusätzlich 25 µmol/l Naloxon zugegeben. Nach Beendigung der neunzigminütigen Inkubationszeit wurden die Mikrotiterplatten für 20 Minuten bei 1000 g abzentrifugiert und die Radioaktivität in einem ß-Counter (Microbeta-Trilux, Firma PerkinElmer Wallac, Freiburg, Deutschland) vermessen. Es wurde die prozentuale Verdrängung des radioaktiven Liganden aus seiner Bindung zum humanen µ-Opiatrezeptor bei einer Konzentration der Prüfsubstanzen von 1 µmol/l bestimmt und als prozentuale Hemmung (%Hemmung) der spezifischen Bindung angegeben. Teilweise wurden ausgehend von der prozentualen Verdrängung durch unterschiedliche Konzentrationen der zu prüfenden Verbindungen der allgemeinen Formel I IC₅₀ Hemmkonzentrationen berechnet, die eine 50-prozentige Verdrängung des radioaktiven Liganden bewirken. Durch Umrechnung mittels der Cheng-Prusoff-Beziehung wurden Ki-Werte für die Prüfsubstanzen erhalten.

### Messung der Serotonin-Wiederaufnahme

Um diese in vitro Studien durchführen zu können, werden Synaptosomen aus Rattenhirnarealen frisch isoliert. Es findet jeweils eine sogenannte "P₂"-Fraktion Verwendung, die nach der Vorschrift von Gray und Whittaker (E.G. Gray und V.P. Whittaker (1962) J. Anat. 76, 79-88) präpariert wird. Für den 5HT-Uptake werden diese vesikülären Partikel aus der Medulla + Pons-Region von männlichen Rattengehirnen isoliert.

Eine detaillierte Methodenbeschreibung kann der Literatur entnommen werden (M.Ch. Frink, H.-H. Hennies, W. Englberger, M. Haurand und B. Wilffert (1996) Arzneim.-Forsch./Drug Res. 46 (III), 11, 1029-1036).

### Messung der Noradrenalin-Wiederaufnahme

Um diese in vitro Studien durchführen zu können, werden Synaptosomen aus Rattenhirnarealen frisch isoliert. Es findet jeweils eine sogenannte "P₂"-Fraktion Verwendung, die nach der Vorschrift von Gray und Whittaker (E.G. Gray und V.P. Whittaker (1962) J. Anat. 76, 79-88) präpariert wird. Für den NA-Uptake werden diese vesikulären Partikel aus dem Hypothalamus männlicher Rattengehirne isoliert.

Eine detaillierte Methodenbeschreibung kann der Literatur entnommen werden (M.Ch. Frink, H.-H. Hennies, W. Englberger, M. Haurand und B. Wilffert (1996) Arzneim.-Forsch./Drug Res. 46 (III), 11, 1029-1036).

Beispielhaft wurden die folgenden Bindungsdaten bestimmt:

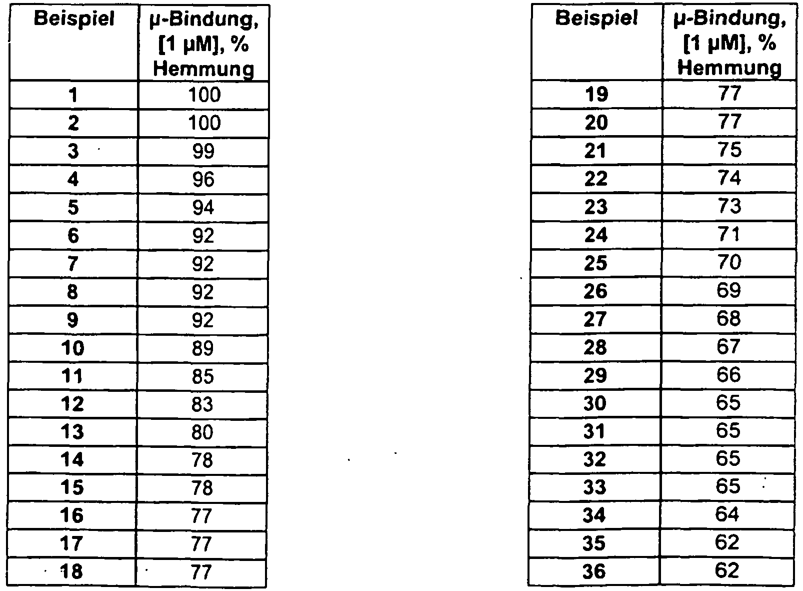

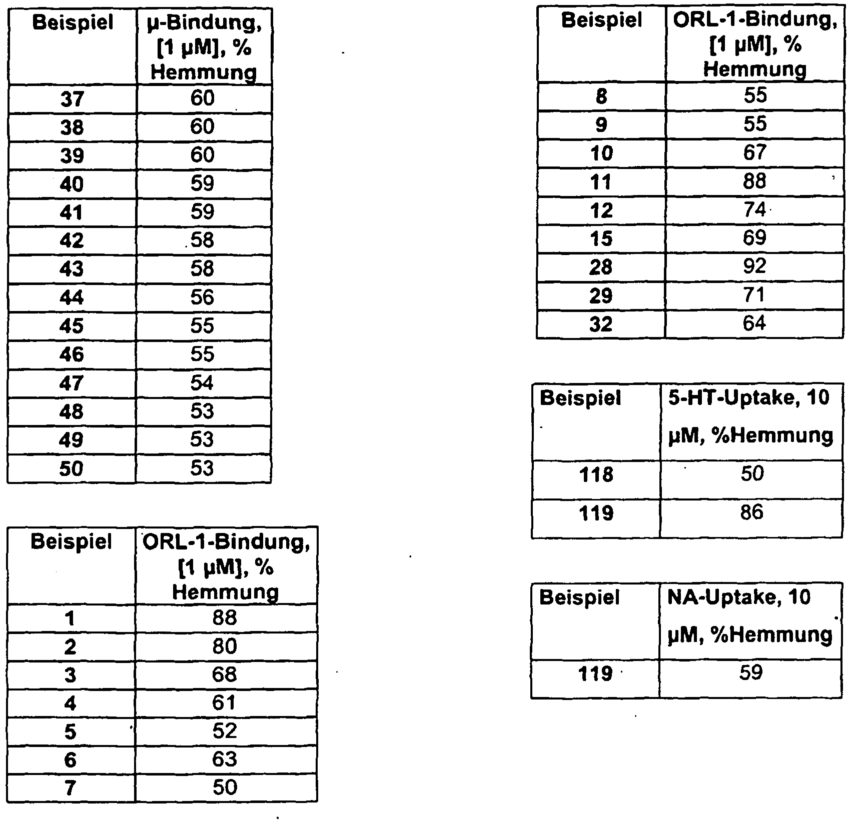

### Parenterale Lösung eines erfindungsgemäßen substituierten Cyclohexyl-1,4-diamin-Derivats

38 g eines der erfindungsgemäßen substituierten Cyclohexyl-1,4-diamin-Derivate, hier Beispiel 1, wird in 1 l Wasser für Injektionszwecke bei Raumtemperatur gelöst und anschließend durch Zugabe von wasserfreier Glukose für Injektionszwecke auf isotone Bedingungen eingestellt.

## Patentansprüche

1. Substituierte Cyclohexyl-1,4-diamin-Derivate-Derivate der allgemeinen Formel I, 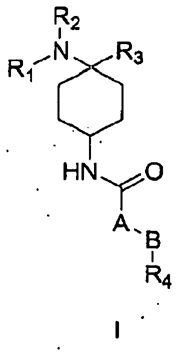 worin
R¹ und R², unabhängig voneinander für H; C₁₋₅-Alkyl jeweils gesättigt oder ungesättigt, verzweigt oder unverzweigt, einfach oder mehrfach substituiert oder unsubstituiert; C₃₋₈-Cycloalkyl, jeweils einfach oder mehrfach substituiert oder unsubstituiert; oder über C₁₋₃-Alkyl gebundenes Aryl, C₃₋₈-Cycloalkyl oder Heteroaryl, jeweils einfach oder mehrfach substituiert oder unsubstituiert, stehen;
oder die Reste R¹ und R² zusammen für CH₂CH₂OCH₂CH₂, CH₂CH₂NR¹⁰CH₂CH₂ oder (CH₂)₃₋₆ stehen,
wobei R¹⁰ H; C₁₋₅-Alkyl, jeweils gesättigt oder ungesättigt, verzweigt oder unverzweigt, einfach oder mehrfach substituiert oder unsubstituiert; C₃₋₈-Cycloalkyl, jeweils einfach oder mehrfach substituiert oder unsubstituiert; Aryl-, oder Heteroaryl, jeweils einfach oder mehrfach substituiert oder unsubstituiert; oder über C₁₋₃-Alkyl gebundenes Aryl, C₃₋₈-Cycloalkyl oder Heteroaryl, jeweils einfach oder mehrfach substituiert oder unsubstituiert; C(O)Phenyl, C(O)Heteroaryl, C(O)C₁₋₅-Alkyl, jeweils substituiert oder unsubstituiert, bedeutet;
R³ für C₁₋₅-Alkyl, jeweils gesättigt oder ungesättigt, verzweigt oder unverzweigt, einfach oder mehrfach substituiert oder unsubstituiert; C₃₋₈-Cycloalkyl, jeweils einfach oder mehrfach substituiert oder unsubstituiert; Aryl oder Heteroaryl, jeweils unsubstituiert oder einfach oder mehrfach substituiert; über C₁₋₃-Alkyl-Gruppe gebundenes Aryl, Heteroaryl oder C₃₋₈-Cycloalkyl, jeweils unsubstituiert oder einfach oder mehrfach substituiert, steht;
A für Heteroaryl, unsubstituiert oder einfach oder mehrfach substituiert, steht
B für (CH₂)ₘ mit in = 0, 1; SO₂, O, S, C(O), C(S) steht,
R⁴ für Heteroaryl oder Aryl, unsubstituiert oder einfach oder mehrfach substituiert, oder einen über eine C₁₋₃-Alkylkette verknüpften Heteroaryl- oder Aryl-Rest, unsubstituiert oder einfach oder mehrfach substituiert, steht;
in Form des Razemats; der Enantiomere, Diastereomere, Mischungen der Enantiomere oder Diastereomere oder eines einzelnen Enantiomers oder Diastereomers; der Basen und/oder Salze physiologisch verträglicher Säuren oder Kationen.

2. Substituierte Cyclohexyl-1,4-diamin-Derivate gemäß Anspruch 1, **dadurch gekennzeichnet, dass**
R¹ und R² unabhängig voneinander für H; C₁₋₅-Alkyl, gesättigt oder ungesättigt, verzweigt oder unverzweigt, einfach oder mehrfach substituiert oder unsubstituiert, stehen;
oder die Reste R¹ und R² zusammen einen Ring bilden und CH₂CH₂OCH₂CH₂, CH₂CH₂NR¹⁰CH₂CH₂ oder (CH₂)₃₋₆ bedeuten, wobei R¹⁰ H; C₁₋₅-Alkyl, gesättigt oder ungesättigt, verzweigt oder unverzweigt, einfach oder mehrfach substituiert oder unsubstituiert, bedeutet.

3. Substituierte Cyclohexyl-1,4-diamin-Derivate, gemäß einem der Ansprüche 1 oder 2, **dadurch gekennzeichnet, dass** R¹ und R² unabhängig voneinander für CH₃ oder H stehen, wobei R¹ und R² nicht gleichzeitig H bedeuten, oder R¹ und R² für CH₂CH₂OCH₂CH₂, (CH₂)₄, (CH₂)₅ oder (CH₂)₆ stehen.

4. Substituierte Cyclohexyl-1,4-diamin -Derivate gemäß einem der Ansprüche 1-3, **dadurch gekennzeichnet, dass**
R³ für Cyclopentyl, Cyclohexyl, Phenyl, Benzyl, Naphthyl, Anthracenyl, Thiophenyl, Benzothiophenyl, Furyl, Benzofuranyl, Benzodioxolanyl, Indolyl, Indanyl, Benzodioxanyl, Pyrrolyl, Pyridyl, Pyrimidyl oder Pyrazinyl, jeweils unsubstituiert oder einfach oder mehrfach substituiert; über eine gesättigte, unverzweigte C₁₋₂-Alkyl-Gruppe gebundenen C₅₋₆-Cycloalkyl, Phenyl, Naphthyl, Anthracenyl, Thiophenyl, Benzothiophenyl, Pyridyl, Furyl, Benzofuranyl, Benzodioxolanyl, Indolyl, Indanyl, Benzodioxanyl, Pyrrolyl, Pyrimidyl oder Pyrazinyl, jeweils unsubstituiert oder einfach oder mehrfach substituiert, steht;
insbesondere
R³ Phenyl, Furyl, Thiophenyl, Naphthyl, Benzyl, Benzofuranyl, Indolyl, Indanyl, Benzodioxanyl, Benzodioxolanyl, Pyridyl, Pyrimidyl, Pyrazinyl oder Benzothiophenyl, jeweils unsubstituiert oder einfach oder mehrfach substituiert; über eine gesättigte, unverzweigte C₁₋₂-Alkyl-Gruppe gebundenen Phenyl, Furyl oder Thiophenyl, jeweils unsubstituiert oder einfach oder mehrfach substituiert, bedeutet.

5. Substituierte Cyclohexyl-1,4-diamin -Derivate gemäß einem der Ansprüche 1-4, **dadurch gekennzeichnet, dass** R³ für Phenyl, Phenethyl, Thiophenyl, Pyridyl oder Benzyl, jeweils substituiert oder unsubstituiert, steht.

6. Substituierte Cyclohexyl-1,4-diamin -Derivate gemäß einem der Ansprüche 1-4, **dadurch gekennzeichnet, dass** R³ für 4-Methylbenzyl, 2-Methylbenzyl, 3-Chlorbenzyl, 4-Chlorbenzyl, 2-Chlorbenzyl, 4-Fluorbenzyl, 3-Fluorbenzyl, 3-Methylbenzyl, 2-Fluorbenzyl, Benzyl, Phenethyl, Phenyl, Pyridyl, Thiophenyl und 3-fluorophenyl, steht.

7. Substituierte Cyclohexyl-1,4-diamin -Derivate gemäß einem der Ansprüche 1-6, **dadurch gekennzeichnet, dass** A für Pyrrolyl, Thiophenyl, Furanyl, Pyrazolyl, Isoxazolyl, Oxazolyl, Isothiazolyl, Imidazolyl, Triazolyl, Tetrahydroisoxazolyl, Pyridinyl, Pyrimidinyl, Pyrazinyl, Triazinyl, Indolyl, Benzofuranyl, Benzothiophenyl, Indazolyl, Benzimidazolyl, Purinyl, Chinolinyl, Isochinolinyl, Phthalazin, Benzothiadiazolyl, Benzothiazolyl, Benzotriazolyl, Benzodioxolanyl, Benzodioxanyl und Carbazolyl, unsubstituiert oder einfach oder mehrfach substituiert, steht.

8. Substituierte Cyclohexyl-1,4-diamin-Derivate gemäß einem der Ansprüche 1-6, **dadurch gekennzeichnet, dass** A für Pyridyl, Thiophenyl, Pyrazolyl, Triazolyl, Tetrahydroisoxazolyl, Isoxazolyl, Thiazolyl und Furanyl steht.

9. Substituierte Cyclohexyl-1,4-diamin -Derivate gemäß einem der Ansprüche 1-8, **dadurch gekennzeichnet, dass** B für (CH₂)ₘ mit m = 0, 1; SO₂, O, S steht.

10. Substituierte Cyclohexyl-1,4-diamin -Derivate gemäß einem der Ansprüche 1-9, **dadurch gekennzeichnet, dass** R⁴ für Phenyl, Benzyl, Naphthyl, Anthracenyl, Thiophenyl, Benzothiophenyl, Furanyl, Isothiazolyl, Imidazolyl, Triazolyl, Triazinyl, Pyrazolyl, Benzofuranyl, Benzodioxolanyl, Isochinolinyl, Phthalazin, Benzo[1,2,5]thiadiazol, Benzothiazol, Benzotriazol, Chinolinyl, Carbazol, Isoxazolyl, Oxazolyl, Indolyl, Indanyl, Benzodioxanyl, Indazolyl, Benzimidazolyl, Pyrrolyl, Pyridyl, Pyrimidyl oder Pyrazinyl, jeweils unsubstituiert oder einfach oder mehrfach substituiert; über eine gesättigte, unverzweigte C₁₋₂-Alkyl-Gruppe gebundenen Phenyl, Naphthyl, Anthracenyl, Thiophenyl, Benzothiophenyl, Pyridyl, Furyl, Benzofuranyl, Indolyl, Indanyl, Benzodioxanyl, Pyrrolyl, Pyrimidyl oder Pyrazinyl, jeweils unsubstituiert oder einfach oder mehrfach substituiert, steht,
insbesondere
R⁴ für Phenyl, Benzyl, Naphthyl, Thiophenyl, Benzothiophenyl, Furanyl, Isothiazolyl, Imidazolyl, Triazolyl, Pyrazolyl, Benzofuranyl, Isochinolinyl, Benzothiazol, Benzotriazol, Chinolinyl, Isoxazolyl, Oxazolyl, Indolyl, Pyrrolyl, Pyridyl, Pyrimidyl oder Pyrazinyl, jeweils unsubstituiert oder einfach oder mehrfach substituiert; Benzyl oder Phenethyl, jeweils unsubstituiert oder einfach oder mehrfach substituiert, steht.

11. Substituierte Cyclohexyl-1,4-diamin -Derivate gemäß einem der Ansprüche 1-9, **dadurch gekennzeichnet, dass** R⁴ für Phenyl, Pyrazolyl oder Thiophenyl, unsubstituiert oder einfach oder mehrfach substituiert, steht.

12. Substituierte Cyclohexyl-1,4-diamin-Derivate gemäß einem der Ansprüche 1-11 aus der Gruppe
5-Methyl-2-phenyl-2H-[1 ,2,3]triazol-4-carbonsäure (4-dimethylamino-4-phenyl-cyclohexyl)-amid
1-Phenyl-5-propyl-1H-pyrazol-4-carbonsäure [4-dimethylamino-4-(3-fluor-phenyl)-cyclohexyl]-amid
3-(2,6-Dichlor-phenyl)-5-methyl-isoxazol-4-carbonsäure (4-benzyl-4-pyrrolidin-1-yl-cyclohexyl)-amid
5-Methyl-2-phenyl-2H-[1,2,3]triazol-4-carbonsäure (4-benzyl-4-piperidin-1-yl-cyclohexyl)-amid
5-Methyl-2-phenyl-2H-[1,2,3]triazol-4-carbonsäure (4-phenyl-4-piperidin-1-yl-cyclohexyl)-amid
N-(4-Dimethylamino-4-phenyl-cyclohexyl)-2-phenoxy-nicotinamid
2-(4-Chlor-phenylsulfanyl)-N-(4-dimethylamino-4-pyridin-2-yl-cyclohexyl)-nicotinamid
N-(4-Dimethylamino-4-thiophen-2-yl-cyclohexyl)-2-(5-methyl-2-phenyl-thiazol-4-yl)-acetamid
2-(4-Chlor-phenylsulfanyl)-N-[4-dimethylamino-4-(3-fluor-phenyl)-cyclohexyl]-nicotinamid
N-(4-Dimethylamino-4-thiophen-2-yl-cyclohexyl)-2-phenoxy-nicotinamid
3-(2-Chlor-phenyl)-5-methyl-isoxazol-4-carbonsäure (4-dimethylamino-4-phenyl-cyclohexyl)-amid
5-(4-Chlor-phenyl)-2-methyl-furan-3-carbonsäure [4-dimethylamino-4-(3-fluor-phenyl)-cyclohexyl]-amid
3-(2-Chlor-6-fluor-phenyl)-5-methyl-isoxazol-4-carbonsäure (4-dimethylamino-4-phenethyl-cyclohexyl)-amid
N-(4-Benzyl-4-pyrrolidin-1-yl-cyclohexyl)-2-(4-chlor-phenylsulfanyl)-nicotinamid
3-(2-Chlor-6-fluor-phenyl)-5-methyl-isoxazol-4-carbonsäure (4-dimethylamino-4-phenyl-cyclohexyl)-amid
3-(2,6-Dichlor-phenyl)-5-methyl-isoxazol-4-carbonsäure (4-benzyl-4-piperidin-1-yl-cyclohexyl)-amid
N-(4-Dimethylamino-4-phenethyl-cyclohexyl)-2-(5-methyl-2-phenyl-thiazol-4-yl)-acetamid
3-(2-Chlor-phenyl)-5-methyl-isoxazol-4-carbonsäure (4-dimethylamino-4-phenethyl-cyclohexyl)-amid
5-Benzyl-furan-2-carbonsäure (4-benzyl-4-dimethylamino-cyclohexyl)-amid
1-(4-Chlor-phenyl)-5-trifluormethyl-1H-pyrazol-4-carbonsäure [4-dimethylamino-4-(3-fluor-phenyl)-cyclohexyl]-amid
N-(4-Dimethylamino-4-pyridin-2-yl-cyclohexyl)-2-phenoxy-nicotinamid
5-Benzyl-furan-2-carbonsäure (4-phenyl-4-piperidin-1-yl-cyclohexyl)-amid
5-Methyl-2-phenyl-2H-[1,2,3]triazol-4-carbonsäure [4-dimethylamino-4-(2-methylbenzyl)-cyclohexyl]-amid
2-(4-Chlor-phenylsulfanyl)-N-[4-dimethylamino-4-(2-fluor-benzyl)-cyclohexyl]-nicotinamid
3-(2,6-Dichlor-phenyl)-5-methyl-isoxazol-4-carbonsäure [4-dimethylamino-4-(2-fluorbenzyl)-cyclohexyl]-amid
5-Methyl-2-phenyl-2H-[1,2,3]triazol-4-carbonsäure [4-dimethylamino-4-(4-fluor-benzyl)-cyclohexyl]-amid
5-Benzyl-furan-2-carbonsäure [4-dimethylamino-4-(2-fluor-benzyl)-cyclohexyl]-amid
3-(2-Chlor-phenyl)-5-methyl-isoxazol-4-carbonsäure [4-dimethylamino-4-(3-fluorphenyl)-cyclohexyl]-amid
3-(2-Chlor-6-fluor-phenyl)-5-methyl-isoxazol-4-carbonsäure [4-dimethylamino-4-(3-fluor-phenyl)-cyclohexyl]-amid
N-(4-Dimethylamino-4-phenethyl-cyclohexyl)-2-phenoxy-nicotinamid
3-(2-Chlor-phenyl)-5-methyl-isoxazol-4-carbonsäure [4-(3-chlor-benzyl)-4-dimethylamino-cyclohexyl]-amid
3-(2,6-Dichlor-phenyl)-5-methyl-isoxazol-4-carbonsäure [4-dimethylamino-4-(3-fluorphenyl)-cyclohexyl]-amid
5-Benzyl-furan-2-carbonsäure (4-dimethylamino-4-phenethyl-cyclohexyl)-amid
5-Methyl-2-phenyl-2H-[1,2,3]triazol-4-carbonsäure [4-dimethylamino-4-(4-methylbenzyl)-cyclohexyl]-amid
5-Benzyl-furan-2-carbonsäure [4-dimethylamino-4-(2-methyl-benzyl)-cyclohexyl]-amid
3-(2,6-Dichlor-phenyl)-5-methyl-Isoxazol-4-carbonsäure (4-dimethylamino-4-phenethyl-cyclohexyl)-amid
N-(4-Benzyl-4-pyrrolidin-1-yl-cyclohexyl)-2-phenoxy-nicotinamid
5-Benzyl-furan-2-carbonsäure [4-dimethylamino-4-(3-methyl-benzyl)-cyclohexyl]-amid
5-Benzyl-furan-2-carbonsäure [4-dimethylamino-4-(3-fluor-benzyl)-cyclohexyl]-amid
N-[4-(3-Chlor-benyl)-4-dimethylamino-cyclohexyl]-2-(4-chlor-phenylsulfanyl)-nicotinamid
2-(5-Methyl-2-phenyl-thiazol-4-yl)-N-(4-phenyl-4-piperidin-1-yl-cyclohexyl)-acetamid
5-Methyl-2-phenyl-2H-[1,2,3]triazol-4-carbonsäure [4-dimethylamino-4-(3-methylbenzyl)-cyclohexyl]-amid
N-[4-Dimethylamino-4-(2-methyl-benzyl)-cyclohexyl]-2-(5-methyl-2-phenyl-thiazol-4-yl)-acetamid
1-(4-Chlor-phenyl)-5-trifluormethyl-1H-pyrazol-4-carbonsäure [4-(3-chlor-benzyl)-4-dimethylamino-cyclohexyl]-amid
5-Methyl-2-phenyl-2H-[1,2,3]triazol-4-carbonsäure [4-(3-chlor-benzyl)-4-dimethylamino-cyclohexyl]-amid
5-Methyl-2-phenyl-2H-[1,2,3]triazol-4-carbonsäure (4-dimethylamino-4-phenethyl-cyclohexyl)-amid
3-(2-Chlor-6-fluor-phenyl)-5-methyl-isoxazol-4-carbonsäure (4-benzyl-4-pyrrolidin-1-yl-cyclohexyl)-amid
N-[4-Dimethylamino-4-(2-fluor-benzyl)-cyclohexyl]-2-phenoxy-nicotinamid .
N-[4-Dimethylamino-4-(3-fluor-benzyl)-cyclohexyl]-2-(5-methyl-2-phenyl-thiazol-4-yl)-acetamid
N-(4-Benzyl-4-dimethylamino-cyclohexyl)-2-phenoxy-nicotinamid
5-(Thiophen-2-sulfonyl)-4,5,6,7-tetrahydro-isoxazolo[4,5-c]pyridine-3-carbonsäure (4-benzyl-4-dimethylamino-cyclohexyl)-amid
1-(4-Chlor-phenyl)-5-trifluormethyl-1H-pyrazol-4-carbonsäure [4-dimethylamino-4-(2-fluor-benzyl)-cyclohexyl]-amid
5-Benzyl-furan-2-carbonsäure [4-(4-chlor-benzyl)-4-dimethylamino-cyclohexyl]-amid
5-Methyl-2-phenyl-2H-[1,2,3]triazol-4-carbonsäure (4-benzyl-4-dimethylamino-cyclohexyl)-amid
N-(4-Benzyl-4-dimethylamino-cyclohexyl)-2-(5-methyl-2-phenyl-thiazol-4-yl)-acetamid
N-[4-Dimethylamino-4-(2-fluor-benzyl)-cyclohexyl]-2-(5-methyl-2-phenyl-thiazol-4-yl)-acetamid
3-(2-Chlor-phenyl)-5-methyl-isoxazol-4-carbonsäure [4-dimethylamino-4-(2-fluorbenzyl)-cyclohexyl]-amid
5-Methyl-2-phenyl-2H-[1,2,3]triazol-4-carbonsäure [4-(2-chlor-benzyl)-4-dimethylamino-cyclohexyl]-amid
2-(4-Chlor-phenylsulfanyl)-N-[4-dimethylamino-4-(3-fluor-benzyl)-cyclohexyl]-nicotinamid
3-(2,6-Dichlor-phenyl)-5-methyl-isoxazol-4-carbonsäure (4-benzyl-4-dimethylamino-cyclohexyl)-amid
N-[4-(3-Chlor-benzyl)-4-dimethylamino-cyclohexyl]-2-phenoxy-nicotinamid
2-Phenoxy-N-(4-phenyl-4-piperidin-1-yl-cyclohexyl)-nicotinamid
5-(4-Chlor-phenyl)-2-methyl-furan-3-carbonsäure (4-benzyl-4-pyrrolidin-1-yl-cyclohexyl)-amid
5-(3-Trifluormethyl-benzylsulfonyl)-4,5,6,7-tetrahydro-isoxazolo[4,5-c]pyridine-3-carbonsäure (4-benzyl-4-dimethylamino-cyclohexyl)-amid
N-[4-Dimethylamino-4-(4-methyl-benzyl)-cyclohexyl]-2-phenoxy-nicotinamid
N-[4-Dimethylamino-4-(3-fluor-benzyl)-cyclohexyl]-2-phenoxy-nicotinamid
3-(2-Chlor-6-fluor-phenyl)-5-methyl-isoxazol-4-carbonsäure (4-phenyl-4-piperidin-1-yl-cyclohexyl)-amid
5-(4-Chlor-phenyl)-2-methyl-furan-3-carbonsäure [4-dimethylamino-4-(4-fluor-benzyl)-cyclohexyl]-amid
1-(4-Chlor-phenyl)-5-trifluormethyl-1H-pyrazol-4-carbonsäure [4-dimethylamino-4-(4-methyl-benzyl)-cyclohexyl]-amid
5-Methyl-2-phenyl-2H-[1,2,3]triazol-4-carbonsäure [4-(4-chlor-benzyl)-4-dimethylamino-cyclohexyl]-amid
5-Methyl-2-phenyl-2H-[1,2,3]triazol-4-carbonsäure [4-dimethylamino-4-(3-fluor-benzyl)-cyclohexyl]-amid
3-(2,6-Dichlor-phenyl)-5-methyl-isoxazol-4-carbonsäure (4-phenyl-4-piperidin-1-yl-cyclohexyl)-amid
1-(4-Chlor-phenyl)-5-trifluormethyl-1H-pyrazol-4-carbonsäure (4-morpholin-4-yl-4-phenyl-cyclohexyl)-amid
N-[4-(4-Chlor-benzyl)-4-dimethylamino-cyclohexyl]-2-phenoxy-nicotinamid
N-[4-(4-Chlor-benzyl)-4-dimethylamino-cyclohexyl]-2-(5-methyl-2-phenyl-thiazol-4-yl)-acetamid
3-(2-Chlor-phenyl)-5-methyl-isoxazol-4-carbonsäure (4-azepan-1-yl-4-benzyl-cyclohexyl)-amid
3-(2-Chlor-6-fluor-phenyl)-5-methyl-isoxazol-4-carbonsäure [4-dimethylamino-4-(4-fluor-benzyl)-cyclohexyl]-amid
3-(2-Chlor-6-fluor-phenyl)-5-methyl-isoxazol-4-carbonsäure [4-(3-chlor-benzyl)-4-dimethylamino-cyclohexyl]-amid
3-(2-Chlor-phenyl)-5-methyl-isoxazol-4-carbonsäure [4-dimethylamino-4-(2-methylbenzyl)-cyclohexyl]-amid
3-(2-Chlor-6-fluor-phenyl)-5-methyl-isoxazol-carbonsäure [4-dimethylamino-4-(2-methyl-benzyl)-cyclohexyl]-amid
5-(1-Methyl-5-trifluormethyl-1H-pyrazol-3-yl)-thiophen-2-carbonsäure (4-benzyl-4-piperidin-1-yl-cyclohexyl)-amid
3-(2-Chlor-phenyl)-5-methyl-isoxazol-4-carbonsäure [4-dimethylamino-4-(4-fluorbenzyl)-cyclohexyl]-amid
3-(2,6-Dichlor-phenyl)-5-methyl-isoxazol-4-carbonsäure [4-dimethylamino-4-(4-methylbenzyl)-cyclohexyl]-amid
3-(2-Chlor-phenyl)-5-methyl-isoxazol-4-carbonsäure (4-benzyl-4-pyrrolidin-1-yl-cyclohexyl)-amid
3-(2-chlor-6-fluor-phenyl)-5-methyl-isoxazol-4-carbonsäure [4-dimethylamino-4-(3-fluor-benzyl)-cyclohexyl]-amid
3-(2-Chlor-6-fluor-phenyl)-5-methyl-isoxazol-4-carbonsäure [4-dimethylamino-4-(4-methyl-benzyl)-cyclohexyl]-amid
1-(4-Chlor-phenyl)-5-trifluormethyl-1H-pyrazol-4-carbonsäure [4-(4-chlor-benzyl)-4-dimethylamino-cyclohexyl]-amid
1-Phenyl-5-propyl-1H-pyrazol-4-carbonsäure (4-azepan-1-yl-4-benzyl-cyclohexyl)-amid
3-(2-Chlor-phenyl)-5-methyl-isoxazol-4-carbonsäure [4-dimethylamino-4-(3-fluorbenzyl)-cyclohexyl]-amid
1-Phenyl-5-propyl-1H-pyrazol-4-carbonsäure [4-(2-chlor-benzyl)-4-dimethylamino-cyclohexyl]-amid
3-(2-Chlor-6-fluor-phenyl)-5-methyl-isoxazol-4-carbonsäure (4-benzyl-4-dimethylamino-cyclohexyl)-amid
3-(2,6-Dichlor-phenyl)-5-methyl-isoxazol-4-carbonsäure [4-dimethylamino-4-(3-fluorbenzyl)-cyclohexyl]-amid
1-Phenyl-5-propyl-1H-pyrazol-4-carbonsäure [4-dimethylamino-4-(4-fluor-benzyl)-cyclohexyl]-amid
1-(4-Chlor-phenyl)-5-trifluormethyl-1H-pyrazol-4-carbonsäure (4-benzyl-4-dimethylamino-cyclohexyl)-amid
5-(1-Methyl-5-trifluormethyl-1H-pyrazol-3-yl)-thiophen-2-carbonsäure [4-dimethylamino-4-(3-fluor-benzyl)-cyclohexyl]-amid
3-(2-Chlor-6-fluor-phenyl)-5-methyl-isoxazol-4-carbonsäure [4-dimethylamino-4-(2-fluor-benzyl)-cyclohexyl]-amid
1-(4-Chlor-phenyl)-5-trifluormethyl-1H-pyrazol-4-carbonsäure (4-dimethylamino-4-phenethyl-cyclohexyl)-amid
5-(1-Methyl-5-trifluormethyl-1H-pyrazol-3-yl)-thiophen-2-carbonsäure [4-(3-chlorbenzyl)-4-dimethylamino-cyclohexyl]-amid
5-(1-Methyl-5-trifluormethyl-1H-pyrazol-3-yl)-thiophen-2-carbonsäure [4-dimethylamino-4-(2-fluor-benzyl)-cyclohexyl]-amid
5-(1-Methyl-5-trifluormethyl-1H-pyrazol-3-yl)-thiophen-2-carbonsäure [4-(4-chlorbenzyl)-4-dimethylamino-cyclohexyl]-amid
5-(1-Methyl-5-trifluormethyl-1H-pyrazol-3-yl)-thiophen-2-carbonsäure [4-dlmethylamino-4-(4-methyl-benzyl)-cyclohexyl]-amid
5-Benzyl-furan-2-carbonsäure [4-dimethylamino-4-(4-methyl-benzyl)-cyclohexyl]-amid
5-Methyl-2-phenyl-2H-[1,2,3]triazol-4-carbonsäure (4-azepan-1-yl-4-benzyl-cyclohexyl)-amid
2-(4-Chlor-phenylsulfanyl)-N-[4-dimethylamino-4-(4-fluor-benzyl)-cyclohexyl]-nicotinamid
N-[4-Dimethylamino-4-(4-fluor-benzyl)-cyclohexyl]-2-phenoxy-nicotinamid
5-Benzyl-furan-2-carbonsäure (4-benzyl-4-piperidin-1-yl-cyclohexyl)-amid
3-(2,6-Dichlor-phenyl)-5-methyl-isoxazol-4-carbonsäure [4-(4-chlor-benoyl)-4-dimethylamino-cyclohexyl]-amid
N-(4-Benzyl-4-morpholin-4-yl-cyclohexyl)-2-(4-chlor-phenylsulfanyl)-nicotinamid
5-(1-Methyl-5-trifluomethyl-1H-pyrazol-3-yl)-thiophen-2-carbonsäure (4-benzyl-4-dimethylamino-cyclohexyl)-amid
1-(4-Chlor-phenyl)-5-trifluormethyl-1H-pyrazol-4-carbonsäure [4-dimethylamino-4-(4-fluor-benzyl)-cyclohexyl]-amid
1-(4-Chlor-phenyl)-5-trifluormethyl-1H-pyrazol-4-carbonsäure [4-dimethylamino-4-(2-methyl-benzyl)-cyclohexyl]-amid
N-(4-Azepan-1-yl-4-benzyl-cyclohexyl)-2-phenoxy-nicotinamid
3-(2,6-Dichlor-phenyl)-5-methyl-isoxazol-4-carbonsäure [4-(2-chlor-benzyl)-4-dimethylamino-cyclohexyl]-amid
N-[4-Dimethylamino-4-(2-methyl-benzyl)-cyclohexyl]-2-phenoxy-nicotinamid
3-(2,6-Dichlor-phenyl)-5-methyl-isoxazol-4-carbonsäure [4-dimethylamino-4-(4-fluor-benzyl)-cyclohexyl]-amid
2-(4-Chlor-phenylsulfanyl)-N-[4-dimethylamino-4-(2-methyl-benzyl)-cyclohexyl]-nicotinamid
3-(2,6-Dichlor-phenyl)-5-methyl-isoxazol-4-carbonsäure [4-dimethylamino-4-(2-methylbenzyl)-cyclohexyl]-amid
3-(2-Chlorphenyl)-5-methylisoxazol-4-carbonsäure [4-dimethylamino-4-(3-fluorphenyl)-cyclohexyl]amid Hydrochlorid, unpolareres Diastereoisomer
3-(2-Chlorphenyl)-5-methylisoxazol-4-carbonsäure [4-dimethylamino-4-(3-fluorphenyl)-cyclohexyl]amid Hydrochlorid, polareres Diastereoisomer
5-Methyl-2-phenyl-2H-[1,2,3]triazol-4-carbonsäure (4-dimethylamino-4-phenylcyclohexyl)amid Hydrochlorid, unpolareres Diastereoisomer
5-Methyl-2-phenyl-2H-[1,2,3]triazol-4-carbonsäure (4-dimethylamino-4-phenylcyclohexyl)amid Hydrochlorid, polareres Diastereoisomer
3-(2,6-Dichlorphenyl)-5-methylisoxazol-4-carbonsäure [4-dimethylamino-4-(3-fluorphenyl)cyclohexyl]amid Hydrochlorid, unpolareres Diastereoisomer
3-(2,6-Dichlorphenyl)-5-methylisoxazol-4-carbonsäure [4-dimethylamino-4-(3-fluorphenyl)cyclohexyl]amid Hydrochlorid, polareres Diastereoisomer
3-(2,6-Dichlorphenyl)-5-methylisoxazol-4-carbonsäure (4-dimethylamino-4-phenylcyclohexyl)amid Hydrochlorid, unpolareres Diastereoisomer
3-(2,6-Dichlorphenyl)-5-methylisoxazol-4-carbonsäure (4-dimethylamino-4-phenylcyclohexyl)amid Hydrochlorid, polareres Diastereoisomer
5-Methyl-3-phenylisoxazol-4-carbonsäure [4-dimethylamino-4-(3-fluorphenyl)cyclohexyl]amid Hydrochlorid, unpolareres Diastereoisomer
5-Methyl-3-phenylisoxazol-4-carbonsäure (4-dimethylamino-4-phenylcyclohexyl)amid Hydrochlorid, unpolareres Diastereoisomer
5-Methyl-3-phenylisoxazol-4-carbonsäure (4-dimethylamino-4-phenylcyclohexyl)amid Hydrochlorid, polareres Diastereoisomer
in Form des Razemats; der Enantiomere, Diastereomere, Mischungen der Enantiomere oder Diastereomere oder eines einzelnen Enantiomers oder Diastereomers; der Basen und/oder Salze physiologisch verträglicher Säuren oder Kationen.

13. Verfahren zur Herstellung von substituierten Cyclohexyl-1,4-diamin-Derivaten gemäß einem der Ansprüche 1-12, **dadurch gekennzeichnet, dass** substituierte Cyclohexan-1,4-diamine mit Carbonsäuren der allgemeinen Formel II 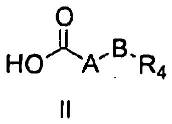 unter Zugabe von Kupplungsreagenzien oder durch Aktivierung der Carbonsäurekomponente, insbesondere durch Herstellung des Säurechlorids, verknüpft werden.

14. Arzneimittel enthaltend wenigstens ein substituiertes Cyclohexyl-1,4-diamin-Derivat gemäß einem der Ansprüche 1 bis 12, gegebenenfalls in Form seines Razemats, der reinen Stereoisomeren, insbesondere Enantiomeren und Diastereomeren, in einem beliebigen Mischungsverhältnis; in Form seiner Säuren oder seiner Basen oder in Form seiner Salze, insbesondere der physiologisch verträglichen Salze oder Salze physiologisch verträglicher Säuren oder Kationen; oder in Form seiner Solvate, insbesondere der Hydrate, sowie gegebenenfalls enthaltend geeignete Zusatz- und/oder Hilfsstoffe und/oder gegebenenfalls weiterer Wirkstoffe.

15. Substituierte Cyclohexyl-1,4-diamin-Derivate gemäß einem der Ansprüche 1 bis 12, gegebenenfalls in Form seines Razemats, der reinen Stereoisomeren, insbesondere Enantiomeren und Diastereomeren, in einem beliebigen Mischungsverhältnis; in Form seiner Säuren oder seiner Basen oder in Form seiner Salze, insbesondere der physiologisch verträglichen Salze oder Salze physiologisch verträglicher Säuren oder Kationen; oder in Form seiner Solvate, insbesondere der Hydrate; zur Anwendung bei der Behandlung von Schmerz, insbesondere von akutem, neuropathischem oder chronischem Schmerz,
zur Anwendung bei der Behandlung von Angstzuständen, von Stress und mit Stress verbundenen Syndromen, Depressionen, Epilepsie, Alzheimer Erkrankung, seniler Demenz, Katalepsie, allgemeinen kognitiven Dysfunktionen, Lern- und Gedächtnis-Störungen (als Nootropikum), Entzugserscheinungen, Alkohol- und/oder Drogen- und/oder Medikamentenmißbrauch und/oder -abhängigkeit, sexuellen Dysfunktionen, cardiovaskulären Erkrankungen, Hypotension, Hypertension, Tinitus, Pruritus, Migräne, Schwerhörigkeit, mangelnder Darmmotilität, gestörter Nahrungsaufnahme, Anorexie, Fettsucht, lokomotorischen Störungen, Diarrhoe, Kachexie, Harninkontinenz bzw. als Muskelrelaxanz, Antikonvulsivum oder Anesthetikum bzw. zur Coadministration bei Behandlung mit einem opioiden Analgetikum oder mit einem Anesthetikum, zur Diurese oder Antinatriurese, Anxiolyse, zur Modulation der Bewegungsaktivität, zur Modulation der Neurotransmitter-Ausschüttung und Behandlung damit verbundener neurodegenerativer Erkrankungen, zur Behandlung von Entzugserscheinungen und/oder zur Reduzierung des Suchtpotentials von Opioiden.

## Claims

1. Substituted cyclohexyl-1,4-diamine derivatives of general formula I 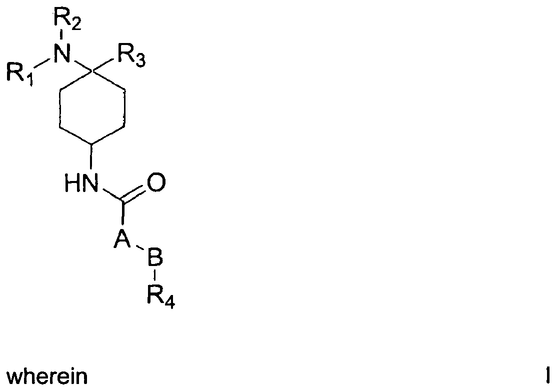 wherein
R¹ and R² independently of one another represent H; respectively saturated or unsaturated, branched or unbranched, singly or multiply substituted or unsubstituted C₁₋₅ alkyl; respectively singly or multiply substituted or unsubstituted C₃₋₈ cycloalkyl; or respectively singly or multiply substituted or unsubstituted aryl, C₃₋₈ cycloalkyl or heteroaryl bound by C₁₋₃ alkyl
or the radicals R¹ and R² together represent CH₂CH₂OCH₂CH₂, CH₂CH₂NR¹⁰CH₂CH₂ or (CH₂)₃₋₆,
wherein R¹⁰ represents H; respectively saturated or unsaturated, branched or unbranched, singly or multiply substituted or unsubstituted C₁₋₅ alkyl; respectively singly or multiply substituted or unsubstituted C₃₋₈ cycloalkyl; respectively singly or multiply substituted or unsubstituted aryl or heteroaryl; or respectively singly or multiply substituted or unsubstituted aryl, C₃₋₈ cycloalkyl or heteroaryl bound by C₁₋₃ alkyl; respectively substituted or unsubstituted C(O)phenyl,C(O)heteroaryl, C(O)C₁₋₅ alkyl;
R³ represents respectively saturated or unsaturated, branched or unbranched, singly or multiply substituted or unsubstituted C₁₋₅ alkyl; respectively singly or multiply substituted or unsubstituted C₃₋₈ cycloalkyl; respectively unsubstituted or singly or multiply substituted aryl or heteroaryl; respectively unsubstituted or singly or multiply substituted aryl, heteroaryl or C₃₋₈ cycloalkyl bound by a C₁₋₃ alkyl group;
A represents unsubstituted or singly or multiply substituted heteroaryl,
B represents (CH₂)ₘ, with m = 0, 1; SO₂, O, S, C(O), C(S),
R⁴ represents unsubstituted or singly or multiply substituted heteroaryl or aryl, or an unsubstituted or singly or multiply substituted heteroaryl or aryl radical linked by a C₁₋₃ alkyl chain
in the form of the racemate; the enantiomers, diastereomers, mixtures of the enantiomers or diastereomers or an individual enantiomer or diastereomer; the bases and/or salts of physiologically acceptable acids or cations.

2. Substituted cyclohexyl-1,4-diamine derivatives according to claim 1, **characterised in that**
R¹ and R² independently of one another represent H; saturated or unsaturated, branched or unbranched, singly or multiply substituted or unsubstituted C₁₋₅ alkyl;
or the radicals R¹ and R² together form a ring and represent CH₂CH₂OCH₂CH₂, CH₂CH₂NR¹⁰CH₂CH₂ or (CH₂)₃₋₆.
wherein R¹⁰ represents H; saturated or unsaturated, branched or unbranched, singly or multiply substituted or unsubstituted C₁₋₅ alkyl.

3. Substituted cyclohexyl-1,4-diamine derivatives according to either claim 1 or claim 2, **characterised in that** R¹ and R² independently of one another represent CH₃ or H, wherein R¹ and R² do not simultaneously represent H, or R¹ and R² represent CH₂CH₂OCH₂CH₂, (CH₂)₄, (CH₂)₅ or (CH₂)₆.

4. Substituted cyclohexyl-1,4-diamine derivatives according to any one of claims 1 to 3, **characterised in that** R³ represents respectively unsubstituted or singly or multiply substituted cyclopentyl, cyclohexyl, phenyl, benzyl, naphthyl, anthracenyl, thiophenyl, benzothiophenyl, furyl, benzofuranyl, benzodioxolanyl, indolyl, indanyl, benzodioxanyl, pyrrolyl, pyridyl, pyrimidyl or pyrazinyl; respectively unsubstituted or singly or multiply substituted C₅₋₆ cycloalkyl, phenyl, naphthyl, anthracenyl, thiophenyl, benzothiophenyl, pyridyl, furyl, benzofuranyl, benzodioxolanyl, indolyl, indanyl, benzodioxanyl, pyrrolyl, pyrimidyl or pyrazinyl bound by a saturated, unbranched C₁₋₂ alkyl group;
in particular
R³ represents respectively unsubstituted or singly or multiply substituted phenyl, furyl, thiophenyl, naphthyl, benzyl, benzofuranyl, indolyl, indanyl, benzodioxanyl, benzodioxolanyl, pyridyl, pyrimidyl, pyrazinyl or benzothiophenyl; respectively unsubstituted or singly or multiply substituted phenyl, furyl or thiophenyl bound by a saturated, unbranched C₁₋₂ alkyl group.

5. Substituted cyclohexyl-1,4-diamine derivatives according to any one of claims 1 to 4, **characterised in that** R³ represents respectively substituted or unsubstituted phenyl, phenethyl, thiophenyl, pyridyl or benzyl.

6. Substituted cyclohexyl-1,4-diamine derivatives according to any one of claims 1 to 4, **characterised in that** R³ represents 4-methylbenzyl, 2-methylbenzyl, 3-chlorobenzyl, 4-chlorobenzyl, 2-chlorobenzyl, 4-fluorobenzyl, 3-fluorobenzyl, 3-methylbenzyl, 2-fluorobenzyl, benzyl, phenethyl, phenyl, pyridyl, thiophenyl and 3-fluorophenyl.

7. Substituted cyclohexyl-1,4-diamine derivatives according to any one of claims 1 to 6, **characterised in that** A represents unsubstituted or singly or multiply substituted pyrrolyl, thiophenyl, furanyl, pyrazolyl, isoxazolyl, oxazolyl, isothiazolyl, imidazolyl, triazolyl, tetrahydroisoxazolyl, pyridinyl, pyrimidinyl, pyrazinyl, triazinyl, indolyl, benzofuranyl, benzothiophenyl, indazolyl, benzimidazolyl, purinyl, quinolinyl, isoquinolinyl, phthalazine, benzothiadiazolyl, benzothiazolyl, benzotriazolyl, benzodioxolanyl, benzodioxanyl and carbazolyl.

8. Substituted cyclohexyl-1,4-diamine derivatives according to any one of claims 1 to 6, **characterised in that** A represents pyridyl, thiophenyl, pyrazolyl, triazolyl, tetrahydroisoxazolyl, isoxazolyl, thiazolyl and furanyl.

9. Substituted cyclohexyl-1,4-diamine derivatives according to any one of claims 1 to 8, **characterised in that** B represents (CH₂)ₘ, with m = 0, 1; SO₂, O, S.

10. Substituted cyclohexyl-1,4-diamine derivatives according to any one of claims 1 to 9, **characterised in that** R⁴ represents respectively unsubstituted or singly or multiply substituted phenyl, benzyl, naphthyl, anthracenyl, thiophenyl, benzothiophenyl, furanyl, isothiazolyl, imidazolyl, triazolyl, triazinyl, pyrazolyl, benzofuranyl, benzodioxolanyl, isoquinolinyl, phthalazine, benzo[1,2,5]thiadiazole, benzothiazole, benzotriazole, quinolinyl, carbazole, isoxazolyl, oxazolyl, indolyl, indanyl, benzodioxanyl, indazolyl, benzimidazolyl, pyrrolyl, pyridyl, pyrimidyl or pyrazinyl; respectively unsubstituted or singly or multiply substituted phenyl, naphthyl, anthracenyl, thiophenyl, benzothiophenyl, pyridyl, furyl, benzofuranyl, indolyl, indanyl, benzodioxanyl, pyrrolyl, pyrimidyl or pyrazinyl bound by a saturated, unbranched C₁₋₂ alkyl group,
in particular
R⁴ represents respectively unsubstituted or singly or multiply substituted phenyl, benzyl, naphthyl, thiophenyl, benzothiophenyl, furanyl, isothiazolyl, imidazolyl, triazolyl, pyrazolyl, benzofuranyl, isoquinolinyl, benzothiazole, benzotriazole, quinolinyl, isoxazolyl, oxazolyl, indolyl, pyrrolyl, pyridyl, pyrimidyl or pyrazinyl; respectively unsubstituted or singly or multiply substituted benzyl or phenethyl.

11. Substituted cyclohexyl-1,4-diamine derivatives according to any one of claims 1 to 9, **characterised in that** R⁴ represents unsubstituted or singly or multiply substituted phenyl, pyrazolyl or thiophenyl.

12. Substituted cyclohexyl-1,4-diamine derivatives according to any one of claims 1 to 11 from the group comprising
5-methyl-2-phenyl-2H-[1,2,3]triazole-4-carboxylic acid (4-dimethylamino-4-phenylcyclohexyl)-amide
1-phenyl-5-propyl-1H-pyrazole-4-carboxylic acid [4-dimethylamino-4-(3-fluorophenyl)-cyclohexyl]-amide
3-(2,6-dichloro-phenyl)-5-methyl-isoxazole-4-carboxylic acid (4-benzyl-4-pyrrolidin-1-yl-cyclohexyl)-amide
5-methyl-2-phenyl-2H-[1,2,3]triazole-4-carboxylic acid (4-benzyl-4-piperidin-1-yl-cyclohexyl)-amide
5-methyl-2-phenyl-2H-[1,2,3]triazole-4-carboxylic acid (4-phenyl-4-piperidin-1-yl-cyclohexyl)-amide
N-(4-dimethylamino-4-phenyl-cyclohexyl)-2-phenoxy-nicotinamide
2-(4-chloro-phenylsulphanyl)-N-(4-dimethylamino-4-pyridin-2-yl-cyclohexyl)-nicotinamide
N-(4-dimethylamino-4-thiophen-2-yl-cyclohexyl)-2-(5-methyl-2-phenyl-thiazol-4-yl)-acetamide
2-(4-chloro-phenylsulphanyl)-N-[4-dimethylamino-4-(3-fluoro-phenyl)-cyclohexyl]-nicotinamide
N-(4-dimethylamino-4-thiophen-2-yl-cyclohexyl)-2-phenoxy-nicotinamide
3-(2-chloro-phenyl)-5-methyl-isoxazole-4-carboxylic acid (4-dimethylamino-4-phenyl-cyclohexyl)-amide
5-(4-chloro-phenyl)-2-methyl-furan-3-carboxylic acid [4-dimethylamino-4-(3-fluorophenyl)-cyclohexyl]-amide
3-(2-chloro-6-fluoro-phenyl)-5-methyl-isoxazole-4-carboxylic acid (4-dimethylamino-4-phenethyl-cyclohexyl)-amide
N-(4-benzyl-4-pyrrolidin-1-yl-cyclohexyl)-2-(4-chloro-phenylsulphanyl)-nicotinamide
3-(2-chloro-6-fluoro-phenyl)-5-methyl-isoxazole-4-carboxylic acid (4-dimethylamino-4-phenyl-cyclohexyl)-amide
3-(2,6-dichloro-phenyl)-5-methyl-isoxazole-4-carboxylic acid (4-benzyl-4-piperidin-1-yl-cyclohexyl)-amide
N-(4-dimethylamino-4-phenethyl-cyclohexyl)-2-(5-methyl-2-phenyl-thiazol-4-yl)-acetamide
3-(2-chloro-phenyl)-5-methyl-isoxazole-4-carboxylic acid (4-dimethylamino-4-phenethyl-cyclohexyl)-amide
5-benzyl-furan-2-carboxylic acid (4-benzyl-4-dimethylamino-cyclohexyl)-amide
1-(4-chloro-phenyl)-5-trifluoromethyl-1H-pyrazole-4-carboxylic acid [4-dimethylamino-4-(3-fluoro-phenyl)-cyclohexyl]-amide
N-(4-dimethylamino-4-pyridin-2-yl-cyclohexyl)-2-phenoxy-nicotinamide
5-benzyl-furan-2-carboxylic acid (4-phenyl-4-piperidin-1-yl-cyclohexyl)-amide
5-methyl-2-phenyl-2H-[1,2,3]triazole-4-carboxylic acid [4-dimethylamino-4-(2-methyl-benzyl)-cyclohexyl]-amide
2-(4-chloro-phenylsulphanyl)-N-[4-dimethylamino-4-(2-fluoro-benzyl)-cyclohexyl]-nicotinamide
3-(2,6-dichloro-phenyl)-5-methyl-isoxazole-4-carboxylic acid [4-dimethylamino-4-(2-fluoro-benzyl)-cyclohexyl]-amide
5-methyl-2-phenyl-2H-[1,2,3]triazole-4-carboxylic acid [4-dimethylamino-4-(4-fluorobenzyl)-cyclohexyl]-amide
5-benzyl-furan-2-carboxylic acid [4-dimethylamino-4-(2-fluoro-benzyl)-cyclohexyl]-amide
3-(2-chloro-phenyl)-5-methyl-isoxazole-4-carboxylic acid [4-dimethylamino-4-(3-fluoro-phenyl)-cyclohexyl]-amide
3-(2-chloro-6-fluoro-phenyl)-5-methyl-isoxazole-4-carboxylic acid [4-dimethylamino-4-(3-fluoro-phenyl)-cyclohexyl]-amide
N-(4-dimethylamino-4-phenethyl-cyclohexyl)-2-phenoxy-nicotinamide
3-(2-chloro-phenyl)-5-methyl-isoxazole-4-carboxylic acid [4-(3-chloro-benzyl)-4-dimethylamino-cyclohexyl]-amide
3-(2,6-dichloro-phenyl)-5-methyl-isoxazole-4-carboxylic acid [4-dimethylamino-4-(3-fluoro-phenyl)-cyclohexyl]-amide
5-benzyl-furan-2-carboxylic acid (4-dimethylamino-4-phenethyl-cyclohexyl)-amide
5-methyl-2-phenyl-2H-[1,2,3]triazole-4-carboxylic acid [4-dimethylamino-4-(4-methyl-benzyl)-cyclohexyl]-amide
5-benzyl-furan-2-carboxylic acid [4-dimethylamino-4-(2-methyl-benzyl)-cyclohexyl]-amide
3-(2,6-dichloro-phenyl)-5-methyl-isoxazole-4-carboxylic acid (4-dimethylamino-4-phenethyl-cyclohexyl)-amide
N-(4-benzyl-4-pyrrolidin-1-yl-cyclohexyl)-2-phenoxy-nicotinamide
5-benzyl-furan-2-carboxylic acid [4-dimethylamino-4-(3-methyl-benzyl)-cyclohexyl]-amide
5-benzyl-furan-2-carboxylic acid [4-dimethylamino-4-(3-fluoro-benzyl)-cyclohexyl]-amide
N-[4-(3-chloro-benzyl)-4-dimethylamino-cyclohexyl]-2-(4-chloro-phenylsulphanyl)-nicotinamide
2-(5-methyl-2-phenyl-thiazol-4-yl)-N-(4-phenyl-4-piperidin-1-yl-cyclohexyl)-acetamide
5-methyl-2-phenyl-2H-[1,2,3]triazole-4-carboxylic acid [4-dimethylamino-4-(3-methyl-benzyl)-cyclohexyl]-amide
N-[4-dimethylamino-4-(2-methyl-benzyl)-cyclohexyl]-2-(5-methyl-2-phenyl-thiazol-4-yl)-acetamide
1-(4-chloro-phenyl)-5-trifluoromethyl-1H-pyrazole-4-carboxylic acid [4-(3-chlorobenzyl)-4-dimethylamino-cyclohexyl]-amide
5-methyl-2-phenyl-2H-[1,2,3]triazole-4-carboxylic acid [4-(3-chloro-benzyl)-4-dimethylamino-cyclohexyl]-amide
5-methyl-2-phenyl-2H-[1,2,3]triazole-4-carboxylic acid (4-dimethylamino-4-phenethyl-cyclohexyl)-amide
3-(2-chloro-6-fluoro-phenyl)-5-methyl-isoxazole-4-carboxylic acid (4-benzyl-4-pyrrolidin-1-yl-cyclohexyl)-amide
N-[4-dimethylamino-4-(2-fluoro-benzyl)-cyclohexyl]-2-phenoxy-nicotinamide
N-[4-dimethylamino-4-(3-fluoro-benzyl)-cyclohexyl]-2-(5-methyl-2-phenyl-thiazol-4-yl)-acetamide
N-(4-benzyl-4-dimethylamino-cyclohexyl)-2-phenoxy-nicotinamide
5-(thiophene-2-sulphonyl]-4,5,6,7-tetrahydro-isoxazolo[4,5-c]pyridine-3-carboxylic acid (4-benzyl-4-dimethylamino-cyclohexyl)-amide
1-(4-chloro-phenyl)-5-trifluoromethyl-1H-pyrazole-4-carboxylic acid [4-dimethylamino-4-(2-fluoro-benzyl)-cyclohexyl]-amide
5-benzyl-furan-2-carboxylic acid [4-(4-chloro-benzyl)-4-dimethylamino-cyclohexyl]-amide
5-methyl-2-phenyl-2H-[1,2,3]triazole-4-carboxylic acid (4-benzyl-4-dimethylamino-cyclohexyl)-amide
N-(4-benzyl-4-dimethylamino-cyclohexyl)-2-(5-methyl-2-phenyl-thiazol-4-yl)-acetamide
N-[4-dimethylamino-4-(2-fluoro-benzyl)-cyclohexyl]-2-(5-methyl-2-phenyl-thiazol-4-yl)-acetamide
3-(2-chloro-phenyl)-5-methyl-isoxazole-4-carboxylic acid [4-dimethylamino-4-(2-fluoro-benzyl)-cyclohexyl]-amide
5-methyl-2-phenyl-2H-[1,2,3]triazole-4-carboxylic acid [4-(2-chloro-benzyl)-4-dimethylamino-cyclohexyl]-amide
2-(4-chloro-phenylsulphanyl)-N-[4-dimethylamino-4-(3-fluoro-benzyl)-cyclohexyl]-nicotinamide
3-(2,6-dichloro-phenyl)-5-methyl-isoxazole-4-carboxylic acid (4-benzyl-4-dimethylamino-cyclohexyl)-amide
N-[4-(3-chloro-benzyl)-4-dimethylamino-cyclohexyl]-2-phenoxy-nicotinamide
2-phenoxy-N-(4-phenyl-4-piperidin-1-yl-cyclohexyl)-nicotinamide
5-(4-chloro-phenyl)-2-methyl-furan-3-carboxylic acid (4-benzyl-4-pyrrolidin-1-yl-cyclohexyl)-amide
5-(3-trifluoromethyl-benzylsulphonyl)-4,5,6,7-tetrahydro-isoxazolo[4,5-c]pyridine-3-carboxylic acid (4-benzyl-4-dimethylamino-cyclohexyl)-amide
N-[4-dimethylamino-4-(4-methyl-benzyl)-cyclohexyl]-2-phenoxy-nicotinamide
N-[4-dimethylamino-4-(3-fluoro-benzyl)-cyclohexyl]-2-phenoxy-nicotinamide
3-(2-chloro-6-fluoro-phenyl)-5-methyl-isoxazole-4-carboxylic acid (4-phenyl-4-piperidin-1-yl-cyclohexyl)-amide
5-(4-chloro-phenyl)-2-methyl-furan-3-carboxylic acid [4-dimethylamino-4-(4-fluorobenzyl)-cyclohexyl]-amide
1-(4-chloro-phenyl)-5-trifluoromethyl-1H-pyrazole-4-carboxylic acid [4-dimethylamino-4-(4-methyl-benzyl)-cyclohexyl]-amide
5-methyl-2-phenyl-2H-[1,2,3]triazole-4-carboxylic acid [4-(4-chloro-benzyl)-4-dimethylamino-cyclohexyl]-amide
5-methyl-2-phenyl-2H-[1,2,3]triazole-4-carboxylic acid [4-dimethylamino-4-(3-fluorobenzyl)-cyclohexyl]-amide
3-(2,6-dichloro-phenyl)-5-methyl-isoxazole-4-carboxylic acid (4-phenyl-4-piperidin-1-yl-cyclohexyl)-amide
1-(4-chloro-phenyl)-5-trifluoromethyl-1H-pyrazole-4-carboxylic acid (4-morpholin-4-yl-phenyl-cyclohexyl)-amide
N-[4-(4-chloro-benzyl)-4-dimethylamino-cyclohexyl]-2-phenoxy-nicotinamide
N-[4-(4-chloro-benzyl)-4-dimethylamino-cyclohexyl]-2-(5-methyl-2-phenyl-thiazol-4-yl)-acetamide
3-(2-chloro-phenyl)-5-methyl-isoxazole-4-carboxylic acid (4-azepan-1-yl-4-benzyl-cyclohexyl)-amide
3-(2-chloro-6-fluoro-phenyl)-5-methyl-isoxazole-4-carboxylic acid [4-dimethylamino-4-(4-fluoro-benzyl)-cyclohexyl]-amide
3-(2-chloro-6-fluoro-phenyl)-5-methyl-isoxazole-4-carboxylic acid [4-(3-chlorobenzyl)-4-dimethylamino-cyclohexyl]-amide
3-(2-chloro-phenyl)-5-methyl-isoxazole-4-carboxylic acid [4-dimethylamino-4-(2-methyl-benzyl)-cyclohexyl]-amide
3-(2-chloro-6-fluoro-phenyl)-5-methyl-isoxazole-4-carboxylic acid [4-dimethylamino-4-(2-methylbenzyl)-cyclohexyl]-amide
5-(1-methyl-5-trifluoromethyl-1H-pyrazol-3-yl)-thiophene-2-carboxylic acid (4-benzyl-4-piperidin-1-yl-cyclohexyl)-amide
3-(2-chloro-phenyl)-5-methyl-isoxazole-4-carboxylic acid [4-dimethylamino-4-(4-fluoro-benzyl)-cyclohexyl]-amide
3-(2,6-dichloro-phenyl)-5-methyl-isoxazole-4-carboxylic acid [4-dimethylamino-4-(4-methyl-benzyl)-cyclohexyl]-amide
3-(2-chloro-phenyl)-5-methyl-isoxazole-4-carboxylic acid (4-benzyl-4-pyrrolidin-1-yl-cyclohexyl)-amide
3-(2-chloro-6-fluoro-phenyl)-5-methyl-isoxazole-4-carboxylic acid [4-dimethylamino-4-(3-fluorobenzyl)-cyclohexyl]-amide
3-(2-chloro-6-fluoro-phenyl)-5-methyl-isoxazole-4-carboxylic acid [4-dimethylamino-4-(4-methyl-benzyl)-cyclohexyl]-amide
1-(4-chloro-phenyl)-5-trifluoromethyl-1H-pyrazole-4-carboxylic acid [4-(4-chlorobenzyl)-4-dimethylamino-cyclohexyl]-amide
1-phenyl-5-propyl-1H-pyrazole-4-carboxylic acid (4-azepan-1-yl-4-benzyl-cyclohexyl)-amide
3-(2-chloro-phenyl)-5-methyl-isoxazole-4-carboxylic acid [4-dimethylamino-4-(3-fluoro-benzyl)-cyclohexyl]-amide
1-phenyl-5-propyl-1H-pyrazole-4-carboxylic acid [4-(2-chloro-benzyl)-4-dimethylamino-cyclohexyl]-amide
3-(2-chloro-6-fluoro-phenyl)-5-methyl-isoxazole-4-carboxylic acid (4-benzyl-4-dimethylamino-cyclohexyl)-amide
3-(2,6-dichloro-phenyl)-5-methyl-isoxazole-4-carboxylic acid [4-dimethylamino-4-(3-fluoro-benzyl)-cyclohexyl]-amide
1-phenyl-5-propyl-1H-pyrazole-4-carboxylic acid [4-dimethylamino-4-(4-fluorobenzyl)-cyclohexyl]-amide
1-(4-chloro-phenyl)-5-trifluoromethyl-1H-pyrazole-4-carboxylic acid (4-benzyl-4-dimethylamino-cyclohexyl)-amide
5-(1-methyl-5-trifluoromethyl-1H-pyrazol-3-yl)-thiophene-2-carboxylic acid [4-dimethylamino-4-(3-fluoro-benzyl)-cyclohexyl]-amide
3-(2-chloro-6-fluoro-phenyl)-5-methyl-isoxazole-4-carboxylic acid [4-dimethylamino-4-(2-fluoro-benzyl)-cyclohexyl]-amide
1-(4-chloro-phenyl)-5-trifluoromethyl-1H-pyrazole-4-carboxylic acid (4-dimethylamino-4-phenethyl-cyclohexyl)-amide
5-(1-methyl-5-trifluoromethyl-1H-pyrazol-3-yl)-thiophene-2-carboxylic acid [4-(3-chloro-benzyl)-4-dimethylamino-cyclohexyl]-amide
5-(1-methyl-5-trifluoromethyl-1H-pyrazol-3-yl)-thiophene-2-carboxylic acid [4-dimethylamino-4-(2-fluoro-benzyl)-cyclohexyl]-amide
5-(1-methyl-5-trifluoromethyl-1H-pyrazol-3-yl)-thiophene-2-carboxylic acid [4-(4-chloro-benzyl)-4-dimethylamino-cyclohexyl]-amide
5-(1-methyl-5-trifluoromethyl-1H-pyrazol-3-yl)-thiophene-2-carboxylic acid [4-dimethylamino-4-(4-methyl-benzyl)-cyclohexyl]-amide
5-benzyl-furan-2-carboxylic acid [4-dimethylamino-4-(4-methyl-benzyl)-cyclohexyl]-amide
5-methyl-2-phenyl-2H-[1,2,3]triazole-4-carboxylic acid (4-azepan-1-yl-4-benzyl-cyclohexyl)-amide
2-(4-chloro-phenylsulphanyl)-N-[4-dimethylamino-4-(4-fluoro-benzyl)-cyclohexyl]-nicotinamide
N-[4-dimethylamino-4-(4-fluoro-benzyl)-cyclohexyl]-2-phenoxy-nicotinamide
5-benzyl-furan-2-carboxylic acid (4-benzyl-4-piperidin-1-yl-cyclohexyl)-amide
3-(2,6-dichloro-phenyl)-5-methyl-isoxazole-4-carboxylic acid [4-(4-chloro-benzyl)-4-dimethylamino-cyclohexyl]-amide
N-(4-benzyl-4-morpholin-4-yl-cyclohexyl)-2-(4-chloro-phenylsulphanyl)-nicotinamide
5-(1-methyl-5-trifluoromethyl-1H-pyrazol-3-yl)-thiophene-2-carboxylic acid (4-benzyl-4-dimethylamino-cyclohexyl)-amide
1-(4-chloro-phenyl)-5-trifluoromethyl-1H-pyrazole-4-carboxylic acid [4-dimethylamino-4-(4-fluoro-benzyl)-cyclohexyl]-amide
1-(4-chloro-phenyl)-5-trifluoromethyl-1H-pyrazole-4-carboxylic acid [4-dimethylamino-4-(2-methyl-benzyl)-cyclohexyl]-amide
N-(4-azepan-1-yl-4-benzyl-cyclohexyl)-2-phenoxy-nicotinamide
3-(2,6-dichloro-phenyl)-5-methyl-isoxazole-4-carboxylic acid [4-(2-chloro-benzyl)-4-dimethylamino-cyclohexyl]-amide
N-[4-dimethylamino-4-(2-methyl-benzyl)-cyclohexyl]-2-phenoxy-nicotinamide
3-(2,6-dichloro-phenyl)-5-methyl-isoxazole-4-carboxylic acid [4-dimethylamino-4-(4-fluoro-benzyl)-cyclohexyl]-amide
2-(4-chloro-phenylsulphanyl)-N-[4-dimethylamino-4-(2-methyl-benzyl)-cyclohexyl]-nicotinamide
3-(2,6-dichloro-phenyl)-5-methyl-isoxazole-4-carboxylic acid [4-dimethylamino-4-(2-methyl-benzyl)-cyclohexyl]-amide
3-(2-chlorophenyl)-5-methylisoxazole-4-carboxylic acid [4-dimethylamino-4-(3-fluorophenyl)cyclo-hexyl]amide hydrochloride, non-polar diastereoisomer
3-(2-chlorophenyl)-5-methylisoxazole-4-carboxylic acid [4-dimethylamino-4-(3-fluorophenyl)cyclo-hexyl]amide hydrochloride, polar diastereoisomer
5-methyl-2-phenyl-2H-[1,2,3]triazole-4-carboxylic acid (4-dimethylamino-4-phenylcyclohexyl)-amide hydrochloride, non-polar diastereoisomer
5-methyl-2-phenyl-2H-[1,2,3]triazole-4-carboxylic acid (4-dimethylamino-4-phenylcyclohexyl)-amide hydrochloride, polar diastereoisomer
3-(2,6-dichlorophenyl)-5-methylisoxazole-4-carboxylic acid [4-dimethylamino-4-(3-fluorophenyl)-cyclohexyl]amide hydrochloride, non-polar diastereoisomer
3-(2,6-dichlorophenyl)-5-methylisoxazole-4-carboxylic acid [4-dimethylamino-4-(3-fluorophenyl)-cyclohexyl]amide hydrochloride, polar diastereoisomer
3-(2,6-dichlorophenyl)-5-methylisoxazole-4-carboxylic acid (4-dimethylamino-4-phenylcyclohexyl)amide hydrochloride, non-polar diastereoisomer
3-(2,6-dichlorophenyl)-5-methylisoxazole-4-carboxylic acid (4-dimethylamino-4-phenylcyclohexyl)amide hydrochloride, polar diastereoisomer
5-methyl-3-phenylisoxazole-4-carboxylic acid [4-dimethylamino-4-(3-fluorophenyl)cyclohexyl]amide hydrochloride, non-polar diastereoisomer
5-methyl-3-phenylisoxazole-4-carboxylic acid (4-dimethylamino-4-phenylcyclohexyl)amide hydrochloride, non-polar diastereoisomer
5-methyl-3-phenylisoxazole-4-carboxylic acid (4-dimethylamino-4-phenylcyclohexyl)amide hydrochloride, polar diastereoisomer
in the form of the racemate; the enantiomers, diastereomers, mixtures of the enantiomers or diastereomers or an individual enantiomer or diastereomer; the bases and/or salts of physiologically acceptable acids or cations.

13. Process for the production of substituted cyclohexyl-1,4-diamine derivatives according to any one of claims 1 to 12, **characterised in that** substituted cyclohexane-1,4-diamines are linked with carboxylic acids of general formula II 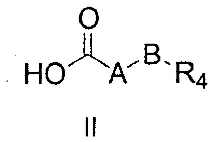 by adding coupling reagents or by activating the carboxylic acid component, in particular by producing the acid chloride.

14. Pharmaceutical composition containing at least one substituted cyclohexyl-1,4-diamine derivative according to any one of claims 1 to 12, optionally in the form of its racemate, the pure stereoisomers, in particular enantiomers and diastereomers, in any desired mixing ratio; in the form of its acids or its bases or in the form of its salts, in particular the physiologically acceptable salts or salts of physiologically acceptable acids or cations, or in the form of its solvates, in particular the hydrates, optionally containing suitable additives and/or auxiliaries and/or optionally further active ingredients.

15. Substituted cyclohexyl-1,4-diamine derivatives according to any one of claims 1 to 12, optionally in the form of the racemate thereof, the pure stereoisomers, in particular enantiomers and diastereomers, in any desired mixing ratio; in the form of the acids or the bases thereof or in the form of the salts thereof, in particular the physiologically acceptable salts or salts of physiologically acceptable acids or cations; or in the form if the solvates thereof, in particular the hydrates; for use in the treatment of pain, in particular acute, neuropathic or chronic pain, for use in the treatment of anxiety, stress and stress-related syndromes, depression, epilepsy, Alzheimer's disease, senile dementia, catalepsy, general cognitive dysfunction, learning and memory difficulties (as a nootropic), withdrawal symptoms, alcohol- and/or drug- and/or medicines abuse and/or dependency, sexual dysfunction, cardiovascular diseases, hypotension, hypertension, tinnitus, pruritus, migraine, hearing difficulties, deficient intestinal motility, impaired absorption of nutrients, anorexia, obesity, locomotive disorders, diarrhoea, cachexia, urinary incontinence, or as a muscle relaxant, anti-convulsive or anaesthetic for co-administration in treatment with an opioid analgesic or with an anaesthetic, for diuresis or anti-natriuresis, anxiolysis, for modulation of motor activity, for modulation of neurotransmitter release and treatment of neurodegenerative diseases associated therewith, for the treatment of withdrawal symptoms and/or for reducing the potential for addiction to opioids.

## Revendications

1. Dérivés de cyclohexyl-1,4-diamine substitués de formule générale I 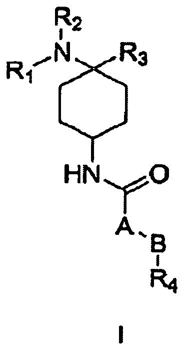 dans laquelle
R¹ et R² représentent indépendamment l'un de l'autre H ; alkyle en C₁₋₅ à chaque fois saturé ou insaturé, ramifié ou non ramifié, substitué une ou plusieurs fois ou non substitué ; cycloalkyle en C₃₋₈, à chaque fois substitué une ou plusieurs fois ou non substitué ; ou aryle, cycloalkyle en C₃₋₈ ou hétéroaryle relié par alkyle en C₁₋₃, à chaque fois substitué une ou plusieurs fois ou non substitué ;
ou les radicaux R¹ et R² représentent ensemble CH₂CH₂OCH₂CH₂, CH₂CH₂NR¹⁰CH₂CH₂ ou (CH₂)₃₋₆,
R¹⁰ signifiant H ; alkyle en C₁₋₅, à chaque fois saturé ou insaturé, ramifié ou non ramifié, substitué une ou plusieurs fois ou non substitué ; cycloalkyle en C₃₋₈, à chaque fois substitué une ou plusieurs fois ou non substitué ; aryle ou hétéroaryle, à chaque fois substitué une ou plusieurs fois ou non substitué ; ou aryle, cycloalkyle en C₃₋₈ ou hétéroaryle relié par alkyle en C₁₋₃, à chaque fois substitué une ou plusieurs fois ou non substitué ; C(O)phényle, C(O)hétéroaryle, C(O)alkyle en C₁₋₅, à chaque fois substitué ou non substitué ;
R³ représente alkyle en C₁₋₅, à chaque fois saturé ou insaturé, ramifié ou non ramifié, substitué une ou plusieurs fois ou non substitué ; cycloalkyle en C₃₋₈, à chaque fois substitué une ou plusieurs fois ou non substitué ; aryle ou hétéroaryle, à chaque fois non substitué ou substitué une ou plusieurs fois ; aryle, hétéroaryle ou cycloalkyle en C₃₋₈ relié par un groupe alkyle en C₁₋₃, à chaque fois non substitué ou substitué une ou plusieurs fois ;
A représente hétéroaryle, non substitué ou substitué une ou plusieurs fois ;
B représente (CH₂)ₘ avec m = 0, 1 ; SO₂, O, S, C(O), C(S) ;
R⁴ représente hétéroaryle ou aryle, non substitué ou substitué une ou plusieurs fois ; ou un radical hétéroaryle ou aryle relié par une chaîne alkyle en C₁₋₃, non substitué ou substitué une ou plusieurs fois ;
sous la forme du racémat ; des énantiomères, diastéréomères, mélanges des énantiomères ou diastéréomères ou d'un énantiomère ou diastéréomère individuel ; des bases et/ou des sels d'acides ou de cations physiologiquement acceptables.

2. Dérivés de cyclohexyl-1,4-diamine substitués selon la revendication 1, **caractérisés en ce que** R¹ et R² représentent indépendamment l'un de l'autre H ; alkyle en C₁₋₅ saturé ou insaturé, ramifié ou non ramifié, substitué une ou plusieurs fois ou non substitué ;
ou les radicaux R¹ et R² forment ensemble un cycle et signifient CH₂CH₂OCH₂CH₂, CH₂CH₂NR¹⁰CH₂CH₂ ou (CH₂)₃₋₆,
R¹⁰ signifiant H ; alkyle en C₁₋₅ saturé ou insaturé, ramifié ou non ramifié, substitué une ou plusieurs fois ou non substitué.

3. Dérivés de cyclohexyl-1,4-diamine substitués selon l'une quelconque des revendications 1 ou 2, **caractérisés en ce que** R¹ et R² représentent indépendamment l'un de l'autre CH₃ ou H, R¹ et R² ne signifiant pas simultanément H, ou R¹ et R² représentent CH₂CH₂OCH₂CH₂, (CH₂)₄, (CH₂)₅ ou (CH₂)₆.

4. Dérivés de cyclohexyl-1,4-diamine substitués selon l'une quelconque des revendications 1 à 3, **caractérisés en ce que**
R³ représente cyclopentyle, cyclohexyle, phényle, benzyle, naphtyle, anthracényle, thiophényle, benzothiophényle, furyle, benzofuranyle, benzodioxolanyle, indolyle, indanyle, benzodioxanyle, pyrrolyle, pyridyle, pyrimidyle ou pyrazinyle, à chaque fois non substitué ou substitué une ou plusieurs fois ; cycloalkyle en C₅₋₆, phényle, naphtyle, anthracényle, thiophényle, benzothiophényle, pyridyle, furyle, benzofuranyle, benzodioxolanyle, indolyle, indanyle, benzodioxanyle, pyrrolyle, pyrimidyle ou pyrazinyle relié par un groupe alkyle en C₁₋₂ saturé non ramifié, à chaque fois non substitué ou substitué une ou plusieurs fois ;
notamment
R³ signifie phényle, furyle, thiophényle, naphtyle, benzyle, benzofuranyle, indolyle, indanyle, benzodioxanyle, benzodioxolanyle, pyridyle, pyrimidyle, pyrazinyle ou benzothiophényle, à chaque fois non substitué ou substitué une ou plusieurs fois ; phényle, furyle ou thiophényle relié par un groupe alkyle en C₁₋₂ saturé non ramifié, à chaque fois non substitué ou substitué une ou plusieurs fois.

5. Dérivés de cyclohexyl-1,4-diamine substitués selon l'une quelconque des revendications 1 à 4, **caractérisés en ce que** R³ représente phényle, phénéthyle, thiophényle, pyridyle ou benzyle, à chaque fois substitué ou non substitué.

6. Dérivés de cyclohexyl-1,4-diamine substitués selon l'une quelconque des revendications 1 à 4, **caractérisés en ce que** R³ représente 4-méthylbenzyle, 2-méthylbenzyle, 3-chlorobenzyle, 4-chlorobenzyle, 2-chlorobenzyle, 4-fluorobenzyle, 3-fluorobenzyle, 3-méthylbenzyle, 2-fluorobenzyle, benzyle, phénéthyle, phényle, pyridyle, thiophényle et 3-fluorophényle.

7. Dérivés de cyclohexyl-1,4-diamine substitués selon l'une quelconque des revendications 1 à 6, **caractérisés en ce que** A représente pyrrolyle, thiophényle, furanyle, pyrazolyle, isoxazolyle, oxazolyle, isothiazolyle, imidazolyle, triazolyle, tétrahydroisoxazolyle, pyridinyle, pyrimidinyle, pyrazinyle, triazinyle, indolyle, benzofuranyle, benzothiophényle, indazolyle, benzimidazolyle, purinyle, quinoléinyle, isoquinoléinyle, phtalazine, benzothiadiazolyle, benzothiazolyle, benzotriazolyle, benzodioxolanyle, benzodioxanyle et carbazolyle, non substitué ou substitué une ou plusieurs fois.

8. Dérivés de cyclohexyl-1,4-diamine substitués selon l'une quelconque des revendications 1 à 6, **caractérisés en ce que** A représente pyridyle, thiophényle, pyrazolyle, triazolyle, tétrahydroisoxazolyle, isoxazolyle, thiazolyle et furanyle.

9. Dérivés de cyclohexyl-1,4-diamine substitués selon l'une quelconque des revendications 1 à 8, **caractérisés en ce que** B représente (CH₂)ₘ avec m = 0, 1 ; SO₂, O, S.

10. Dérivés de cyclohexyl-1,4-diamine substitués selon l'une quelconque des revendications 1 à 9, **caractérisés en ce que** R⁴ représente phényle, benzyle, naphtyle, anthracényle, thiophényle, benzothiophényle, furanyle, isothiazolyle, imidazolyle, triazolyle, triazinyle, pyrazolyle, benzofuranyle, benzodioxolanyle, isoquinoléinyle, phtalazine, benzo[1,2,5]thiadiazole, benzothiazole, benzotriazole, quinoléinyle, carbazole, isoxazolyle, oxazolyle, indolyle, indanyle, benzodioxanyle, indazolyle, benzimidazolyle, pyrrolyle, pyridyle, pyrimidyle ou pyrazinyle, à chaque fois non substitué ou substitué une ou plusieurs fois ; phényle, naphtyle, anthracényle, thiophényle, benzothiophényle, pyridyle, furyle, benzofuranyle, indolyle, indanyle, benzodioxanyle, pyrrolyle, pyrimidyle ou pyrazinyle relié par un groupe alkyle en C₁₋₂ saturé non ramifié, à chaque fois non substitué ou substitué une ou plusieurs fois,
notamment R⁴ représente phényle, benzyle, naphtyle, thiophényle, benzothiophényle, furanyle, isothiazolyle, imidazolyle, triazolyle, pyrazolyle, benzofuranyle, isoquinoléinyle, benzothiazole, benzotriazole, quinoléinyle, isoxazolyle, oxazolyle, indolyle, pyrrolyle, pyridyle, pyrimidyle ou pyrazinyle, à chaque fois non substitué ou substitué une ou plusieurs fois ; benzyle ou phénéthyle, à chaque fois non substitué ou substitué une ou plusieurs fois.

11. Dérivés de cyclohexyl-1,4-diamine substitués selon l'une quelconque des revendications 1 à 9, **caractérisés en ce que** R⁴ représente phényle, pyrazolyle ou thiophényle, non substitué ou substitué une ou plusieurs fois.

12. Dérivés de cyclohexyl-1,4-diamine substitués selon l'une quelconque des revendications 1 à 11, du groupe
(4-diméthylamino-4-phényl-cyclohexyl)-amide de l'acide 5-méthyl-2-phényl-2H-[1,2,3]triazole-4-carboxylique
[4-diméthylamino-4-(3-fluoro-phényl)-cyclohexyl]-amide de l'acide 1-phényl-5-propyl-1H-pyrazole-4-carboxylique
(4-benzyl-4-pyrrolidin-1-yl-cyclohexyl)-amide de l'acide 3-(2,6-dichloro-phényl)-5-méthyl-isoxazole-4-carboxylique
(4-benzyl-4-pipéridin-1-yl-cyclohexyl)-amide de l'acide 5-méthyl-2-phényl-2H-[1,2,3]triazole-4-carboxylique
(4-phényl-4-pipéridin-1-yl-cyclohexyl)-amide de l'acide 5-méthyl-2-phényl-2H-[1,2,3]triazole-4-carboxylique
N-(4-diméthylamino-4-phényl-cyclohexyl)-2-phénoxy-nicotinamide
2-(4-chloro-phénylsulfanyl)-N-(4-diméthylamino-4-pyridin-2-yl-cyclohexyl)-nicotinamide
N-(4-diméthylamino-4-thiophén-2-yl-cyclohexyl)-2-(5-méthyl-2-phényl-thiazol-4-yl)-acétamide
2-(4-chloro-phénylsulfanyl)-N-[4-diméthylamino-4-(3-fluoro-phényl)-cyclohexyl]-nicotinamide
N-(4-diméthylamino-4-thiophén-2-yl-cyclohexyl)-2-phénoxy-nicotinamide
(4-diméthylamino-4-phényl-cyclohexyl)-amide de l'acide 3-(2-chloro-phényl)-5-méthyl-isoxazole-4-carboxylique
[4-diméthylamino-4-(3-fluoro-phényl)-cyclohexyl]-amide de l'acide 5-(4-chloro-phényl)-2-méthyl-furane-3-carboxylique
(4-diméthylamino-4-phénéthyl-cyclohexyl)-amide de l'acide 3-(2-chloro-6-fluoro-phényl)-5-méthyl-isoxazole-4-carboxylique
N-(4-benzyl-4-pyrrolidin-1-yl-cyclohexyl)-2-(4-chloro-phénylsulfanyl)-nicotinamide
(4-diméthylamino-4-phényl-cyclohexyl)-amide de l'acide
3-(2-chloro-6-fluoro-phényl)-5-méthyl-isoxazole-4-carboxylique
(4-benzyl-4-pipéridin-1-yl-cyclohexyl)-amide de l'acide 3-(2,6-dichloro-phényl)-5-méthyl-isoxazole-4-carboxylique
N-(4-diméthylamino-4-phénéthyl-cyclohexyl)-2-(5-méthyl-2-phényl-thiazol-4-yl)-acétamide
(4-diméthylamino-4-phénéthyl-cyclohexyl)-amide de l'acide 3-(2-chloro-phényl)-5-méthyl-isoxazole-4-carboxylique
(4-benzyl-4-diméthylamino-cyclohexyl)-amide de l'acide 5-benzyl-furane-2-carboxylique
[4-diméthylamino-4-(3-fluoro-phényl)-cyclohexyl]-amide de l'acide 1-(4-chloro-phényl)-5-trifluorométhyl-1H-pyrazole-4-carboxylique
N-(4-diméthylamino-4-pyridin-2-yl-cyclohexyl)-2-phénoxy-nicotinamide
(4-phényl-4-pipéridin-1-yl-cyclohexyl)-amide de l'acide 5-benzyl-furane-2-carboxylique
[4-diméthylamino-4-(2-méthyl-benzyl)-cyclohexyl]-amide de l'acide 5-méthyl-2-phényl-2H-[1,2,3]triazole-4-carboxylique
2-(4-chloro-phénylsulfanyl)-N-[4-diméthylamino-4-(2-fluoro-benzyl)-cyclohexyl]-nicotinamide
[4-diméthylamino-4-(2-fluoro-benzyl)-cyclohexyl]-amide de l'acide 3-(2,6-dichloro-phényl)-5-méthyl-isoxazole-4-carboxylique
[4-diméthylamino-4-(4-fluoro-benzyl)-cyclohexyl]-amide de l'acide 5-méthyl-2-phényl-2H-[1,2,3]triazole-4-carboxylique
[4-diméthylamino-4-(2-fluoro-benzyl)-cyclohexyl]-amide de l'acide 5-benzyl-furane-2-carboxylique
[4-diméthylamino-4-(3-fluoro-phényl)-cyclohexyl]-amide de l'acide 3-(2-chloro-phényl)-5-méthyl-isoxazole-4-carboxylique
[4-diméthylamino-4-(3-fluoro-phényl)-cyclohexyl]-amide de l'acide 3-(2-chloro-6-fluoro-phényl)-5-méthyl-isoxazole-4-carboxylique
N-(4-diméthylamino-4-phénéthyl-cyclohexyl)-2-phénoxy-nicotinamide
[4-(3-chloro-benzyl)-4-diméthylamino-cyclohexyl]-amide de l'acide 3-(2-chloro-phényl)-5-méthyl-isoxazole-4-carboxylique
[4-diméthylamino-4-(3-fluoro-phényl)-cyclohexyl]-amide de l'acide 3-(2,6-dichloro-phényl)-5-méthyl-isoxazole-4-carboxylique
(4-diméthylamino-4-phénéthyl-cyclohexyl)-amide de l'acide 5-benzyl-furane-2-carboxylique
[4-diméthylamino-4-(4-méthyl-benzyl)-cyclohexyl]-amide de l'acide 5-méthyl-2-phényl-2H-[1,2,3]triazole-4-carboxylique
[4-diméthylamino-4-(2-méthyl-benzyl)-cyclohexyl]-amide de l'acide 5-benzyl-furane-2-carboxylique
(4-diméthylamino-4-phénéthyl-cyclohexyl)-amide de l'acide 3-(2,6-dichloro-phényl)-5-méthyl-isoxazole-4-carboxylique
N-(4-benzyl-4-pyrrolidin-1-yl-cyclohexyl)-2-phénoxy-nicotinamide
[4-diméthylamino-4-(3-méthyl-benzyl)-cyclohexyl]-amide de l'acide 5-benzyl-furane-2-carboxylique
[4-diméthylamino-4-(3-fluoro-benzyl)-cyclohexyl]-amide de l'acide 5-benzyl-furane-2-carboxylique
N-[4-(3-chloro-benzyl)-4-diméthylamino-cyclohexyl]-2-(4-chloro-phénylsulfanyl)-nicotinamide
2-(5-méthyl-2-phényl-thiazol-4-yl)-N-(4-phényl-4-pipéridin-1-yl-cyclohexyl)-acétamide
[4-diméthylamino-4-(3-méthyl-benzyl)-cyclohexyl]-amide de l'acide 5-méthyl-2-phényl-2H-[1,2,3]triazole-4-carboxylique
N-[4-diméthylamino-4-(2-méthyl-benzyl)-cyclohexyl]-2-(5-méthyl-2-phényl-thiazol-4-yl)-acétamide
[4-(3-chloro-benzyl)-4-diméthylamino-cyclohexyl]-amide de l'acide 1-(4-chloro-phényl)-5-trifluorométhyl-1H-pyrazole-4-carboxylique
[4-(3-chloro-benzyl)-4-diméthylamino-cyclohexyl]-amide de l'acide 5-méthyl-2-phényl-2H-[1,2,3]triazole-4-carboxylique
(4-diméthylamino-4-phénéthyl-cyclohexyl)-amide de l'acide 5-méthyl-2-phényl-2H-[1,2,3]triazole-4-carboxylique
(4-benzyl-4-pyrrolidin-1-yl-cyclohexyl)-amide de l'acide 3-(2-chloro-6-fluoro-phényl)-5-méthyl-isoxazole-4-carboxylique
N-[4-diméthylamino-4-(2-fluoro-benzyl)-cyclohexyl]-2-phénoxy-nicotinamide
N-[4-diméthylamino-4-(3-fluoro-benzyl)-cyclohexyl]-2-(5-méthyl-2-phényl-thiazol-4-yl)-acétamide
N-(4-benzyl-4-diméthylamino-cyclohexyl)-2-phénoxy-nicotinamide
(4-benzyl-4-diméthylaminocyclohexyl)-amide de l'acide 5-(thiophène-2-sulfonyl)-4,5,6,7-tétrahydro-isoxazolo[4,5-c]pyridine-3-carboxylique
[4-diméthylamino-4-(2-fluoro-benzyl)-cyclohexyl]-amide de l'acide 1-(4-chloro-phényl)-5-trifluorométhyl-1H-pyrazole-4-carboxylique
[4-(4-chloro-benzyl)-4-diméthylamino-cyclohexyl]-amide de l'acide 5-benzyl-furane-2-carboxylique
(4-benzyl-4-diméthylamino-cyclohexyl)-amide de l'acide 5-méthyl-2-phényl-2H-[1,2,3]triazole-4-carboxylique
N-(4-benzyl-4-diméthylamino-cyclohexyl)-2-(5-méthyl-2-phényl-thiazol-4-yl)-acétamide
N-[4-diméthylamino-4-(2-fluoro-benzyl)-cyclohexyl]-2-(5-méthyl-2-phényl-thiazol-4-yl)-acétamide
[4-diméthylamino-4-(2-fluoro-benzyl)-cyclohexyl]-amide de l'acide 3-(2-chloro-phényl)-5-méthyl-isoxazole-4-carboxylique
[4-(2-chloro-benzyl)-4-diméthylamino-cyclohexyl]-amide de l'acide 5-méthyl-2-phényl-2H-[1,2,3]triazole-4-carboxylique
2-(4-chloro-phénylsulfanyl)-N-[4-diméthylamino-4-(3-fluoro-benzyl)-cyclohexyl]-nicotinamide
(4-benzyl-4-diméthylamino-cyclohexyl)-amide de l'acide 3-(2,6-dichloro-phényl)-5-méthyl-isoxazole-4-carboxylique
N-[4-(3-chloro-benzyl)-4-diméthylamino-cyclohexyl]-2-phénoxy-nicotinamide
2-phénoxy-N-(4-phényl-4-pipéridin-1-yl-cyclohexyl)-nicotinamide
(4-benzyl-4-pyrrolidin-1-yl-cyclohexyl)-amide de l'acide 5-(4-chloro-phényl)-2-méthyl-furane-3-carboxylique
(4-benzyl-4-diméthylamino-cyclohexyl)-amide de l'acide 5-(3-trifluorométhyl-benzylsulfonyl)-4,5,6,7-tétrahydro-isoxazolo[4,5-c]pyridine-3-carboxylique
N-[4-diméthylamino-4-(4-méthyl-benzyl)-cyclohexyl]-2-phénoxy-nicotinamide
N-[4-diméthylamino-4-(3-fluoro-benzyl)-cyclohexyl]-2-phénoxy-nicotinamide
(4-phényl-4-pipéridin-1-yl-cyclohexyl)-amide de l'acide 3-(2-chloro-6-fluoro-phényl)-5-méthyl-isoxazole-4-carboxylique
[4-diméthylamino-4-(4-fluoro-benzyl)-cyclohexyl]-amide de l'acide 5-(4-chloro-phényl)-2-méthyl-furane-3-carboxylique
[4-diméthylamino-4-(4-méthyl-benzyl)-cyclohexyl]-amide de l'acide 1-(4-chloro-phényl)-5-trifluorométhyl-1H-pyrazole-4-carboxylique
[4-(4-chloro-benzyl)-4-diméthylamino-cyclohexyl]-amide de l'acide 5-méthyl-2-phényl-2H-[1,2,3]triazole-4-carboxylique
[4-diméthylamino-4-(3-fluoro-benzyl)-cyclohexyl]-amide de l'acide 5-méthyl-2-phényl-2H-[1,2,3]triazole-4-carboxylique
(4-phényl-4-pipéridin-1-yl-cyclohexyl)-amide de l'acide 3-(2,6-dichloro-phényl)-5-méthyl-isoxazole-4-carboxylique
(4-morpholin-4-yl-4-phényl-cyclohexyl)-amide de l'acide 1-(4-chloro-phényl)-5-trifluorométhyl-1H-pyrazole-4-carboxylique
N-[4-(4-chloro-benzyl)-4-diméthylamino-cyclohexyl]-2-phénoxy-nicotinamide
N-[4-(4-chloro-benzyl)-4-diméthylamino-cyclohexyl]-2-(5-méthyl-2-phényl-thiazol-4-yl)-acétamide
(4-azépan-1-yl-4-benzyl-cyclohexyl)-amide de l'acide 3-(2-chloro-phényl)-5-méthyl-isoxazole-4-carboxylique
[4-diméthylamino-4-(4-fluoro-benzyl)-cyclohexyl]-amide de l'acide 3-(2-chloro-6-fluoro-phényl)-5-méthyl-isoxazole-4-carboxylique
[4-(3-chloro-benzyl)-4-diméthylamino-cyclohexyl]-amide de l'acide 3-(2-chloro-6-fluoro-phényl)-5-méthyl-isoxazole-4-carboxylique
[4-diméthylamino-4-(2-méthyl-benzyl)-cyclohexyl]-amide de l'acide 3-(2-chloro-phényl)-5-méthyl-isoxazole-4-carboxylique
[4-diméthylamino-4-(2-méthyl-benzyl)-cyclohexyl]-amide de l'acide 3-(2-chloro-6-fluoro-phényl)-5-méthyl-isoxazole-4-carboxylique
(4-benzyl-4-pipéridin-1-yl-cyclohexyl)-amide de l'acide 5-(1-méthyl-5-trifluorométhyl-1H-pyrazol-3-yl)-thiophène-2-carboxylique
[4-diméthylamino-4-(4-fluoro-benzyl)-cyclohexyl]-amide de l'acide 3-(2-chloro-phényl)-5-méthyl-isoxazole-4-carboxylique
[4-diméthylamino-4-(4-méthyl-benzyl)-cyclohexyl]-amide de l'acide 3-(2,6-dichloro-phényl)-5-méthyl-isoxazole-4-carboxylique
(4-benzyl-4-pyrrolidin-1-yl-cyclohexyl)-amide de l'acide 3-(2-chloro-phényl)-5-méthyl-isoxazole-4-carboxylique
[4-diméthylamino-4-(3-fluoro-benzyl)-cyclohexyl]-amide de l'acide 3-(2-chloro-6-fluoro-phényl)-5-méthyl-isoxazole-4-carboxylique
[4-diméthylamino-4-(4-méthyl-benzyl)-cyclohexyl]-amide de l'acide 3-(2-chloro-6-fluoro-phényl)-5-méthyl-isoxazole-4-carboxylique
[4-(4-chloro-benzyl)-4-diméthylamino-cyclohexyl]-amide de l'acide 1-(4-chloro-phényl)-5-trifluorométhyl-1H-pyrazole-4-carboxylique
(4-azépan-1-yl-4-benzyl-cyclohexyl)-amide de l'acide 1-phényl-5-propyl-1H-pyrazole-4-carboxylique
[4-diméthylamino-4-(3-fluoro-benzyl)-cyclohexyl]-amide de l'acide 3-(2-chloro-phényl)-5-méthyl-isoxazole-4-carboxylique
[4-(2-chloro-benzyl)-4-diméthylamino-cyclohexyl]-amide de l'acide 1-phényl-5-propyl-1H-pyrazole-4-carboxylique
(4-benzyl-4-diméthylamino-cyclohexyl)-amide de l'acide 3-(2-chloro-6-fluoro-phényl)-5-méthyl-isoxazole-4-carboxylique
[4-diméthylamino-4-(3-fluoro-benzyl)-cyclohexyl]-amide de l'acide 3-(2,6-dichloro-phényl)-5-méthyl-isoxazole-4-carboxylique
[4-diméthylamino-4-(4-fluoro-benzyl)-cyclohexyl]-amide de l'acide 1-phényl-5-propyl-1H-pyrazole-4-carboxylique
(4-benzyl-4-diméthylamino-cyclohexyl)-amide de l'acide 1-(4-chloro-phényl)-5-trifluorométhyl-1H-pyrazole-4-carboxylique
[4-diméthylamino-4-(3-fluoro-benzyl)-cyclohexyl]-amide de l'acide 5-(1-méthyl-5-trifluorométhyl-1H-pyrazol-3-yl)-thiophène-2-carboxylique
[4-diméthylamino-4-(2-fluoro-benzyl)-cyclohexyl]-amide de l'acide 3-(2-chloro-6-fluoro-phényl)-5-méthyl-isoxazole-4-carboxylique
(4-diméthylamino-4-phénéthyl-cyclohexyl)-amide de l'acide 1-(4-chloro-phényl)-5-trifluorométhyl-1H-pyrazole-4-carboxylique
[4-(3-chloro-benzyl)-4-diméthylamino-cyclohexyl]-amide de l'acide 5-(1-méthyl-5-trifluorométhyl-1H-pyrazol-3-yl)-thiophène-2-carboxylique
[4-diméthylamino-4-(2-fluoro-benzyl)-cyclohexyl]-amide de l'acide 5-(1-méthyl-5-trifluorométhyl-1H-pyrazol-3-yl)-thiophène-2-carboxylique
[4-(4-chloro-benzyl)-diméthylamino-cyclohexyl]-amide de l'acide 5-(1-méthyl-5-trifluorométhyl-1H-pyrazol-3-yl)-thiophène-2-carboxylique
[4-diméthylamino-4-(4-méthyl-benzyl)-cyclohexyl]-amide de l'acide 5-(1-méthyl-5-trifluorométhyl-1H-pyrazol-3-yl)-thiophène-2-carboxylique
[4-diméthylamino-4-(4-méthyl-benzyl)-cyclohexyl]-amide de l'acide 5-benzyl-furane-2-carboxylique
(4-azépan-1-yl-4-benzyl-cyclohexyl)-amide de l'acide 5-méthyl-2-phényl-2H-[1,2,3]triazole-4-carboxylique
2-(4-chloro-phénylsulfanyl)-N-[4-diméthylamino-4-(4-fluoro-benzyl)-cyclohexyl]-nicotinamide
N-[4-diméthylamino-4-(4-fluoro-benzyl)-cyclohexyl]-2-phénoxy-nicotinamide
(4-benzyl-4-pipéridin-1-yl-cyclohexyl)-amide de l'acide 5-benzyl-furane-2-carboxylique
[4-(4-chloro-benzyl)-4-diméthylamino-cyclohexyl]-amide de l'acide 3-(2,6-dichloro-phényl)-5-méthyl-isoxazole-4-carboxylique
N-(4-benzyl-4-morpholin-4-yl-cyclohexyl)-2-(4-chloro-phénylsulfanyl)-nicotinamide
(4-benzyl-4-diméthylamino-cyclohexyl)-amide de l'acide 5-(1-méthyl-5-trifluorométhyl-1H-pyrazol-3-yl)-thiophène-2-carboxylique
[4-diméthylamino-4-(4-fluoro-benzyl)-cyclohexyl]-amide de l'acide 1-(4-chloro-phényl)-5-trifluorométhyl-1H-pyrazole-4-carboxylique
[4-diméthylamino-4-(2-méthyl-benzyl)-cyclohexyl]-amide de l'acide 1-(4-chloro-phényl)-5-trifluorométhyl-1H-pyrazole-4-carboxylique
N-(4-azépan-1-yl-4-benzyl-cyclohexyl)-2-phénoxy-nicotinamide
[4-(2-chloro-benzyl)-4-diméthylamino-cyclohexyl]-amide de l'acide 3-(2,6-dichloro-phényl)-5-méthyl-isoxazole-4-carboxylique
N-[4-diméthylamino-4-(2-méthyl-benzyl)-cyclohexyl]-2-phénoxy-nicotinamide
[4-diméthylamino-4-(4-fluoro-benzyl)-cyclohexyl]-amide de l'acide 3-(2,6-dichloro-phényl)-5-méthyl-isoxazole-4-carboxylique
2-(4-chloro-phénylsulfanyl)-N-[4-diméthylamino-4-(2-méthyl-benzyl)-cyclohexyl]-nicotinamide
[4-diméthylamino-4-(2-méthyl-benzyl)-cyclohexyl]-amide de l'acide 3-(2,6-dichloro-phényl)-5-méthyl-isoxazole-4-carboxylique
chlorhydrate de [4-diméthylamino-4-(3-fluorophényl)-cyclohexyl]-amide de l'acide 3-(2-chlorophényl)-5-méthylisoxazole-4-carboxylique, diastéréoisomère non polaire
chlorhydrate de [4-diméthylamino-4-(3-fluorophényl)-cyclohexyl]-amide de l'acide 3-(2-chlorophényl)-5-méthylisoxazole-4-carboxylique, diastéréoisomère polaire
chlorhydrate de (4-diméthylamino-4-phénylcyclohexyl)-amide de l'acide 5-méthyl-2-phényl-2H-[1,2,3]triazole-4-carboxylique, diastéréoisomère non polaire
chlorhydrate de (4-diméthylamino-4-phénylcyclohexyl)-amide de l'acide 5-méthyl-2-phényl-2H-[1,2,3]triazole-4-carboxylique, diastéréoisomère polaire
chlorhydrate de [4-diméthylamino-4-(3-fluoro-phényl)cyclohexyl]-amide de l'acide 3-(2,6-dichlorophényl)-5-méthylisoxazole-4-carboxylique, diastéréoisomère non polaire
chlorhydrate de [4-diméthylamino-4-(3-fluoro-phényl)cyclohexyl]-amide de l'acide 3-(2,6-dichlorophényl)-5-méthylisoxazole-4-carboxylique, diastéréoisomère polaire
chlorhydrate de (4-diméthylamino-4-phénylcyclohexyl)-amide de l'acide 3-(2,6-dichlorophényl)-5-méthylisoxazole-4-carboxylique, diastéréoisomère non polaire
chlorhydrate de (4-diméthylamino-4-phénylcyclohexyl)-amide de l'acide 3-(2,6-dichlorophényl)-5-méthylisoxazole-4-carboxylique, diastéréoisomère polaire
chlorhydrate de [4-diméthylamino-4-(3-fluoro-phényl)cyclohexyl]-amide de l'acide 5-méthyl-3-phénylisoxazole-4-carboxylique, diastéréoisomère non polaire
chlorhydrate de (4-diméthylamino-4-phénylcyclohexyl)-amide de l'acide 5-méthyl-3-phénylisoxazole-4-carboxylique, diastéréoisomère non polaire
chlorhydrate de (4-diméthylamino-4-phénylcyclohexyl)-amide de l'acide 5-méthyl-3-phénylisoxazole-4-carboxylique, diastéréoisomère polaire,
sous la forme du racémat ; des énantiomères, diastéréomères, mélanges des énantiomères ou diastéréomères ou d'un énantiomère ou diastéréomère individuel ; des bases et/ou des sels d'acides ou de cations physiologiquement acceptables.

13. Procédé de fabrication de dérivés de cyclohexyl-1,4-diamine substitués selon l'une quelconque des revendications 1 à 12, **caractérisé en ce que** des cyclohexane-1,4-diamines substituées sont reliées avec des acides carboxyliques de formule générale II 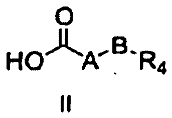 avec ajout de réactifs de couplage ou par activation du composant acide carboxylique, notamment par fabrication du chlorure d'acide.

14. Médicament contenant au moins un dérivé de cyclohexyl-1,4-diamine substitué selon l'une quelconque des revendications 1 à 12, éventuellement sous la forme de son racémat, des stéréoisomères purs, notamment des énantiomères et des diastéréomères, en un rapport de mélange quelconque ; sous la forme de ses acides ou de ses bases ou sous la forme de ses sels, notamment des sels physiologiquement acceptables ou des sels d'acides ou de cations physiologiquement acceptables ; ou sous la forme de ses solvates, notamment des hydrates, et contenant éventuellement des additifs et/ou adjuvants appropriés et/ou éventuellement d'autres agents actifs.

15. Dérivé de cyclohexyl-1,4-diamine substitué selon l'une quelconque des revendications 1 à 12, éventuellement sous la forme de son racémat, des stéréoisomères purs, notamment des énantiomères et des diastéréomères, en un rapport de mélange quelconque ; sous la forme de ses acides ou de ses bases ou sous la forme de ses sels, notamment des sels physiologiquement acceptables ou des sels d'acides ou de cations physiologiquement acceptables ; ou sous la forme de ses solvates, notamment des hydrates, pour une utilisation pour le traitement de la douleur, notamment de la douleur aiguë, neuropathique ou chronique, pour une utilisation pour le traitement des états d'anxiété, du stress et des syndromes associés avec le stress, des dépressions, de l'épilepsie, de la maladie d'Alzheimer, de la démence sénile, de la catalepsie, des dysfonctionnements cognitifs généraux, des difficultés d'apprentissage et de mémorisation (en tant que nootropique), des phénomènes de sevrage, de l'abus de et/ou de la dépendance à l'alcool et/ou aux drogues et/ou aux médicaments, des dysfonctionnements sexuels, des maladies cardiovasculaires, de l'hypotension, de l'hypertension, des acouphènes, du prurit, de la migraine, de la surdité, du manque de motilité intestinale, des troubles de l'alimentation, de l'anorexie, de l'obésité, des troubles locomoteurs, de la diarrhée, de la cachexie, de l'incontinence urinaire ou en tant que relaxant musculaire, anticonvulsif ou anesthésique ou pour la co-administration lors du traitement avec un analgésique opioïde ou avec un anesthésique, pour la diurèse ou l'antinatriurèse, l'anxiolyse, pour la modulation de l'activité motrice, pour la modulation de la distribution des neurotransmetteurs et le traitement des maladies neurodégénératives associées, pour le traitement des phénomènes de sevrage et/ou pour la réduction du potentiel d'accoutumance des opioïdes.
